# EUROPEAN PATENT SPECIFICATION

(11) **EP 2 178 556 B1**
(45) Date of publication and mention of the grant of the patent: **17.08.2011**
(21) Application number: 08771345.9
(22) Date of filing: 18.06.2008
(51) Int. Cl.: A61K 38/48, A61P 7/02, A61P 7/04

(54) **PROTEIN C FOR USE IN MAINTAINING HEMOSTASIS**
PROTEIN C ZUR VERWENDUNG BEI DER AUFRECHTERHALTUNG DER HÄMOSTASE
PROTÉINE C POUR MAINTENIR L'HÉMOSTASE

(30) Priority: 18.06.2007 US 944693 P
(43) Date of publication of application: 28.04.2010
(73) Proprietor: Oregon Health & Science University, Portland, OR 97201 (US); The Scripps Research Institute, La Jolla, CA 92037 (US)
(72) Inventor: MCCARTY, Owen, J.T., Portland, OR 97202 (US); GRUBER, Andras, Portland, OR 97221 (US); GRIFFIN, John , H., Del Mar, California 92104 (US)
(74) Representative: Walton, Seán Malcolm
(86) International application number: PCT/US2008/067321
(87) International publication number: WO 2008/157593

(56) References cited:
- EP-A- 0 575 054
- WO-A-01/36462
- WO-A-94/02172
- WO-A-03/039585
- WO-A-2005/007820
- WO-A-2006/114105
- SQUIRES R A ET AL: "Blockade of protein C activation reduces microvascular surgical blood loss" JOURNAL OF SURGICAL RESEARCH, ACADEMIC PRESS INC., SAN DIEGO, CA, US, vol. 52, no. 6, 1 June 1992 (1992-06-01), pages 560-564, XP023022750 ISSN: 0022-4804 [retrieved on 1992-06-01]
- LUST M ET AL: "The protein C pathway in inflammatory bowel disease: the missing link between inflammation and coagulation" TRENDS IN MOLECULAR MEDICINE, ELSEVIER CURRENT TRENDS, vol. 14, no. 6, 1 June 2008 (2008-06-01), pages 237-244, XP022711881 ISSN: 1471-4914 [retrieved on 2008-05-03]
- ESMON C T: "The protein C pathway" CHEST 20030901 US, vol. 124, no. 3 SUPPL., 1 September 2003 (2003-09-01), pages 26S-32S, XP002508411 ISSN: 0012-3692
- WHITE T C ET AL: "Protein C supports platelet binding and activation under flow: Role of glycoprotein Ib and apolipoprotein E receptor 2" JOURNAL OF THROMBOSIS AND HAEMOSTASIS 200806 GB, vol. 6, no. 6, June 2008 (2008-06), pages 995-1002, XP002508412 ISSN: 1538-7933 1538-7836
- "Hemostasis Introduction" MERCK MANUALS ONLINE MEDICAL LIBRARY, [Online] November 2005 (2005-11), XP002508413 Retrieved from the Internet: URL:http://www.merck.com/mmpe/print/sec11/ ch134/ch134a.html> [retrieved on 2008-12-17]
- "Thrombotic disorders" MERCK MANUALS ONLINE MEDICAL LIBRARY, [Online] November 2005 (2005-11), XP002508414 Retrieved from the Internet: URL:http://www.merck.com/mmpe/print/sec11/ ch135/ch135a.html> [retrieved on 2008-12-17]

### SEQUENCE LISTING

<110> Oregon Health and Sci ence University
   The Scripps Research Institute
   McCarty, Owen J. T.
   Gruber, Andras
   Giffin, John H.
<120> HEMOSTATIC AGENTS
<130> 899-78996-02
<140> PCTUS0867321
   <141> 2008- 06- 18
<150> US 60/944,693
   <151> 2007- 06- 18
<160> 18
<170> Patent Inversion 3.3
<210> 1
   <211> 1776
   <212> DNA
   <213> Homo sapi ens
<220>
   <221> CDS
   <222> (74)..(1459)
<400> 1
gctgtcatgg cggcaggacg gcgaacttgc agtatctcca cgacccgccc ctacaggtgc      60
   cagtgcctcc aga atg tgg cag ctc aca agc ctc ctg ctg ttc gtg gcc        109
   acc tgg gga att tcc ggc aca cca gct cct ctt gac tca gtg ttc tcc        157
   agc agc gag cgt gcc cac cag gtg ctg cgg atc cgc aaa cgt gcc aac        205
   tcc ttc ctg gag gag ctc cgt cac agc agc ctg gag cgg gag tgc ata        253
   gag gag atc tgt gac ttc gag gag gcc aag gaa att ttc caa aat gtg        301
   gat gac aca ctg gcc ttc tgg tcc aag cac gtc gac ggt gac cag tgc    349
   ttg gtc ttg ccc ttg gag cac ccg tgc gcc agc ctg tgc tgc ggg cac    397
   ggc acg tgc atc gac ggc atc ggc agc ttc agc tgc gac tgc cgc agc    445
   ggc tgg gag ggc cgc ttc tgc cag cgc gag gtg agc ttc ctc aat tgc    493
   tcg ctg gac aac ggc ggc tgc acg cat tac tgc cta gag gag gtg ggc    541
   tgg cgg cgc tgt agc tgt gcg cct ggc tac aag ctg ggg gac gac ctc    589
   ctg cag tgt cac ccc gca gtg aag ttc cct tgt ggg agg ccc tgg aag    637
   cgg atg gag aag aag cgc agt cac ctg aaa cga gac aca gaa gac caa    685
   gaa gac caa gta gat ccg cgg ctc att gat ggg aag atg acc agg cgg    733
   gga gac agc ccc tgg cag gtg gtc ctg ctg gac tca aag aag aag ctg    781
   gcc tgc ggg gca gtg ctc atc cac ccc tcc tgg gtg ctg aca gcg gcc    829
   cac tgc atg gat gag tcc aag aag ctc ctt gtc agg ctt gga gag tat    877
   gac ctg cgg cgc tgg gag aag tgg gag ctg gac ctg gac atc aag gag    925
   gtc ttc gtc cac ccc aac tac agc aag agc acc acc gac aat gac atc    973
   gca ctg ctg cac ctg gcc cag ccc gcc acc ctc tcg cag acc ata gtg    1021
   ccc atc tgc ctc ccg gac agc ggc ctt gca gag cgc gag ctc aat cag    1069
   gcc ggc cag gag acc ctc gtg acg ggc tgg ggc tac cac agc agc cga    1117
   gag aag gag gcc aag aga aac cgc acc ttc gtc ctc aac ttc atc aag    1165
   att ccc gtg gtc ccg cac aat gag tgc agc gag gtc atg agc aac atg     1213
   gtg tct gag aac atg ctg tgt gcg ggc atc ctc ggg gac cgg cag gat     1261
   gcc tgc gag ggc gac agt ggg ggg ccc atg gtc gcc tcc ttc cac ggc     1309
   acc tgg ttc ctg gtg ggc ctg gtg agc tgg ggt gag ggc tgt ggg ctc     1357
   ctt cac aac tac ggc gtt tac acc aaa gtc agc cgc tac ctc gac tgg     1405
   atc cat ggg cac atc aga gac aag gaa gcc ccc cag aag agc tgg gca     1453
   cct tag cgaccctccc tgcagggctg ggcttttgca tggcaatgga tgggacatta      1509
   aagggacatg taacaagcac accggcctgc tgttctgtcc ttccatccct cttttgggct   1569
   cttctggagg gaagtaacat ttactgagca cctgttgtat gtcacatgcc ttatgaatag   1629
   aatcttaact cctagagcaa ctctgtgggg tggggaggag cagatccaag ttttgcgggg   1689
   tctaaagctg tgtgtgttga gggggatact ctgtttatga aaaagaataa aaaacacaac   1749
   cacgaaaaaa aaaaaaaaaa aaaaaaa                                       1776
<210> 2
   <211> 461
   <212> PRT
   <213> Homo sapiens
<400> 2
<210> 3
   <211> 1792
   <212> DNA
   <213> Homo sapiens
<220>
   <221> CDS
   <222> (56)..(1441)
<400> 3
cggcgaactt gcagtatctc cacgacccgc ccctacaggt gccagtgcct ccaga atg      58
   tgg cag ctc aca agc ctc ctg ctg ttc gtg gcc acc tgg gga att tcc      106
   ggc aca cca gct cct ctt gac tca gtg ttc tcc agc agc gag cgt gcc      154
   cac cag gtg ctg cgg atc cgc aaa cgt gcc aac tcc ttc ctg gag gag      202
   ctc cgt cac agc agc ctg gag cgg gag tgc ata gag gag atc tgt gac      250
   ttc gag gag gcc aag gaa att ttc caa aat gtg gat gac aca ctg gcc      298
   ttc tgg tcc aag cac gtc gac ggt gac cag tgc ttg gtc ttg ccc ttg      346
   gag cac ccg tgc gcc agc ctg tgc tgc ggg cac ggc acg tgc atc gac      394
   ggc atc ggc agc ttc agc tgc gac tgc cgc agc ggc tgg gag ggc cgc      442
   ttc tgc cag cgc gag gtg agc ttc ctc aat tgc tct ctg gac aac ggc      490
   ggc tgc acg cat tac tgc cta gag gag gtg ggc tgg cgg cgc tgt agc      538
   tgt gcg cct ggc tac aag ctg ggg gac gac ctc ctg cag tgt cac ccc      586
   gca gtg aag ttc cct tgt ggg agg ccc tgg aag cgg atg gag aag aag      634
   cgc agt cac ctg aaa cga gac aca gaa gac caa gaa gac caa gta gat      682
   ccg cgg ctc att gat ggg aag atg acc agg cgg gga gac agc ccc tgg      730
   cag gtg gtc ctg ctg gac tca aag aag aag ctg gcc tgc ggg gca gtg      778
   ctc atc cac ccc tcc tgg gtg ctg aca gcg gcc cac tgc atg gat gag      826
   tcc aag aag ctc ctt gtc agg ctt gga gag tat gac ctg cgg cgc tgg      874
   gag aag tgg gag ctg gac ctg gac atc aag gag gtc ttc gtc cac ccc      922
   aac tac agc aag agc acc acc gac aat gac atc gca ctg ctg cac ctg      970
   gcc cag ccc gcc acc ctc tcg cag acc ata gtg ccc atc tgc ctc ccg     1018
   gac agc ggc ctt gca gag cgc gag ctc aat cag gcc ggc cag gag acc     1066
   ctc gtg acg ggc tgg ggc tac cac agc agc cga gag aag gag gcc aag     1114
   aga aac cgc acc ttc gtc ctc aac ttc atc aag att ccc gtg gtc ccg     1162
   cac aat gag tgc agc gag gtc atg agc aac atg gtg tct gag aac atg     1210
   ctg tgt gcg ggc atc ctc ggg gac cgg cag gat gcc tgc gag ggc gac      1258
   agt ggg ggg ccc atg gtc gcc tcc ttc cac ggc acc tgg ttc ctg gtg      1306
   ggc ctg gtg agc tgg ggt gag ggc tgt ggg ctc ctt cac aac tac ggc      1354
   gtt tac acc aaa gtc agc cgc tac ctc gac tgg atc cat ggg cac atc      1402
   aga gac aag gaa gcc ccc cag aag agc tgg gca cct tag cgaccctccc       1451
   tgcagggctg ggcttttgca tggcaatgga tgggacatta aagggacatg taacaagcac    1511
   accggcctgc tgttctgtcc ttccatccct cttttgggct cttctggagg gaagtaacat    1571
   ttactgagca cctgttgtat gtcacatgcc ttatgaatag aatcttaact cctagagcaa    1631
   ctctgtgggg tggggaggag cagatccaag ttttgcgggg tctaaagctg tgtgtgttga    1691
   gggggatact ctgtttatga aaaagaataa aaaacacaac cacgaaaaaa aaaaaaaaaa    1751
   aaaaaaaaaa aaaaaaaaaa aaaaaaaaaa aaaaaaaaaa a                        1792
<210> 4
   <211> 461
   <212> PRT
   <213> Homo sapiens
<400> 4
<210> 5
   <211> 1366
   <212> DNA
   <213> Homo sapi ens
<400> 5
   ccaagggcac ggcacgtgca tcgacggcat cggcagcttc agctgcgact gccgcagcgg 60
   ctgggagggc cgcttctgcc agcgcgaggt gagcttcctc aattgctctc tggacaacgg 120
   cggctgcacg cattactgcc tagaggaggt gggctggcgg cgctgtagct gtgcgcctgg 180
   ctacaagctg ggggacgacc tcctgcagtg tcaccccgca gtgaagttcc cttgtgggag 240
   gccctggaag cggatggaga agaagcgcag tcacctgaaa cgagacacag aagaccaaga 300
   agaccaagta gatccgcggc tcattgatgg gaagatgacc aggcggggag acagcccctg 360
   gcaggtggtc ctgctggact caaagaagaa gctggcctgc ggggcagtgc tcatccaccc 420
   ctcctgggtg ctgacagcgg cccactgcat ggacgagtcc aagaagctcc ttgtcaggct 480
   tggagagtat gacctgcggc gctgggagaa gtgggagctg gacctggaca tcaaggaggt 540
   cttcgtccac cccaactaca gcaagagcac caccgacaat gacatcgcac tgctgcacct 600
   ggcccagccc gccaccctct cgcagaccat agtgcccatc tgcctcccgg acagcggcct 660
   tgcagagcgc gagctcaatc aggccggcca ggagaccctc gtgacgggct ggggctacca 720
   cagcagccga gagaaggagg ccaagagaaa ccgcaccttc gtcctcaact tcatcaagat 780
   tcccgtggtc ccgcacaatg agtgcagcga ggtcatgagc aacatggtgt ctgagaacat 840
   gctgtgtgcg ggcatcctcg ggaccggcag ggatgcctgc gagggcgaca gtggggggcc 900
   catggtcgcc tccttccacg gcacctggtt cctggtgggc ctggtgagct ggggtgaggg 960
   ctgtgggctc cttcacaact acggcgttta caccaaagtc agccgctacc tcgactggat 1020
   ccatgggcac atcagagaca aggaagcccc ccagaagagc tgggcacctt agcgaccctc 1080
   cctgcagggc tgggcttttg catggcaatg gatgggacat taaagggaca tgtaacaagc 1140
   acaccggcct gctgttctgt ccttccatcc ctcttttggg ctcttctgga gggaagtaac 1200
   atttactgag cacctgttgt atgtcacatg ccttatgaat agaatcttaa ctcctagagc 1260
   aactctgtcg ggtggggagg agcagatcca agttttgcgg ggtctaaagc tgtgtgtgtt 1320
   gagggggata ctctgtttat gaaaaagaat aaaaaacaca accacg 1366
<210> 6
   <211> 356
   <212> PRT
   <213> Homo sapiens
<400> 6
   Gln Gly His Gly Thr Cys Ile Asp Gly Ile Gly Ser Phe Ser Cys Asp
   1               5                   10                  15
   Cys Arg Ser Gly Trp Glu Gly Arg Phe Cys Gln Arg Glu Val Ser Phe
               20                  25                  30
   Leu Asn Cys Ser Leu Asp Asn Gly Gly Cys Thr His Tyr Cys Leu Glu
           35                  40                  45
   Glu Val Gly Trp Arg Arg Cys Ser Cys Ala Pro Gly Tyr Lys Leu Gly
       50                  55                  60
   Asp Asp Leu Leu Gln Cys His Pro Ala Val Lys Phe Pro Cys Gly Arg
   65                  70                  75                  80
   Pro Trp Lys Arg Met Glu Lys Lys Arg Ser His Leu Lys Arg Asp Thr
                   85                  90                  95
   Glu Asp Gln Glu Asp Gln Val Asp Pro Arg Leu Ile Asp Gly Lys Met
               100                 105                 110
   Thr Arg Arg Gly Asp Ser Pro Trp Gln Val Val Leu Leu Asp Ser Lys
           115                 120                 125
   Lys Lys Leu Ala Cys Gly Ala Val Leu Ile His Pro Ser Trp Val Leu
       130                 135                 140
   Thr Ala Ala His Cys Met Asp Glu Ser Lys Lys Leu Leu Val Arg Leu
   145                 150                 155                 160
   Gly Glu Tyr Asp Leu Arg Arg Trp Glu Lys Trp Glu Leu Asp Leu Asp
                   165                 170                 175
   Ile Lys Glu Val Phe Val His Pro Asn Tyr Ser Lys Ser Thr Thr Asp
               180                 185                 190
   Asn Asp Ile Ala Leu Leu His Leu Ala Gln Pro Ala Thr Leu Ser Gln
           195                 200                 205
   Thr Ile Val Pro Ile Cys Leu Pro Asp Ser Gly Leu Ala Glu Arg Glu
       210                 215                 220
   Leu Asn Gln Ala Gly Gln Glu Thr Leu Val Thr Gly Trp Gly Tyr His
   225                 230                 235                 240
   Ser Ser Arg Glu Lys Glu Ala Lys Arg Asn Arg Thr Phe Val Leu Asn
   245 250 255
   Phe Ile Lys Ile Pro Val Val Pro His Asn Glu Cys Ser Glu Val Met
   260 265 270
   Ser Asn Met Val Ser Glu Asn Met Leu Cys Ala Gly Ile Leu Gly Asp
   275 280 285
   Arg Gln Asp Ala Cys Glu Gly Asp Ser Gly Gly Pro Met Val Ala Ser
   290 295 300
   Phe His Gly Thr Trp Phe Leu Val Gly Leu Val Ser Trp Gly Glu Gly
   305 310 315 320
   Cys Gly Leu Leu His Asn Tyr Gly Val Tyr Thr Lys Val Ser Arg Tyr
   325 330 335
   Leu Asp Trp Ile His Gly His Ile Arg Asp Lys Glu Ala Pro Gln Lys
   340 345 350
   Ser Trp Ala Pro
   355
<210> 7
   <211> 1499
   <212> DNA
   <213> Mus musculus
<220>
   <221> CDS
   <222> (11)..(1396)
<400> 7
   gagctccagg atg tgg caa ttc aga gtc ttc ctg ctg ctc atg tcc acc 49
   Met Trp Gln Phe Arg Val Phe Leu Leu Leu Met Ser Thr
   1 5 10
   tgg gga ata tct agc ata ccg gcc cat cct gac cca gtg ttc tcc agc 97
   Trp Gly Ile Ser Ser Ile Pro Ala His Pro Asp Pro Val Phe Ser Ser
   15 20 25
   agc gag cat gcc cac cag gtg ctt cgg gtc aga cgt gcc aac agc ttc 145
   Ser Glu His Ala His Gln Val Leu Arg Val Arg Arg Ala Asn Ser Phe
   30 35 40 45
   ctg gaa gag atg cgg cca ggc agc ctg gaa cgg gag tgt atg gag gag 193
   Leu Glu Glu Met Arg Pro Gly Ser Leu Glu Arg Glu Cys Met Glu Glu
   50 55 60
   atc tgt gac ttc gag gag gcc cag gag att ttc caa aat gtg gaa gac 241
   Ile Cys Asp Phe Glu Glu Ala Gln Glu Ile Phe Gln Asn Val Glu Asp
   65 70 75
   aca ctg gcc ttc tgg atc aag tac ttt gac ggt gac cag tgc tcg gct 289
   Thr Leu Ala Phe Trp Ile Lys Tyr Phe Asp Gly Asp Gln Cys Ser Ala
   80 85 90
   cca ccc ttg gac cac cag tgc gac agc cca tgc tgc ggg cat ggc act 337
   Pro Pro Leu Asp His Gln Cys Asp Ser Pro Cys Cys Gly His Gly Thr
           95                        100                       105
   tgc atc gac ggc ata ggc agc ttc agc tgc agc tgc gat aag ggc tgg     385
   Cys Ile Asp Gly Ile Gly Ser Phe Ser Cys Ser Cys Asp Lys Gly Trp
   110                 115                     120                 125
   gag ggc aag ttc tgt cag cag gag ttg cgc ttc cag gac tgt cgg gtg     433
   Glu Gly Lys Phe Cys Gln Gln Glu Leu Arg Phe Gln Asp Cys Arg Val
                   130                     135                 140
   aac aat ggc ggc tgc ttg cac tac tgc ctg gag gag agc aat ggg cgg     481
   Asn Asn Gly Gly Cys Leu His Tyr Cys Leu Glu Glu Ser Asn Gly Arg
               145                     150                 155
   cgc tgc gct tgt gcc ccg ggc tat gag ctg gca gac gac cac atg cgc     529
   Arg Cys Ala Cys Ala Pro Gly Tyr Glu Leu Ala Asp Asp His Met Arg
           160                     165                 170
   tgc aag tcc act gtg aat ttt cca tgt ggg aaa ctg ggg agg tgg ata     577
   Cys Lys Ser Thr Val Asn Phe Pro Cys Gly Lys Leu Gly Arg Trp Ile
       175                     180                     185
   gag aag aaa cgc aag atc ctc aaa cga gac aca gac tta gaa gat gaa     625
   Glu Lys Lys Arg Lys Ile Leu Lys Arg Asp Thr Asp Leu Glu Asp Glu
   190                 195                     200                 205
   ctg gaa cca gat cca agg ata gtc aac gga acg ctg acg aag cag ggt     673
   Leu Glu Pro Asp Pro Arg Ile Val Asn Gly Thr Leu Thr Lys Gln Gly
                   210                 215                     220
   gac agt cct tgg cag gca atc ctt ctg gac tcc aag aag aag ctg gcc     721
   Asp Ser Pro Trp Gln Ala Ile Leu Leu Asp Ser Lys Lys Lys Leu Ala
               225                 230                     235
   tgc gga ggg gtg ctc atc cac act tcc tgg gtg ctg acg gca gcc cac     769
   Cys Gly Gly Val Leu Ile His Thr Ser Trp Val Leu Thr Ala Ala His
           240                 245                     250
   tgc gtg gag ggc acc aag aag ctt acc gtg agg ctt ggt gag tat gat     817
   Cys Val Glu Gly Thr Lys Lys Leu Thr Val Arg Leu Gly Glu Tyr Asp
       255                     260                 265
   ctg cga cgc agg gac cac tgg gag ctg gac ctg gac atc aag gag atc     865
   Leu Arg Arg Arg Asp His Trp Glu Leu Asp Leu Asp Ile Lys Glu Ile
   270                 275                     280                 285
   ctc gtc cac cct aac tac acc cgg agc agc agt gac aac gac att gct     913
   Leu Val His Pro Asn Tyr Thr Arg Ser Ser Ser Asp Asn Asp Ile Ala
                   290                 295                     300
   ctg ctc cgc cta gcc cag cca gcc act ctc tcc aaa acc ata gtg ccc     961
   Leu Leu Arg Leu Ala Gln Pro Ala Thr Leu Ser Lys Thr Ile Val Pro
               305                 310                     315
   atc tgc ctg ccg aac aat ggg ctc gct cag cag gag ctc act cag gct    1009
   Ile Cys Leu Pro Asn Asn Gly Leu Ala Gln Gln Glu Leu Thr Gln Ala
           320                 325                     330
   ggc cag gag aca gtg gtg aca ggc tgg ggc tat caa agc gac aga atc    1057
   Gly Gln Glu Thr Val Val Thr Gly Trp Gly Tyr Gln Ser Asp Arg Ile
       335                 340                     345
   aag gat ggc aga agg aac cgc acc ttc atc ctc acc ttc atc cgc atc    1105
   Lys Asp Gly Arg Arg Asn Arg Thr Phe Ile Leu Thr Phe Ile Arg Ile
   350                 355                     360                 365
   cct ttg gtt gct cga aat gag tgc gtg gag gtc atg aag aat gtg gtc    1153
   Pro Leu Val Ala Arg Asn Glu Cys Val Glu Val Met Lys Asn Val Val
                   370                     375                 380
   tcg gag aac atg ctg tgt gca ggc atc att ggg aac acg aga gac gcc      1201
   Ser Glu Asn Met Leu Cys Ala Gly Ile Ile Gly Asn Thr Arg Asp Ala
               385                 390                 395
   tgt gat ggt gac agt ggg ggg ccc atg gtg gtc ttc ttt cgg ggt acc      1249
   Cys Asp Gly Asp Ser Gly Gly Pro Met Val Val Phe Phe Arg Gly Thr
           400                 405                 410
   tgg ttc ctg gtg ggc ctg gtg agc tgg ggt gag ggc tgt ggg cac acc      1297
   Trp Phe Leu Val Gly Leu Val Ser Trp Gly Glu Gly Cys Gly His Thr
       415                 420                 425
   aac aac tat ggc atc tac acc aaa gtg gga agc tac ctc aaa tgg att      1345
   Asn Asn Tyr Gly Ile Tyr Thr Lys Val Gly Ser Tyr Leu Lys Trp Ile
   430                 435                 440                 445
   cac agt tac att ggg gaa aag ggt gtc tcc ctt aag agc cag aag cta      1393
   His Ser Tyr Ile Gly Glu Lys Gly Val Ser Leu Lys Ser Gln Lys Leu
                   450                 455                 460
   tag cacccctccc tgctcacctc tggaccctag aagtcactct tggagtaagg           1446
   ctgggctagt gagtaccaag acagaggaca ttaaaggagc atgcaacaaa cat           1499
<210> 8
   <211> 461
   <212> PRT
   <213> Mus musculus
<400> 8
   Met Trp Gln Phe Arg Val Phe Leu Leu Leu Met Ser Thr Trp Gly Ile
   1               5                   10                  15
   Ser Ser Ile Pro Ala His Pro Asp Pro Val Phe Ser Ser Ser Glu His
               20                  25                  30
   Ala His Gln Val Leu Arg Val Arg Arg Ala Asn Ser Phe Leu Glu Glu
           35                  40                  45
   Met Arg Pro Gly Ser Leu Glu Arg Glu Cys Met Glu Glu Ile Cys Asp
       50                  55                  60
   Phe Glu Glu Ala Gln Glu Ile Phe Gln Asn Val Glu Asp Thr Leu Ala
   65                  70                  75                  80
   Phe Trp Ile Lys Tyr Phe Asp Gly Asp Gln Cys Ser Ala Pro Pro Leu
                   85                  90                  95
   Asp His Gln Cys Asp Ser Pro Cys Cys Gly His Gly Thr Cys Ile Asp
               100                 105                 110
   Gly Ile Gly Ser Phe Ser Cys Ser Cys Asp Lys Gly Trp Glu Gly Lys
           115                 120                 125
   Phe Cys Gln Gln Glu Leu Arg Phe Gln Asp Cys Arg Val Asn Asn Gly
       130                 135                 140
   Gly Cys Leu His Tyr Cys Leu Glu Glu Ser Asn Gly Arg Arg Cys Ala
   145 150 155 160
   Cys Ala Pro Gly Tyr Glu Leu Ala Asp Asp His Met Arg Cys Lys Ser
   165 170 175
   Thr Val Asn Phe Pro Cys Gly Lys Leu Gly Arg Trp Ile Glu Lys Lys
   180 185 190
   Arg Lys Ile Leu Lys Arg Asp Thr Asp Leu Glu Asp Glu Leu Glu Pro
   195 200 205
   Asp Pro Arg Ile Val Asn Gly Thr Leu Thr Lys Gln Gly Asp Ser Pro
   210 215 220
   Trp Gln Ala Ile Leu Leu Asp Ser Lys Lys Lys Leu Ala Cys Gly Gly
   225 230 235 240
   Val Leu Ile His Thr Ser Trp Val Leu Thr Ala Ala His Cys Val Glu
   245 250 255
   Gly Thr Lys Lys Leu Thr Val Arg Leu Gly Glu Tyr Asp Leu Arg Arg
   260 265 270
   Arg Asp His Trp Glu Leu Asp Leu Asp Ile Lys Glu Ile Leu Val His
   275 280 285
   Pro Asn Tyr Thr Arg Ser Ser Ser Asp Asn Asp Ile Ala Leu Leu Arg
   290 295 300
   Leu Ala Gln Pro Ala Thr Leu Ser Lys Thr Ile Val Pro Ile Cys Leu
   305 310 315 320
   Pro Asn Asn Gly Leu Ala Gln Gln Glu Leu Thr Gln Ala Gly Gln Glu
   325 330 335
   Thr Val Val Thr Gly Trp Gly Tyr Gln Ser Asp Arg Ile Lys Asp Gly
   340 345 350
   Arg Arg Asn Arg Thr Phe Ile Leu Thr Phe Ile Arg Ile Pro Leu Val
   355 360 365
   Ala Arg Asn Glu Cys Val Glu Val Met Lys Asn Val Val Ser Glu Asn
   370 375 380
   Met Leu Cys Ala Gly Ile Ile Gly Asn Thr Arg Asp Ala Cys Asp Gly
   385 390 395 400
   Asp Ser Gly Gly Pro Met Val Val Phe Phe Arg Gly Thr Trp Phe Leu
   405 410 415
   Val Gly Leu Val Ser Trp Gly Glu Gly Cys Gly His Thr Asn Asn Tyr
                   420                     425                     430
   Gly Ile Tyr Thr Lys Val Gly Ser Tyr Leu Lys Trp Ile His Ser Tyr
           435                     440                     445
   Ile Gly Glu Lys Gly Val Ser Leu Lys Ser Gln Lys Leu
       450                     455                     460
<210> 9
   <211> 1661
   <212> DNA
   <213> Mus musculus
<220>
   <221> CDS
   <222> (163)..(1545)
<400> 9
   ggttatggat taacctgact cccagactga catggcgcta cctggacgaa attgcagttt      60
   ctccttggcc cacgcctgtg taggcttgtg acaagccgcg tatctcctcc aagcctttgg     120
   gtcccttccc atgaaatgga gacaggtgtc agcagctcca gg atg tgg caa ttc        174
                                                  Met Trp Gln Phe
                                                  1
   aga gtc ttc ctg ctg ctc atg tcc acc tgg gga ata tct agc ata ccg       222
   Arg Val Phe Leu Leu Leu Met Ser Thr Trp Gly Ile Ser Ser Ile Pro
   5                   10                  15                  20
   gcc cat cct gac cca gtg ttc tcc agc agc gag cat gcc cac cag gtg       270
   Ala His Pro Asp Pro Val Phe Ser Ser Ser Glu His Ala His Gln Val
                   25                  30                  35
   ctt cgg gtc aga cgt gcc aac agc ttc ctg gaa gag atg cgg cca ggc       318
   Leu Arg Val Arg Arg Ala Asn Ser Phe Leu Glu Glu Met Arg Pro Gly
               40                  45                  50
   agc ctg gaa cgg gag tgt atg gag gag atc tgt gac ttc gag gag gcc       366
   Ser Leu Glu Arg Glu Cys Met Glu Glu Ile Cys Asp Phe Glu Glu Ala
           55                  60                  65
   cag gag att ttc caa aat gtg gaa gac aca ctg gcc ttc tgg atc aag       414
   Gln Glu Ile Phe Gln Asn Val Glu Asp Thr Leu Ala Phe Trp Ile Lys
       70                  75                  80
   tac ttt gac ggt gac cag tgc tcg gct cca ccc ttg gac cac cag tgc       462
   Tyr Phe Asp Gly Asp Gln Cys Ser Ala Pro Pro Leu Asp His Gln Cys
   85                  90                  95                  100
   gac agc cca tgc tgc ggg cat ggc act tgc atc gac ggc ata ggc agc       510
   Asp Ser Pro Cys Cys Gly His Gly Thr Cys Ile Asp Gly Ile Gly Ser
                   105                 110                 115
   ttc agc tgc agc tgc gat aag ggc tgg gag ggc aag ttc tgt cag cag       558
   Phe Ser Cys Ser Cys Asp Lys Gly Trp Glu Gly Lys Phe Cys Gln Gln
               120                 125                 130
   gag ttg cgc ttc cag gac tgt cgg gtg aac aat ggc ggc tgc ttg cac       606
   Glu Leu Arg Phe Gln Asp Cys Arg Val Asn Asn Gly Gly Cys Leu His
           135                 140                 145
   tac tgc ctg gag gag agc aat ggg cgg cgc tgc gct tgt gcc ccg ggc       654
   Tyr Cys Leu Glu Glu Ser Asn Gly Arg Arg Cys Ala Cys Ala Pro Gly
       150                 155                 160
   tat gag ctg gca gac gac cac atg cgc tgc aag tcc act gtg aat ttt     702
   Tyr Glu Leu Ala Asp Asp His Met Arg Cys Lys Ser Thr Val Asn Phe
   165                 170                 175                 180
   cca tgt ggg aaa ctg ggg agg tgg ata gag aag aaa cgc aag atc ctc     750
   Pro Cys Gly Lys Leu Gly Arg Trp Ile Glu Lys Lys Arg Lys Ile Leu
                   185                 190                 195
   aaa cga gac aca gac tta gaa gat gaa ctg gaa cca gat cca agg ata     798
   Lys Arg Asp Thr Asp Leu Glu Asp Glu Leu Glu Pro Asp Pro Arg Ile
               200                 205                 210
   gtc aac gga acg ctg acg aag cag ggt gac agt cct tgg cag gca atc     846
   Val Asn Gly Thr Leu Thr Lys Gln Gly Asp Ser Pro Trp Gln Ala Ile
           215                 220                 225
   ctt ctg gac tcc aag aag aag ctg gcc tgc gga ggg gtg ctc atc cac     894
   Leu Leu Asp Ser Lys Lys Lys Leu Ala Cys Gly Gly Val Leu Ile His
       230                 235                 240
   act tcc tgg gtg ctg acg gca gcc cac tgc gtg gag ggc acc aag aag     942
   Thr Ser Trp Val Leu Thr Ala Ala His Cys Val Glu Gly Thr Lys Lys
   245                 250                 255                 260
   ctt acc gtg agg ctt ggt gag tat gat ctg cga cgc agg gac cac tgg     990
   Leu Thr Val Arg Leu Gly Glu Tyr Asp Leu Arg Arg Arg Asp His Trp
                   265                 270                 275
   gag ctg gac ctg gac atc aag gag atc ctc gtc cac cct aac tac acc    1038
   Glu Leu Asp Leu Asp Ile Lys Glu Ile Leu Val His Pro Asn Tyr Thr
               280                 285                 290
   cgg agc agc agt gac aac gac att gct ctg ctc cgc cta gcc cag cca    1086
   Arg Ser Ser Ser Asp Asn Asp Ile Ala Leu Leu Arg Leu Ala Gln Pro
           295                 300                 305
   gcc act ctc tcc aaa acc ata gtg ccc atc tgc ctg ccg aac aat ggg    1134
   Ala Thr Leu Ser Lys Thr Ile Val Pro Ile Cys Leu Pro Asn Asn Gly
       310                 315                 320
   ctg gcg cag gag ctc act cag gct ggc cag gag aca gtg gtg aca ggc    1182
   Leu Ala Gln Glu Leu Thr Gln Ala Gly Gln Glu Thr Val Val Thr Gly
   325                 330                 335                 340
   tgg ggc tat caa agc gac aga atc aag gat ggc aga agg aac cgc acc    1230
   Trp Gly Tyr Gln Ser Asp Arg Ile Lys Asp Gly Arg Arg Asn Arg Thr
                   345                 350                 355
   ttc atc ctc acc ttc atc cgc atc cct ttg gtt gct cga aat gag tgc    1278
   Phe Ile Leu Thr Phe Ile Arg Ile Pro Leu Val Ala Arg Asn Glu Cys
               360                 365                 370
   gtg gag gtc atg aag aat gtg gtc tcg gag aac atg ctg tgt gca ggc    1326
   Val Glu Val Met Lys Asn Val Val Ser Glu Asn Met Leu Cys Ala Gly
           375                 380                 385
   atc att ggg gac acg aga gac gcc tgt gat ggt gac agt ggg ggg ccc    1374
   Ile Ile Gly Asp Thr Arg Asp Ala Cys Asp Gly Asp Ser Gly Gly Pro
       390                 395                 400
   atg gtg gtc ttc ttt cgg ggt acc tgg ttc ctg gtg ggc ctg gtg agc    1422
   Met Val Val Phe Phe Arg Gly Thr Trp Phe Leu Val Gly Leu Val Ser
   405                 410                 415                 420
   tgg ggt gag ggc tgt ggg cac acc aac aac tat ggc atc tac acc aaa    1470
   Trp Gly Glu Gly Cys Gly His Thr Asn Asn Tyr Gly Ile Tyr Thr Lys
                   425                 430                 435
   gtg gga agc tac ctc aaa tgg att cac agt tac att ggg gaa aag ggt      1518
   Val Gly Ser Tyr Leu Lys Trp Ile His Ser Tyr Ile Gly Glu Lys Gly
               440                 445                 450
   gtc tcc ctt aag agc cag aag cta tag cacccctccc tgctcacctc            1565
   Val Ser Leu Lys Ser Gln Lys Leu
           455                 460
   tggaccctag aagtcactct tggagtaagg ctgggctagt gagtaccaag acagaggaca    1625
   ttaaaggagc atgcaacaaa cataaaaaaa aaaaaa                              1661
<210> 10
   <211> 460
   <212> PRT
   <213> Mus musculus
<400> 10
   Met Trp Gln Phe Arg Val Phe Leu Leu Leu Met Ser Thr Trp Gly Ile
   1               5                   10                  15
   Ser Ser Ile Pro Ala His Pro Asp Pro Val Phe Ser Ser Ser Glu His
               20                  25                  30
   Ala His Gln Val Leu Arg Val Arg Arg Ala Asn Ser Phe Leu Glu Glu
           35                  40                  45
   Met Arg Pro Gly Ser Leu Glu Arg Glu Cys Met Glu Glu Ile Cys Asp
       50                  55                  60
   Phe Glu Glu Ala Gln Glu Ile Phe Gln Asn Val Glu Asp Thr Leu Ala
   65                  70                  75                  80
   Phe Trp Ile Lys Tyr Phe Asp Gly Asp Gln Cys Ser Ala Pro Pro Leu
                   85                  90                  95
   Asp His Gln Cys Asp Ser Pro Cys Cys Gly His Gly Thr Cys Ile Asp
               100                 105                 110
   Gly Ile Gly Ser Phe Ser Cys Ser Cys Asp Lys Gly Trp Glu Gly Lys
           115                 120                 125
   Phe Cys Gln Gln Glu Leu Arg Phe Gln Asp Cys Arg Val Asn Asn Gly
       130                 135                 140
   Gly Cys Leu His Tyr Cys Leu Glu Glu Ser Asn Gly Arg Arg Cys Ala
   145                 150                 155                 160
   Cys Ala Pro Gly Tyr Glu Leu Ala Asp Asp His Met Arg Cys Lys Ser
                   165                 170                 175
   Thr Val Asn Phe Pro Cys Gly Lys Leu Gly Arg Trp Ile Glu Lys Lys
               180                 185                 190
   Arg Lys Ile Leu Lys Arg Asp Thr Asp Leu Glu Asp Glu Leu Glu Pro
   195 200 205
   Asp Pro Arg Ile Val Asn Gly Thr Leu Thr Lys Gln Gly Asp Ser Pro
   210 215 220
   Trp Gln Ala Ile Leu Leu Asp Ser Lys Lys Lys Leu Ala Cys Gly Gly
   225 230 235 240
   Val Leu Ile His Thr Ser Trp Val Leu Thr Ala Ala His Cys Val Glu
   245 250 255
   Gly Thr Lys Lys Leu Thr Val Arg Leu Gly Glu Tyr Asp Leu Arg Arg
   260 265 270
   Arg Asp His Trp Glu Leu Asp Leu Asp Ile Lys Glu Ile Leu Val His
   275 280 285
   Pro Asn Tyr Thr Arg Ser Ser Ser Asp Asn Asp Ile Ala Leu Leu Arg
   290 295 300
   Leu Ala Gln Pro Ala Thr Leu Ser Lys Thr Ile Val Pro Ile Cys Leu
   305 310 315 320
   Pro Asn Asn Gly Leu Ala Gln Glu Leu Thr Gln Ala Gly Gln Glu Thr
   325 330 335
   Val Val Thr Gly Trp Gly Tyr Gln Ser Asp Arg Ile Lys Asp Gly Arg
   340 345 350
   Arg Asn Arg Thr Phe Ile Leu Thr Phe Ile Arg Ile Pro Leu Val Ala
   355 360 365
   Arg Asn Glu Cys Val Glu Val Met Lys Asn Val Val Ser Glu Asn Met
   370 375 380
   Leu Cys Ala Gly Ile Ile Gly Asp Thr Arg Asp Ala Cys Asp Gly Asp
   385 390 395 400
   Ser Gly Gly Pro Met Val Val Phe Phe Arg Gly Thr Trp Phe Leu Val
   405 410 415
   Gly Leu Val Ser Trp Gly Glu Gly Cys Gly His Thr Asn Asn Tyr Gly
   420 425 430
   Ile Tyr Thr Lys Val Gly Ser Tyr Leu Lys Trp Ile His Ser Tyr Ile
   435 440 445
   Gly Glu Lys Gly Val Ser Leu Lys Ser Gln Lys Leu
   450 455 460
<210> 11
   <211> 1543
   <212> DNA
   <213> Rat t us nor vegi cus
<220>
   <221> CDS
   <222> (49)..(1434)
<400> 11
   cgaaattgca gtttctcctt ggcccacccc tgtgtcagca gctccagg atg tgg cag      57
                                                        Met Trp Gln
                                                        1
   ttc aga atc ttc ctg ctg ttc gcg tcc acc tgg ggg att tct ggc gta      105
   Phe Arg Ile Phe Leu Leu Phe Ala Ser Thr Trp Gly Ile Ser Gly Val
       5                   10                  15
   tca gcc cat ccc gac cca gtg ttc tcc agc agc gag ggt gcc cac cag      153
   Ser Ala His Pro Asp Pro Val Phe Ser Ser Ser Glu Gly Ala His Gln
   20                  25                  30                  35
   gtg ctt cgg gtc aga cga gcc aac agc ttc ctg gag gag gtg cgg gca      201
   Val Leu Arg Val Arg Arg Ala Asn Ser Phe Leu Glu Glu Val Arg Ala
                   40                  45                  50
   ggc agc ctg gag cgg gag tgt atg gag gag atc tgt gac ttc gag gag      249
   Gly Ser Leu Glu Arg Glu Cys Met Glu Glu Ile Cys Asp Phe Glu Glu
               55                  60                  65
   gcc cag gag att ttc cag aat gtg gaa gac aca ctg gcc ttt tgg atc      297
   Ala Gln Glu Ile Phe Gln Asn Val Glu Asp Thr Leu Ala Phe Trp Ile
           70                  75                  80
   aag tac ttc gat ggt gac cag tgc tca act ccg ccc ttg gac cac caa      345
   Lys Tyr Phe Asp Gly Asp Gln Cys Ser Thr Pro Pro Leu Asp His Gln
       85                  90                  95
   tgc gac agc cca tgc tgc ggc cat ggc aca tgc atc gac ggc ctg ggc      393
   Cys Asp Ser Pro Cys Cys Gly His Gly Thr Cys Ile Asp Gly Leu Gly
   100                 105                 110                 115
   ggc ttc agc tgc agc tgc gat aag ggc tgg gag ggc agg ttc tgt cag      441
   Gly Phe Ser Cys Ser Cys Asp Lys Gly Trp Glu Gly Arg Phe Cys Gln
                   120                 125                 130
   cag gag atg ggc ttc cag gac tgt cgg gtg aaa aat ggc ggc tgc tac      489
   Gln Glu Met Gly Phe Gln Asp Cys Arg Val Lys Asn Gly Gly Cys Tyr
               135                 140                 145
   cac tac tgc ctg gag gag acc aga ggg cgg cgc tgc cgt tgc gcc ccg      537
   His Tyr Cys Leu Glu Glu Thr Arg Gly Arg Arg Cys Arg Cys Ala Pro
           150                 155                 160
   ggc tat gag ctg gca gat gac cac atg cac tgc agg ccc acc gtg aat      585
   Gly Tyr Glu Leu Ala Asp Asp His Met His Cys Arg Pro Thr Val Asn
       165                 170                 175
   ttt ccg tgt ggg aaa ctg tgg aag cgg act gac aag aaa cgc aag aac      633
   Phe Pro Cys Gly Lys Leu Trp Lys Arg Thr Asp Lys Lys Arg Lys Asn
   180                 185                 190                 195
   ttc aaa cgg gac ata gac cca gaa gac gaa gaa cta gaa cta ggt cca      681
   Phe Lys Arg Asp Ile Asp Pro Glu Asp Glu Glu Leu Glu Leu Gly Pro
                   200                 205                 210
   agg ata gtc aat gga aca cta aca aag cag ggt gac agt ccc tgg cag      729
   Arg Ile Val Asn Gly Thr Leu Thr Lys Gln Gly Asp Ser Pro Trp Gln
               215                 220                 225
   gcg atc ctt ctg gac tcc aag aag aag cta gcc tgt gga ggg gtg ctc      777
   Ala Ile Leu Leu Asp Ser Lys Lys Lys Leu Ala Cys Gly Gly Val Leu
           230                 235                 240
   atc cac acc tcc tgg gtg ctg acg gca gcc cac tgt ctg gag agc agc      825
   Ile His Thr Ser Trp Val Leu Thr Ala Ala His Cys Leu Glu Ser Ser
       245                 250                 255
   aag aag ctt acc gtg agg ctt ggt gag tat gat ctg aga cgc agg gac      873
   Lys Lys Leu Thr Val Arg Leu Gly Glu Tyr Asp Leu Arg Arg Arg Asp
   260                 265                 270                 275
   ccc tgg gag ttg gac ctg gac atc aag gag gtc ctc gtc cac cct aac      921
   Pro Trp Glu Leu Asp Leu Asp Ile Lys Glu Val Leu Val His Pro Asn
                   280                 285                 290
   tac acc cgg agc aac agc gac aac gac atc gcc ctg ctc cgc ctg tcc      969
   Tyr Thr Arg Ser Asn Ser Asp Asn Asp Ile Ala Leu Leu Arg Leu Ser
               295                 300                 305
   cag cca gcc aca ctc tct aaa acc ata gtg ccc atc tgt ctg ccg aac     1017
   Gln Pro Ala Thr Leu Ser Lys Thr Ile Val Pro Ile Cys Leu Pro Asn
           310                 315                 320
   agc ggc ctg gcg cag gag ctc agt cag gct ggc cag gag acg gtg gtg     1065
   Ser Gly Leu Ala Gln Glu Leu Ser Gln Ala Gly Gln Glu Thr Val Val
       325                 330                 335
   aca ggc tgg ggc tat caa agc gac aaa gtc aag gat ggc aga agg aac     1113
   Thr Gly Trp Gly Tyr Gln Ser Asp Lys Val Lys Asp Gly Arg Arg Asn
   340                 345                 350                 355
   cgc acc ttt att ctc acc ttc atc cgc atc cct ttg gcc gct cga aat     1161
   Arg Thr Phe Ile Leu Thr Phe Ile Arg Ile Pro Leu Ala Ala Arg Asn
                   360                 365                 370
   gac tgc atg cag gtc atg aac aac gtg gtc tcg gag aac atg ctc tgc     1209
   Asp Cys Met Gln Val Met Asn Asn Val Val Ser Glu Asn Met Leu Cys
               375                 380                 385
   gcc ggc atc att gga gac acg aga gac gcc tgc gac ggc gac agt ggg     1257
   Ala Gly Ile Ile Gly Asp Thr Arg Asp Ala Cys Asp Gly Asp Ser Gly
           390                 395                 400
   gga cct atg gtg gtc ttc ttt cgg ggt acc tgg ttt ctg gtg ggc ctg     1305
   Gly Pro Met Val Val Phe Phe Arg Gly Thr Trp Phe Leu Val Gly Leu
       405                 410                 415
   gtg agc tgg ggt gag ggc tgt ggg cac ctc aac aac tat ggc gtc tac     1353
   Val Ser Trp Gly Glu Gly Cys Gly His Leu Asn Asn Tyr Gly Val Tyr
   420                 425                 430                 435
   acc aaa gtg ggt agc tac ctc aaa tgg atc cac agc tac ata ggg gaa     1401
   Thr Lys Val Gly Ser Tyr Leu Lys Trp Ile His Ser Tyr Ile Gly Glu
                   440                 445                 450
   agg gat gtt tcc ctg aag agc ccg aag ctg tag catccctccc tgctcatctc   1454
   Arg Asp Val Ser Leu Lys Ser Pro Lys Leu
               455                 460
   tggggcccag aggtcactct tagaataagg ctgggctagt gagtaccaag acaggggaca   1514
   ttaaaggggc aagcaacacc tgaaaaaaa                                     1543
<210> 12
   <211> 461
   <212> PRT
   <213> Rattus norvegicus
<400> 12
   Met Trp Gln Phe Arg Ile Phe Leu Leu Phe Ala Ser Thr Trp Gly Ile
   1 5 10 15
   Ser Gly Val Ser Ala His Pro Asp Pro Val Phe Ser Ser Ser Glu Gly
   20 25 30
   Ala His Gln Val Leu Arg Val Arg Arg Ala Asn Ser Phe Leu Glu Glu
   35 40 45
   Val Arg Ala Gly Ser Leu Glu Arg Glu Cys Met Glu Glu Ile Cys Asp
   50 55 60
   Phe Glu Glu Ala Gln Glu Ile Phe Gln Asn Val Glu Asp Thr Leu Ala
   65 70 75 80
   Phe Trp Ile Lys Tyr Phe Asp Gly Asp Gln Cys Ser Thr Pro Pro Leu
   85 90 95
   Asp His Gln Cys Asp Ser Pro Cys Cys Gly His Gly Thr Cys Ile Asp
   100 105 110
   Gly Leu Gly Gly Phe Ser Cys Ser Cys Asp Lys Gly Trp Glu Gly Arg
   115 120 125
   Phe Cys Gln Gln Glu Met Gly Phe Gln Asp Cys Arg Val Lys Asn Gly
   130 135 140
   Gly Cys Tyr His Tyr Cys Leu Glu Glu Thr Arg Gly Arg Arg Cys Arg
   145 150 155 160
   Cys Ala Pro Gly Tyr Glu Leu Ala Asp Asp His Met His Cys Arg Pro
   165 170 175
   Thr Val Asn Phe Pro Cys Gly Lys Leu Trp Lys Arg Thr Asp Lys Lys
   180 185 190
   Arg Lys Asn Phe Lys Arg Asp Ile Asp Pro Glu Asp Glu Glu Leu Glu
   195 200 205
   Leu Gly Pro Arg Ile Val Asn Gly Thr Leu Thr Lys Gln Gly Asp Ser
   210 215 220
   Pro Trp Gln Ala Ile Leu Leu Asp Ser Lys Lys Lys Leu Ala Cys Gly
   225 230 235 240
   Gly Val Leu Ile His Thr Ser Trp Val Leu Thr Ala Ala His Cys Leu
   245 250 255
   Glu Ser Ser Lys Lys Leu Thr Val Arg Leu Gly Glu Tyr Asp Leu Arg
   260 265 270
   Arg Arg Asp Pro Trp Glu Leu Asp Leu Asp Ile Lys Glu Val Leu Val
   275 280 285
   His Pro Asn Tyr Thr Arg Ser Asn Ser Asp Asn Asp Ile Ala Leu Leu
   290 295 300
   Arg Leu Ser Gln Pro Ala Thr Leu Ser Lys Thr Ile Val Pro Ile Cys
   305 310 315 320
   Leu Pro Asn Ser Gly Leu Ala Gln Glu Leu Ser Gln Ala Gly Gln Glu
   325 330 335
   Thr Val Val Thr Gly Trp Gly Tyr Gln Ser Asp Lys Val Lys Asp Gly
   340 345 350
   Arg Arg Asn Arg Thr Phe Ile Leu Thr Phe Ile Arg Ile Pro Leu Ala
   355 360 365
   Ala Arg Asn Asp Cys Met Gln Val Met Asn Asn Val Val Ser Glu Asn
   370 375 380
   Met Leu Cys Ala Gly Ile Ile Gly Asp Thr Arg Asp Ala Cys Asp Gly
   385 390 395 400
   Asp Ser Gly Gly Pro Met Val Val Phe Phe Arg Gly Thr Trp Phe Leu
   405 410 415
   Val Gly Leu Val Ser Trp Gly Glu Gly Cys Gly His Leu Asn Asn Tyr
   420 425 430
   Gly Val Tyr Thr Lys Val Gly Ser Tyr Leu Lys Trp Ile His Ser Tyr
   435 440 445
   Ile Gly Glu Arg Asp Val Ser Leu Lys Ser Pro Lys Leu
   450 455 460
<210> 13
   <211> 1569
   <212> DNA
   <213> Bos taurus
<220>
   <221> CDS
   <222> (18)..(1460)
<400> 13
   tcgagttcca ggctgtc atg gcg gca gga ggg caa cct tgc agt ttc tcc 50
   Met Ala Ala Gly Gly Gln Pro Cys Ser Phe Ser
   1 5 10
   gcg gcc cac ccc agt ggc tgc agc ttc aga atg tgg cag ctt aca agc 98
   Ala Ala His Pro Ser Gly Cys Ser Phe Arg Met Trp Gln Leu Thr Ser
   15 20 25
   ctc tta ctg ttc gtg acc atc tgg gga att tcc agc aca cca gct cct      146
   Leu Leu Leu Phe Val Thr Ile Trp Gly Ile Ser Ser Thr Pro Ala Pro
           30                  35                  40
   cct gac tca gtg ttc tcc agc agc cag cgt gcc cac caa gtg ctg cgg      194
   Pro Asp Ser Val Phe Ser Ser Ser Gln Arg Ala His Gln Val Leu Arg
       45                  50                  55
   atc cgc aaa cgt gcc aac tcc ttc ctg gag gag ctg cgg ccc ggc aac      242
   Ile Arg Lys Arg Ala Asn Ser Phe Leu Glu Glu Leu Arg Pro Gly Asn
   60                  65                  70                  75
   gtg gag cgt gag tgc tca gag gag gtc tgt gag ttc gag gaa gct cgg      290
   Val Glu Arg Glu Cys Ser Glu Glu Val Cys Glu Phe Glu Glu Ala Arg
                   80                  85                  90
   gag att ttc caa aac acg gaa gac aca atg gcc ttc tgg tcc aag tat      338
   Glu Ile Phe Gln Asn Thr Glu Asp Thr Met Ala Phe Trp Ser Lys Tyr
               95                  100                 105
   agc gac ggg gac cag tgc gag gac cgg ccc tca ggg agc ccg tgc gac      386
   Ser Asp Gly Asp Gln Cys Glu Asp Arg Pro Ser Gly Ser Pro Cys Asp
           110                 115                 120
   ctc cca tgc tgt gga cgc ggc aag tgc atc gat ggc ctg ggc ggc ttc      434
   Leu Pro Cys Cys Gly Arg Gly Lys Cys Ile Asp Gly Leu Gly Gly Phe
       125                 130                 135
   cgc tgc gac tgc gcg gag ggc tgg gag ggc cgc ttc tgc ttg cac gag      482
   Arg Cys Asp Cys Ala Glu Gly Trp Glu Gly Arg Phe Cys Leu His Glu
   140                 145                 150                 155
   gtg cgc ttc tcc aac tgc tcg gcg gaa aac ggc ggc tgc gcc cac tac      530
   Val Arg Phe Ser Asn Cys Ser Ala Glu Asn Gly Gly Cys Ala His Tyr
                   160                 165                 170
   tgc atg gag gag gag ggc cgg cgc cac tgc agc tgc gcg ccc ggc tac      578
   Cys Met Glu Glu Glu Gly Arg Arg His Cys Ser Cys Ala Pro Gly Tyr
               175                 180                 185
   cgg ctg gag gac gac cac cag ctc tgc gtg tcc aag gtg acg ttc cct      626
   Arg Leu Glu Asp Asp His Gln Leu Cys Val Ser Lys Val Thr Phe Pro
           190                 195                 200
   tgt ggg agg cta ggg aag cga atg gag aag aaa cgc aag acc ttg aaa      674
   Cys Gly Arg Leu Gly Lys Arg Met Glu Lys Lys Arg Lys Thr Leu Lys
       205                 210                 215
   cgt gac aca aac caa gtc gac caa aaa gac cag ttg gat cca cgg att      722
   Arg Asp Thr Asn Gln Val Asp Gln Lys Asp Gln Leu Asp Pro Arg Ile
   220                 225                 230                 235
   gtc gat ggg cag gag gct gga tgg gga gag agc ccc tgg cag gca gtg      770
   Val Asp Gly Gln Glu Ala Gly Trp Gly Glu Ser Pro Trp Gln Ala Val
                   240                 245                 250
   ctg ctg gac tcc aag aag aag ctg gtc tgt ggg gca gtg cta atc cac      818
   Leu Leu Asp Ser Lys Lys Lys Leu Val Cys Gly Ala Val Leu Ile His
               255                 260                 265
   gtc tcc tgg gtg ctg acg gtg gcc cac tgc ttg gac agc cgc aag aag      866
   Val Ser Trp Val Leu Thr Val Ala His Cys Leu Asp Ser Arg Lys Lys
           270                 275                 280
   ctc atc gtc agg ctc ggg gag tat gac atg cgg cgc tgg gag agc tgg      914
   Leu Ile Val Arg Leu Gly Glu Tyr Asp Met Arg Arg Trp Glu Ser Trp
       285                 290                 295
   gag gtg gac ctg gac atc aag gag gtc atc atc cac cct aac tat acc      962
   Glu Val Asp Leu Asp Ile Lys Glu Val Ile Ile His Pro Asn Tyr Thr
   300 305 310 315
   aag agc acc agt gac aac gac atc gcc ctg ctc cgc ctg gcc aag cct 1010
   Lys Ser Thr Ser Asp Asn Asp Ile Ala Leu Leu Arg Leu Ala Lys Pro
   320 325 330
   gcc act ctt tcg cag acc att gtg ccc atc tgt ctc ccg gat agt ggc 1058
   Ala Thr Leu Ser Gln Thr Ile Val Pro Ile Cys Leu Pro Asp Ser Gly
   335 340 345
   ctc tct gag cgc aag ctc acc cag gtc ggc cag gag act gtg gtg aca 1106
   Leu Ser Glu Arg Lys Leu Thr Gln Val Gly Gln Glu Thr Val Val Thr
   350 355 360
   ggc tgg ggc tac cgt gac gag acc aag aga aac cgc acc ttc gtc ctc 1154
   Gly Trp Gly Tyr Arg Asp Glu Thr Lys Arg Asn Arg Thr Phe Val Leu
   365 370 375
   agc ttc atc aag gtc cct gtg gtc ccg tac aat gca tgt gtc cat gcc 1202
   Ser Phe Ile Lys Val Pro Val Val Pro Tyr Asn Ala Cys Val His Ala
   380 385 390 395
   atg gaa aac aag atc tct gag aac atg ctg tgc gct ggt atc ctc ggg 1250
   Met Glu Asn Lys Ile Ser Glu Asn Met Leu Cys Ala Gly Ile Leu Gly
   400 405 410
   gac ccg agg gat gcc tgt gag ggc gac agt ggg ggg cct atg gtc acc 1298
   Asp Pro Arg Asp Ala Cys Glu Gly Asp Ser Gly Gly Pro Met Val Thr
   415 420 425
   ttc ttc cgt ggc acc tgg ttc ctg gtg ggc ctg gtg agc tgg ggc gag 1346
   Phe Phe Arg Gly Thr Trp Phe Leu Val Gly Leu Val Ser Trp Gly Glu
   430 435 440
   ggc tgt ggg cgc ctc tac aac tac ggc gtt tac acc aaa gtc agc cgt 1394
   Gly Cys Gly Arg Leu Tyr Asn Tyr Gly Val Tyr Thr Lys Val Ser Arg
   445 450 455
   tac ctt gac tgg atc tac ggc cac atc aaa gct cag gag gcc cct ctt 1442
   Tyr Leu Asp Trp Ile Tyr Gly His Ile Lys Ala Gln Glu Ala Pro Leu
   460 465 470 475
   gag agc cag gtg cct tag catcccccgt cacttgtgtc tgggccccag 1490
   Glu Ser Gln Val Pro
   480
   aggacaccct tggatagggg ctggattgtt gaatggcaag atggtggaca ttaaaaaagg 1550
   gcttgctgca agcacacca 1569
<210> 14
   <211> 480
   <212> PRT
   <213> Bos taurus
<400> 14
   Met Ala Ala Gly Gly Gln Pro Cys Ser Phe Ser Ala Ala His Pro Ser
   1 5 10 15
   Gly Cys Ser Phe Arg Met Trp Gln Leu Thr Ser Leu Leu Leu Phe Val
   20 25 30
   Thr Ile Trp Gly Ile Ser Ser Thr Pro Ala Pro Pro Asp Ser Val Phe
   35 40 45
   Ser Ser Ser Gln Arg Ala His Gln Val Leu Arg Ile Arg Lys Arg Ala
   50 55 60
   Asn Ser Phe Leu Glu Glu Leu Arg Pro Gly Asn Val Glu Arg Glu Cys
   65 70 75 80
   Ser Glu Glu Val Cys Glu Phe Glu Glu Ala Arg Glu Ile Phe Gln Asn
   85 90 95
   Thr Glu Asp Thr Met Ala Phe Trp Ser Lys Tyr Ser Asp Gly Asp Gln
   100 105 110
   Cys Glu Asp Arg Pro Ser Gly Ser Pro Cys Asp Leu Pro Cys Cys Gly
   115 120 125
   Arg Gly Lys Cys Ile Asp Gly Leu Gly Gly Phe Arg Cys Asp Cys Ala
   130 135 140
   Glu Gly Trp Glu Gly Arg Phe Cys Leu His Glu Val Arg Phe Ser Asn
   145 150 155 160
   Cys Ser Ala Glu Asn Gly Gly Cys Ala His Tyr Cys Met Glu Glu Glu
   165 170 175
   Gly Arg Arg His Cys Ser Cys Ala Pro Gly Tyr Arg Leu Glu Asp Asp
   180 185 190
   His Gln Leu Cys Val Ser Lys Val Thr Phe Pro Cys Gly Arg Leu Gly
   195 200 205
   Lys Arg Met Glu Lys Lys Arg Lys Thr Leu Lys Arg Asp Thr Asn Gln
   210 215 220
   Val Asp Gln Lys Asp Gln Leu Asp Pro Arg Ile Val Asp Gly Gln Glu
   225 230 235 240
   Ala Gly Trp Gly Glu Ser Pro Trp Gln Ala Val Leu Leu Asp Ser Lys
   245 250 255
   Lys Lys Leu Val Cys Gly Ala Val Leu Ile His Val Ser Trp Val Leu
   260 265 270
   Thr Val Ala His Cys Leu Asp Ser Arg Lys Lys Leu Ile Val Arg Leu
   275 280 285
   Gly Glu Tyr Asp Met Arg Arg Trp Glu Ser Trp Glu Val Asp Leu Asp
   290 295 300
   Ile Lys Glu Val Ile Ile His Pro Asn Tyr Thr Lys Ser Thr Ser Asp
   305 310 315 320
   Asn Asp Ile Ala Leu Leu Arg Leu Ala Lys Pro Ala Thr Leu Ser Gln
   325 330 335
   Thr Ile Val Pro Ile Cys Leu Pro Asp Ser Gly Leu Ser Glu Arg Lys
   340 345 350
   Leu Thr Gln Val Gly Gln Glu Thr Val Val Thr Gly Trp Gly Tyr Arg
   355 360 365
   Asp Glu Thr Lys Arg Asn Arg Thr Phe Val Leu Ser Phe Ile Lys Val
   370 375 380
   Pro Val Val Pro Tyr Asn Ala Cys Val His Ala Met Glu Asn Lys Ile
   385 390 395 400
   Ser Glu Asn Met Leu Cys Ala Gly Ile Leu Gly Asp Pro Arg Asp Ala
   405 410 415
   Cys Glu Gly Asp Ser Gly Gly Pro Met Val Thr Phe Phe Arg Gly Thr
   420 425 430
   Trp Phe Leu Val Gly Leu Val Ser Trp Gly Glu Gly Cys Gly Arg Leu
   435 440 445
   Tyr Asn Tyr Gly Val Tyr Thr Lys Val Ser Arg Tyr Leu Asp Trp Ile
   450 455 460
   Tyr Gly His Ile Lys Ala Gln Glu Ala Pro Leu Glu Ser Gln Val Pro
   465 470 475 480
<210> 15
   <211> 1514
   <212> DNA
   <213> Sus scr of a
<220>
   <221> CDS
   <222> (22)..(1401)
<400> 15
   cgttgctgtc gcaactccag a atg tgg caa ctt gca agc ctc tta ctg ctc 51
   Met Trp Gln Leu Ala Ser Leu Leu Leu Leu
   1 5 10
   ctg atc atc tgg gca gtt tcc agc aca cca gtt cct cct gac tca gtg 99
   Leu Ile Ile Trp Ala Val Ser Ser Thr Pro Val Pro Pro Asp Ser Val
   15 20 25
   ttc tcc agc agc cag cgg gcc cac cag atg ctg cgc agc aaa cgc gcc 147
   Phe Ser Ser Ser Gln Arg Ala His Gln Met Leu Arg Ser Lys Arg Ala
   30 35 40
   aac tcc ttc ctg gag gag ctg cgg ccc agc agc ctg gag cgt gag tgc 195
   Asn Ser Phe Leu Glu Glu Leu Arg Pro Ser Ser Leu Glu Arg Glu Cys
   45 50 55
   aag gag gag acc tgt gat ttc gag gag gct cgg gag att ttc caa aac 243
   Lys Glu Glu Thr Cys Asp Phe Glu Glu Ala Arg Glu Ile Phe Gln Asn
       60                  65                  70
   acg gaa aac aca atg gcc ttc tgg tcc aag tac cat gac ggg gac cag      291
   Thr Glu Asn Thr Met Ala Phe Trp Ser Lys Tyr His Asp Gly Asp Gln
   75                  80                  85                  90
   tgc gcg gtc tcg ccc cct gag cac ctg tgc gac agc ccg tgc tgc ggg      339
   Cys Ala Val Ser Pro Pro Glu His Leu Cys Asp Ser Pro Cys Cys Gly
                   95                  100                 105
   cgt ggc acc tgt atc gac ggc ctg ggc ggc ttc cgc tgc gac tgc gct      387
   Arg Gly Thr Cys Ile Asp Gly Leu Gly Gly Phe Arg Cys Asp Cys Ala
               110                 115                 120
   cag ggc tgg gag ggc cgc ttc tgc ctg cac gag gtg cgc ttc tcc aac      435
   Gln Gly Trp Glu Gly Arg Phe Cys Leu His Glu Val Arg Phe Ser Asn
           125                 130                 135
   tgc tcg acg gaa aac ggt ggc tgt gcg cac tac tgc ctg gag gag gag      483
   Cys Ser Thr Glu Asn Gly Gly Cys Ala His Tyr Cys Leu Glu Glu Glu
       140                 145                 150
   ggc ggg cgc cgc tgc gct tgc gcg cca ggc tac cgg ctg ggg gac gac      531
   Gly Gly Arg Arg Cys Ala Cys Ala Pro Gly Tyr Arg Leu Gly Asp Asp
   155                 160                 165                 170
   cac ctg cag tgc gag cca aag gtg agg tcc cct tgc ggg agg cta ggg      579
   His Leu Gln Cys Glu Pro Lys Val Arg Ser Pro Cys Gly Arg Leu Gly
                   175                 180                 185
   aat cgc atg gag aag aaa cgc aag aac ttg aag cgt gat aca gac caa      627
   Asn Arg Met Glu Lys Lys Arg Lys Asn Leu Lys Arg Asp Thr Asp Gln
               190                 195                 200
   gtt gac aaa aaa gaa gac caa ata gat ccg agg ctc gtc aat ggg aag      675
   Val Asp Lys Lys Glu Asp Gln Ile Asp Pro Arg Leu Val Asn Gly Lys
           205                 210                 215
   cag tcc cca tgg gga gag agc ccc tgg cag gtg atc ctg ctg gac tca      723
   Gln Ser Pro Trp Gly Glu Ser Pro Trp Gln Val Ile Leu Leu Asp Ser
       220                 225                 230
   aag aag aag ctg gcc tgt ggg gca gtg ctc atc cat gtc tcc tgg gtg      771
   Lys Lys Lys Leu Ala Cys Gly Ala Val Leu Ile His Val Ser Trp Val
   235                 240                 245                 250
   ctg aca gcg gcc cac tgc ttg gac gac tac aag aag ctc act gtc agg      819
   Leu Thr Ala Ala His Cys Leu Asp Asp Tyr Lys Lys Leu Thr Val Arg
                   255                 260                 265
   ctc ggc gaa tat gat ctg agg cgt cgg gaa aaa tgg gag gta gac ctg      867
   Leu Gly Glu Tyr Asp Leu Arg Arg Arg Glu Lys Trp Glu Val Asp Leu
               270                 275                 280
   gac atc aag gag ttc ctc gtc cac cct aac tac acc agg agc acc agt      915
   Asp Ile Lys Glu Phe Leu Val His Pro Asn Tyr Thr Arg Ser Thr Ser
           285                 290                 295
   gac aat gac atc gcc ctg ctc cgc ctg gcc gaa ccg gcc act ttc tcg      963
   Asp Asn Asp Ile Ala Leu Leu Arg Leu Ala Glu Pro Ala Thr Phe Ser
       300                 305                 310
   cag acc atc gtg ccc atc tgc ctc cca gac agt ggc ctc tct gag cgt      1011
   Gln Thr Ile Val Pro Ile Cys Leu Pro Asp Ser Gly Leu Ser Glu Arg
   315                 320                 325                 330
   gag ctc acc cgg gtt ggc caa gag acg gtg gtg acc ggc tgg ggc tac      1059
   Glu Leu Thr Arg Val Gly Gln Glu Thr Val Val Thr Gly Trp Gly Tyr
                       335                 340                 345
   cgc agc gag gcc aag aca aac cgc agc ttc atc ctc aac ttc atc aag      1107
   Arg Ser Glu Ala Lys Thr Asn Arg Ser Phe Ile Leu Asn Phe Ile Lys
               350                 355                 360
   gtc cct gtg gcc ccg cac aat gag tgc gtc cag gcc atg cac aac aag      1155
   Val Pro Val Ala Pro His Asn Glu Cys Val Gln Ala Met His Asn Lys
           365                 370                 375
   atc tct gag aac atg ctg tgt gca ggc atc ctg ggg gac tct cgt gat      1203
   Ile Ser Glu Asn Met Leu Cys Ala Gly Ile Leu Gly Asp Ser Arg Asp
       380                 385                 390
   gcc tgc gag ggc gac agc ggg ggg cct atg gtg gcc tcc ttc cgc ggc      1251
   Ala Cys Glu Gly Asp Ser Gly Gly Pro Met Val Ala Ser Phe Arg Gly
   395                 400                 405                 410
   acc tgg ttc ctg gtg ggc ctg gtg agc tgg ggt gag ggc tgt ggg cgc      1299
   Thr Trp Phe Leu Val Gly Leu Val Ser Trp Gly Glu Gly Cys Gly Arg
                   415                 420                 425
   ctc cac aac tat ggc gtt tac acc aaa gtc agc cgt tac ctc gac tgg      1347
   Leu His Asn Tyr Gly Val Tyr Thr Lys Val Ser Arg Tyr Leu Asp Trp
               430                 435                 440
   atc cat ggc cac atc aga atg gag gag gcc ttc cac aag aac cag gtg      1395
   Ile His Gly His Ile Arg Met Glu Glu Ala Phe His Lys Asn Gln Val
           445                 450                 455
   cct tag tgtctcccta ccccactgct tgggccccag gggccatcct cgggtggggg      1451
   Pro
   ctggtttgtt gaatggcaac agtaggacaa taaaggggca tgcagtaagc aaaaaaaaaa    1511
   aaa                                                                  1514
<210> 16
   <211> 459
   <212> PRT
   <213> Sus scr of a
<400> 16
   Met Trp Gln Leu Ala Ser Leu Leu Leu Leu Leu Ile Ile Trp Ala Val
   1               5                   10                  15
   Ser Ser Thr Pro Val Pro Pro Asp Ser Val Phe Ser Ser Ser Gln Arg
               20                  25                  30
   Ala His Gln Met Leu Arg Ser Lys Arg Ala Asn Ser Phe Leu Glu Glu
           35                  40                  45
   Leu Arg Pro Ser Ser Leu Glu Arg Glu Cys Lys Glu Glu Thr Cys Asp
       50                  55                  60
   Phe Glu Glu Ala Arg Glu Ile Phe Gln Asn Thr Glu Asn Thr Met Ala
   65                  70                  75                  80
   Phe Trp Ser Lys Tyr His Asp Gly Asp Gln Cys Ala Val Ser Pro Pro
                   85                  90                  95
   Glu His Leu Cys Asp Ser Pro Cys Cys Gly Arg Gly Thr Cys Ile Asp
   100 105 110
   Gly Leu Gly Gly Phe Arg Cys Asp Cys Ala Gln Gly Trp Glu Gly Arg
   115 120 125
   Phe Cys Leu His Glu Val Arg Phe Ser Asn Cys Ser Thr Glu Asn Gly
   130 135 140
   Gly Cys Ala His Tyr Cys Leu Glu Glu Glu Gly Gly Arg Arg Cys Ala
   145 150 155 160
   Cys Ala Pro Gly Tyr Arg Leu Gly Asp Asp His Leu Gln Cys Glu Pro
   165 170 175
   Lys Val Arg Ser Pro Cys Gly Arg Leu Gly Asn Arg Met Glu Lys Lys
   180 185 190
   Arg Lys Asn Leu Lys Arg Asp Thr Asp Gln Val Asp Lys Lys Glu Asp
   195 200 205
   Gln Ile Asp Pro Arg Leu Val Asn Gly Lys Gln Ser Pro Trp Gly Glu
   210 215 220
   Ser Pro Trp Gln Val Ile Leu Leu Asp Ser Lys Lys Lys Leu Ala Cys
   225 230 235 240
   Gly Ala Val Leu Ile His Val Ser Trp Val Leu Thr Ala Ala His Cys
   245 250 255
   Leu Asp Asp Tyr Lys Lys Leu Thr Val Arg Leu Gly Glu Tyr Asp Leu
   260 265 270
   Arg Arg Arg Glu Lys Trp Glu Val Asp Leu Asp Ile Lys Glu Phe Leu
   275 280 285
   Val His Pro Asn Tyr Thr Arg Ser Thr Ser Asp Asn Asp Ile Ala Leu
   290 295 300
   Leu Arg Leu Ala Glu Pro Ala Thr Phe Ser Gln Thr Ile Val Pro Ile
   305 310 315 320
   Cys Leu Pro Asp Ser Gly Leu Ser Glu Arg Glu Leu Thr Arg Val Gly
   325 330 335
   Gln Glu Thr Val Val Thr Gly Trp Gly Tyr Arg Ser Glu Ala Lys Thr
   340 345 350
   Asn Arg Ser Phe Ile Leu Asn Phe Ile Lys Val Pro Val Ala Pro His
   355 360 365
   Asn Glu Cys Val Gln Ala Met His Asn Lys Ile Ser Glu Asn Met Leu
       370                 375                 380
   Cys Ala Gly Ile Leu Gly Asp Ser Arg Asp Ala Cys Glu Gly Asp Ser
   385                 390                 395                 400
   Gly Gly Pro Met Val Ala Ser Phe Arg Gly Thr Trp Phe Leu Val Gly
                   405                 410                 415
   Leu Val Ser Trp Gly Glu Gly Cys Gly Arg Leu His Asn Tyr Gly Val
               420                 425                 430
   Tyr Thr Lys Val Ser Arg Tyr Leu Asp Trp Ile His Gly His Ile Arg
           435                 440                 445
   Met Glu Glu Ala Phe His Lys Asn Gln Val Pro
       450                 455
<210> 17
   <211> 1776
   <212> DNA
   <213> Homo sapiens
<220>
   <221> CDS
   <222> (74)..(1459)
<400> 17
   gctgtcatgg cggcaggacg gcgaacttgc agtatctcca cgacccgccc ctacaggtgc      60
   cagtgcctcc aga atg tgg cag ctc aca agc ctc ctg ctg ttc gtg gcc        109
                  Met Trp Gln Leu Thr Ser Leu Leu Leu Phe Val Ala
                  1               5                   10
   acc tgg gga att tcc ggc aca cca gct cct ctt gac tca gtg ttc tcc       157
   Thr Trp Gly Ile Ser Gly Thr Pro Ala Pro Leu Asp Ser Val Phe Ser
           15                  20                  25
   agc agc gag cgt gcc cac cag gtg ctg cgg atc cgc aaa cgt gcc aac       205
   Ser Ser Glu Arg Ala His Gln Val Leu Arg Ile Arg Lys Arg Ala Asn
       30                  35                  40
   tcc ttc ctg gag gag ctc cgt cac agc agc ctg gag cgg gag tgc ata       253
   Ser Phe Leu Glu Glu Leu Arg His Ser Ser Leu Glu Arg Glu Cys Ile
   45                  50                  55                  60
   gag gag atc tgt gac ttc gag gag gcc aag gaa att ttc caa aat gtg       301
   Glu Glu Ile Cys Asp Phe Glu Glu Ala Lys Glu Ile Phe Gln Asn Val
                   65                  70                  75
   gat gac aca ctg gcc ttc tgg tcc aag cac gtc gac ggt gac cag tgc       349
   Asp Asp Thr Leu Ala Phe Trp Ser Lys His Val Asp Gly Asp Gln Cys
               80                  85                  90
   ttg gtc ttg ccc ttg gag cac ccg tgc gcc agc ctg tgc tgc ggg cac       397
   Leu Val Leu Pro Leu Glu His Pro Cys Ala Ser Leu Cys Cys Gly His
           95                  100                 105
   ggc acg tgc atc gac ggc atc ggc agc ttc agc tgc gac tgc cgc agc       445
   Gly Thr Cys Ile Asp Gly Ile Gly Ser Phe Ser Cys Asp Cys Arg Ser
       110                 115                 120
   ggc tgg gag ggc cgc ttc tgc cag cgc gag gtg agc ttc ctc aat tgc      493
   Gly Trp Glu Gly Arg Phe Cys Gln Arg Glu Val Ser Phe Leu Asn Cys
   125                 130                 135                 140
   tcg ctg gac aac ggc ggc tgc acg cat tac tgc cta gag gag gtg ggc      541
   Ser Leu Asp Asn Gly Gly Cys Thr His Tyr Cys Leu Glu Glu Val Gly
                   145                 150                 155
   tgg cgg cgc tgt agc tgt gcg cct ggc tac aag ctg ggg gac gac ctc      589
   Trp Arg Arg Cys Ser Cys Ala Pro Gly Tyr Lys Leu Gly Asp Asp Leu
               160                 165                 170
   ctg cag tgt cac ccc gca gtg aag ttc cct tgt ggg agg ccc tgg aag      637
   Leu Gln Cys His Pro Ala Val Lys Phe Pro Cys Gly Arg Pro Trp Lys
           175                 180                 185
   cgg atg gag aag aag cgc agt cac ctg aaa cga gac aca gaa gac caa      685
   Arg Met Glu Lys Lys Arg Ser His Leu Lys Arg Asp Thr Glu Asp Gln
       190                 195                 200
   gaa gac caa gta gat ccg cgg ctc att gat ggg aag atg acc agg cgg      733
   Glu Asp Gln Val Asp Pro Arg Leu Ile Asp Gly Lys Met Thr Arg Arg
   205                 210                 215                 220
   gga gac agc ccc tgg cag gtg gtc ctg ctg gac tca aag aag aag ctg      781
   Gly Asp Ser Pro Trp Gln Val Val Leu Leu Asp Ser Lys Lys Lys Leu
                   225                 230                 235
   gcc tgc ggg gca gtg ctc atc cac ccc tcc tgg gtg ctg aca gcg gcc      829
   Ala Cys Gly Ala Val Leu Ile His Pro Ser Trp Val Leu Thr Ala Ala
               240                 245                 250
   cac tgc atg gat gag tcc aag aag ctc ctt gtc tgc ctt gga gag tat      877
   His Cys Met Asp Glu Ser Lys Lys Leu Leu Val Cys Leu Gly Glu Tyr
           255                 260                 265
   gac ctg cgg cgc tgg gag aag tgg gag ctg tgc ctg gac atc aag gag      925
   Asp Leu Arg Arg Trp Glu Lys Trp Glu Leu Cys Leu Asp Ile Lys Glu
       270                 275                 280
   gtc ttc gtc cac ccc aac tac agc aag agc acc acc gac aat gac atc      973
   Val Phe Val His Pro Asn Tyr Ser Lys Ser Thr Thr Asp Asn Asp Ile
   285                 290                 295                 300
   gca ctg ctg cac ctg gcc cag ccc gcc acc ctc tcg cag acc ata gtg      1021
   Ala Leu Leu His Leu Ala Gln Pro Ala Thr Leu Ser Gln Thr Ile Val
                   305                 310                 315
   ccc atc tgc ctc ccg gac agc ggc ctt gca gag cgc gag ctc aat cag      1069
   Pro Ile Cys Leu Pro Asp Ser Gly Leu Ala Glu Arg Glu Leu Asn Gln
               320                 325                 330
   gcc ggc cag gag acc ctc gtg acg ggc tgg ggc tac cac agc agc cga      1117
   Ala Gly Gln Glu Thr Leu Val Thr Gly Trp Gly Tyr His Ser Ser Arg
           335                 340                 345
   gag aag gag gcc aag aga aac cgc acc ttc gtc ctc aac ttc atc aag      1165
   Glu Lys Glu Ala Lys Arg Asn Arg Thr Phe Val Leu Asn Phe Ile Lys
       350                 355                 360
   att ccc gtg gtc ccg cac aat gag tgc agc gag gtc atg agc aac atg      1213
   Ile Pro Val Val Pro His Asn Glu Cys Ser Glu Val Met Ser Asn Met
   365                 370                 375                 380
   gtg tct gag aac atg ctg tgt gcg ggc atc ctc ggg gac cgg cag gat      1261
   Val Ser Glu Asn Met Leu Cys Ala Gly Ile Leu Gly Asp Arg Gln Asp
                   385                 390                 395
   gcc tgc gag ggc gac agt ggg ggg ccc atg gtc gcc tcc ttc cac ggc      1309
   Ala Cys Glu Gly Asp Ser Gly Gly Pro Met Val Ala Ser Phe His Gly
               400                 405                 410
   acc tgg ttc ctg gtg ggc ctg gtg agc tgg ggt gag ggc tgt ggg ctc      1357
   Thr Trp Phe Leu Val Gly Leu Val Ser Trp Gly Glu Gly Cys Gly Leu
           415                 420                 425
   ctt cac aac tac ggc gtt tac acc aaa gtc agc cgc tac ctc gac tgg      1405
   Leu His Asn Tyr Gly Val Tyr Thr Lys Val Ser Arg Tyr Leu Asp Trp
       430                 435                 440
   atc cat ggg cac atc aga gac aag gaa gcc ccc cag aag agc tgg gca      1453
   Ile His Gly His Ile Arg Asp Lys Glu Ala Pro Gln Lys Ser Trp Ala
   445                 450                 455                 460
   cct tag cgaccctccc tgcagggctg ggcttttgca tggcaatgga tgggacatta       1509
   Pro
   aagggacatg taacaagcac accggcctgc tgttctgtcc ttccatccct cttttgggct    1569
   cttctggagg gaagtaacat ttactgagca cctgttgtat gtcacatgcc ttatgaatag    1629
   aatcttaact cctagagcaa ctctgtgggg tggggaggag cagatccaag ttttgcgggg    1689
   tctaaagctg tgtgtgttga gggggatact ctgtttatga aaaagaataa aaaacacaac    1749
   cacgaaaaaa aaaaaaaaaa aaaaaaa                                        1776
<210> 18
   <211> 461
   <212> PRT
   <213> Homo sapiens
<400> 18
   Met Trp Gln Leu Thr Ser Leu Leu Leu Phe Val Ala Thr Trp Gly Ile
   1 5 10 15
   Ser Gly Thr Pro Ala Pro Leu Asp Ser Val Phe Ser Ser Ser Glu Arg
   20 25 30
   Ala His Gln Val Leu Arg Ile Arg Lys Arg Ala Asn Ser Phe Leu Glu
   35 40 45
   Glu Leu Arg His Ser Ser Leu Glu Arg Glu Cys Ile Glu Glu Ile Cys
   50 55 60
   Asp Phe Glu Glu Ala Lys Glu Ile Phe Gln Asn Val Asp Asp Thr Leu
   65 70 75 80
   Ala Phe Trp Ser Lys His Val Asp Gly Asp Gln Cys Leu Val Leu Pro
   85 90 95
   Leu Glu His Pro Cys Ala Ser Leu Cys Cys Gly His Gly Thr Cys Ile
   100 105 110
   Asp Gly Ile Gly Ser Phe Ser Cys Asp Cys Arg Ser Gly Trp Glu Gly
   115 120 125
   Arg Phe Cys Gln Arg Glu Val Ser Phe Leu Asn Cys Ser Leu Asp Asn
   130 135 140
   Gly Gly Cys Thr His Tyr Cys Leu Glu Glu Val Gly Trp Arg Arg Cys
   145 150 155 160
   Ser Cys Ala Pro Gly Tyr Lys Leu Gly Asp Asp Leu Leu Gln Cys His
   165 170 175
   Pro Ala Val Lys Phe Pro Cys Gly Arg Pro Trp Lys Arg Met Glu Lys
   180 185 190
   Lys Arg Ser His Leu Lys Arg Asp Thr Glu Asp Gln Glu Asp Gln Val
   195 200 205
   Asp Pro Arg Leu Ile Asp Gly Lys Met Thr Arg Arg Gly Asp Ser Pro
   210 215 220
   Trp Gln Val Val Leu Leu Asp Ser Lys Lys Lys Leu Ala Cys Gly Ala
   225 230 235 240
   Val Leu Ile His Pro Ser Trp Val Leu Thr Ala Ala His Cys Met Asp
   245 250 255
   Glu Ser Lys Lys Leu Leu Val Cys Leu Gly Glu Tyr Asp Leu Arg Arg
   260 265 270
   Trp Glu Lys Trp Glu Leu Cys Leu Asp Ile Lys Glu Val Phe Val His
   275 280 285
   Pro Asn Tyr Ser Lys Ser Thr Thr Asp Asn Asp Ile Ala Leu Leu His
   290 295 300
   Leu Ala Gln Pro Ala Thr Leu Ser Gln Thr Ile Val Pro Ile Cys Leu
   305 310 315 320
   Pro Asp Ser Gly Leu Ala Glu Arg Glu Leu Asn Gln Ala Gly Gln Glu
   325 330 335
   Thr Leu Val Thr Gly Trp Gly Tyr His Ser Ser Arg Glu Lys Glu Ala
   340 345 350
   Lys Arg Asn Arg Thr Phe Val Leu Asn Phe Ile Lys Ile Pro Val Val
   355 360 365
   Pro His Asn Glu Cys Ser Glu Val Met Ser Asn Met Val Ser Glu Asn
   370 375 380
   Met Leu Cys Ala Gly Ile Leu Gly Asp Arg Gln Asp Ala Cys Glu Gly
   385 390 395 400
   Asp Ser Gly Gly Pro Met Val Ala Ser Phe His Gly Thr Trp Phe Leu
   405 410 415
   Val Gly Leu Val Ser Trp Gly Glu Gly Cys Gly Leu Leu His Asn Tyr
   420 425 430
   Gly Val Tyr Thr Lys Val Ser Arg Tyr Leu Asp Trp Ile His Gly His
   435 440 445
   Ile Arg Asp Lys Glu Ala Pro Gln Lys Ser Trp Ala Pro
   450 455 460

## Description

### CROSS REFERENCE TO RELATED APPLICATION

This application claims the benefit of priority of U.S. Provisional Application No. 60/944,693, filed June 18. 2007,

### STATEMENT OF GOVERNMENT SUPPORT

This invention was made with United States government support under grant HL052246, from the National Heart, Lung, and Blood Institute, National Institutes of Health. The United States government has certain rights in the invention.

### FIELD

This disclosure concerns protein C polypeptides and polynucleotides and their use as hemostatic agents.

### BACKGROUND

Upon damage to vessel walls, exposed extracellular matrix (ECM) proteins and released tissue factor (TF) triggers a series of events that lead to the formation of a hemostatic plug. The essential role of platelet and platelet receptors in this process is illustrated by the profound bleeding exhibited by patients deficient in the major glycoproteins, including GPIb (Bernard-Soulier syndrome) and the integrin α_{IIb}β₃ (Glanzmann thrombasthenia) (A.T. Nurden, J. Thromb. Haemost. 3:1773-1782, 2005; Rao et al. Semin. Thromb. Hemost. 30:525-535, 2004). In addition, the critical role of coagulation factors is evidenced by the severe bleeding associated with hemophilic patients, who are deficient in key clotting factors (Moll and White, Curr. Opin. Hematol. 2:386-394, 1995; Valentino and Scheiflinger, Semin. Thromb. Hemost. 32(Suppl 2):32-38, 2006).

While the activation of platelets and the coagulation cascade are essential for normal hemostasis in the wound, thrombus formation is a pathological event that may lead to diseases, including myocardial infarction, pulmonary embolism, or stroke, which are the leading causes of death and disability in industrialized nations (Gawaz et al. J. Clin. Invest. 115:3378-3384, 2005; Z.M. Ruggeri, Nat. Med. 8:1227-1234, 2002). To combat the pathologic aggregation of activated platelets, anti-platelet and anticoagulation agents, such as aspirin, warfarin, and heparin, are routinely administered to patients who are considered to be at high, long-term risk of thrombotic disease, while α_{IIb}β₃-blockers are used in acute situations in the clinic (S.A. Mousa. Curr. Pharm. Des. 9:2317-2322, 2003: Phillips et al. J. Thromb. Haemost. 3:1577-1589. 2005). Further, anticoagulants and thrombolytic agents (such as heparins and tissue-type plasminogen activator) are used to prevent or to break up thrombi in various diseases, including ischemic stroke (Albers et al. Chest 126:483S-512S, 2004: Hacke et al. JAMA 274:1017-1025, 1995).

Protein C (PC) is a serine protease that circulates in the plasma as a zymogen. Human protein C is produced in the liver as a single chain precursor polypeptide of 461 amino acids. Following a series of post-translational modifications, the protein C zymogen is activated by proteolytic cleavage (mediated by thrombin) to produce activated protein C (APC). The activated form of protein C is an anticoagulant. The anticoagulant activity exhibited by APC is a result of its capacity to proteolytically inactivate coagulation factors FVIIIa and FVa, which leads to the inhibition of other components required for blood coagulation, including Factor X and prothrombin.

Although current anti-thrombotic agents are useful in thrombosis by reducing platelet aggregation and clot formation, or by removing thrombi from the circulation, such agents carry deleterious side effects. Systemic anticoagulants reduce fibrin formation and platelet activation in the hemostatic plug and can have severe hemorrhagic side effects, including ischemic stroke. Plasminogen activator treatment, currently the only approved therapy for ischemic stroke in the USA, has been shown to increase the risk of brain hemorrhage, has only a three hour time window of efficacy, and is capable of directly damaging neurons (Benchenane et al. Trends Neurosci. 27:155-160, 2004). Similarly, the same properties of aspirin that lower the clotting action of platelets also cause bleeding (Gorelick and Weisman, Stroke 36:1801-1807, 2005; M.B. Kimmey. Am. J. Med. 117(Suppl 5A):72S-78S, 2005); and oral α_{IIb}β₃-blockers cause a paradoxical increase in cardiovascular disease mortality (Chew el al. Circulation 103:201-206, 2001; Cox et al. J. Am. Coll. Cardiol. 36:1514-1519, 2000; Holmes et al. Am. J. Cardiol. 85:491-493, A410, 2000; Quinn et al. Arterioscler. Thromb. Vasc. Biol. 23:945-952, 2003; Quinn et al. Circulation 106:379-385, 2002; Topol et al. Circulation 108:399-406, 2003), presumably by inadvertently initiating the coagulation cascade. Thus, improved therapies for ischemic/thrombotic events are needed.

Hemostatic agents that minimize bleeding by promoting clotting, such as FEIBA HTT^{™} (activated prothrombin complex concentrate) and NOVOSEVEN^{™} (Factor VIIa; U.S. Patent No. 4,784,950), can increase the risk of thrombosis (Turecek et al. Curr. Hematol. Rep. 3(5):331-337, 2004; Levi and Buller, Crit. Care Med. 33(4):883-890, 2005). Therefore safe hemostatic agents that can correct hemostatic abnormalities; without promotion of thrombus formation, are desirable.

### SUMMARY

This disclosure concerns the surprising finding that protein C polypeptides are effective hemostatic agents. Unlike its activated form protein C is able to attract platelets and promote localized clotting, while avoiding extension of the clot and formation of a thrombus. Thus, provided herein is a method of promoting hemostasis in a subject by administration of a protein C polypeptide, or a nucleic acid molecule encoding a protein C polypeptide. In some embodiments, the subject has been diagnosed with a bleeding disorder or a bleeding episode. Further provided is a method of preventing, treating or ameliorating a bleeding disorder or a bleeding episode in a subject, comprising administering to the subject a protein C polypeptide, or a nucleic acid molecule encoding a protein C polypeptide. Protein C polypeptides include hemostatic fragments or variants of the protein C polypeptides described herein and known in the art.

The foregoing and other features and advantages will become more apparent from the following detailed description of several embodiments, which proceeds with reference to the accompanying figures.

### BRIEF DESCRIPTION OF THE FIGURES

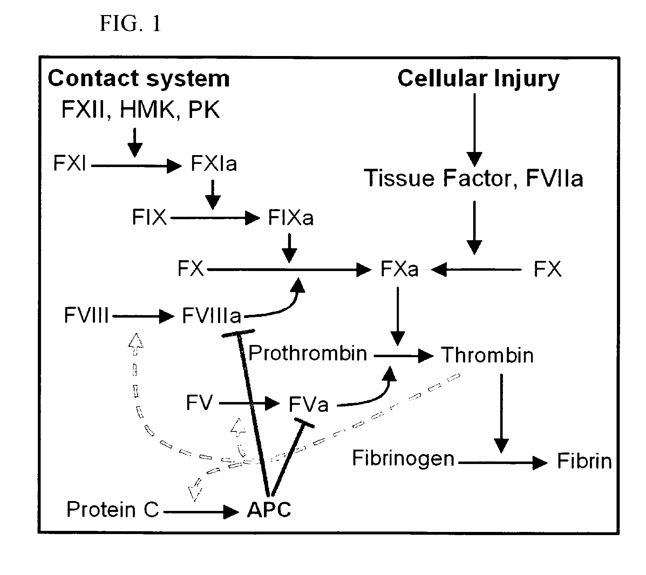

**FIG. 1** is a schematic of the coagulation cascade. Activation of coagulation enzyme leads to the generation of thrombin. An autocatalytic feedback mechanism is propagated by FVIIIa and FVa, while generation of activated protein C (APC) serves to regulate thrombin production.

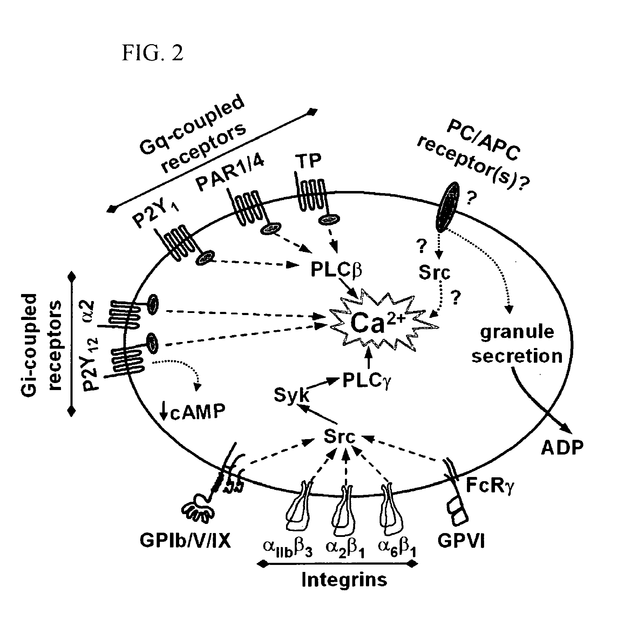

FIG. 2 is a schematic of cellular platelet receptors and signaling cascades. Receptor-mediated signaling leads to platelet activation. The mechanisms mediating platelet-APC and platelet-protein C (PC) binding are currently undefined.

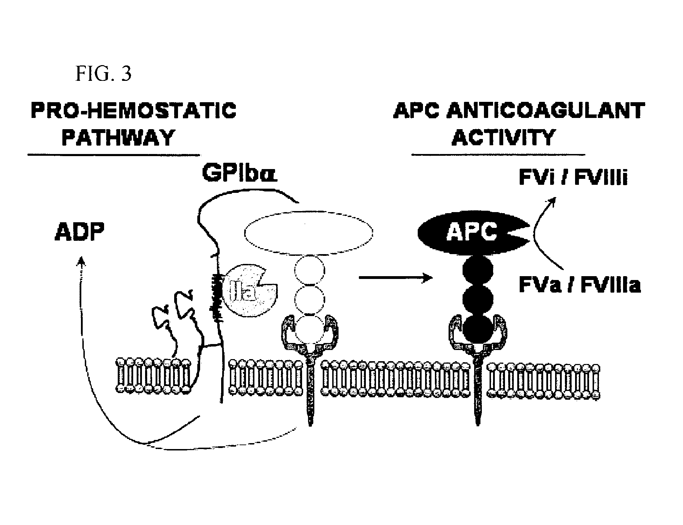

**FIG. 3** shows a hypothetical schematic model of PC-platelet interactions. Platelet GPIb-mediated binding of PC augments ADP release which contributes to platelet activation and thrombus stability (pro-hemostatic activity). Platelet receptors facilitate the activation of PC by thrombin (IIa), leading to localized generation of APC. As an anticoagulant, APC cleaves activated cofactors FVa and FVIIIa (anticoagulant activity).

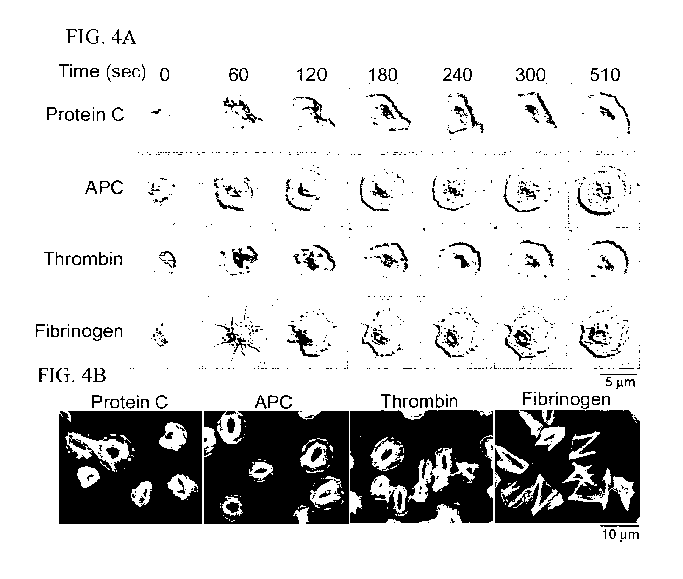

**FIG. 4** is a series of digital images demonstrating platelet spreading and filopodia formation. **FIG. 4A** shows real-time platelet spreading on immobilized PC, APC, thrombin or fibrinogen at 0, 60, 120, 180, 240, 300 and 510 seconds following platelet addition. **FIG. 4B** is a series of images of fluorescent-phalloidin staining to show actin stress fibers of platelets on immobilized PC, APC, thrombin or fibrinogen.

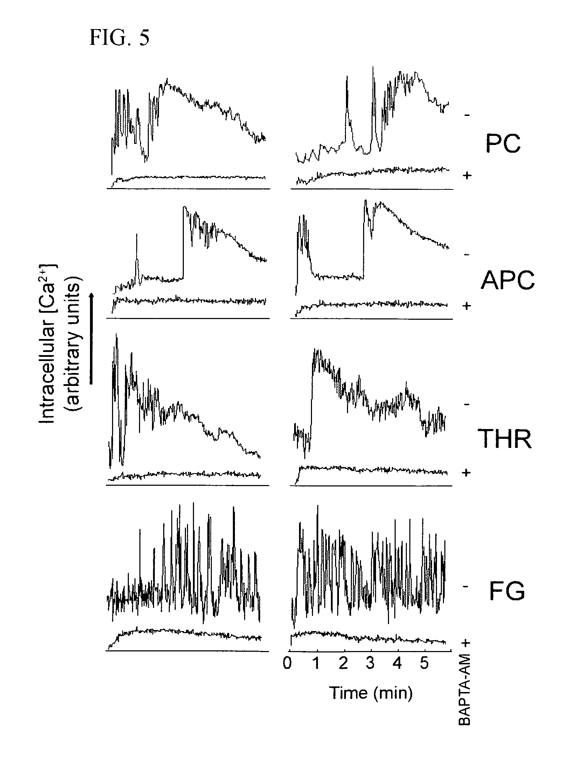

**FIG. 5** is a series of graphs showing intracellular Ca²⁺ elevation of platelets adhered to PC, APC, thrombin (THR) or fibrinogen (FG).

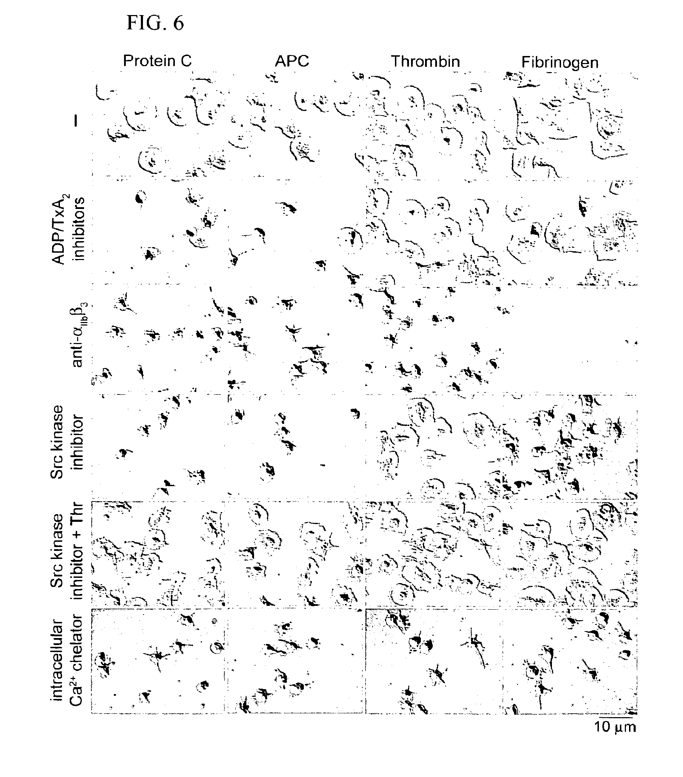

**FIG. 6** is a series of digital images of purified human platelets exposed to immobilized ligands (protein C, APC, thrombin or fibrinogen) in the presence of vehicle (-), function-blocking antibody (anti-α_{IIb}β₃) or pharmacological pathway inhibitors (ADP/TxA₂ inhibitors, Src kinase inhibitor, intracellular Ca²⁺ chelator). Adherent platelets were fixed and imaged by DIC microscopy.

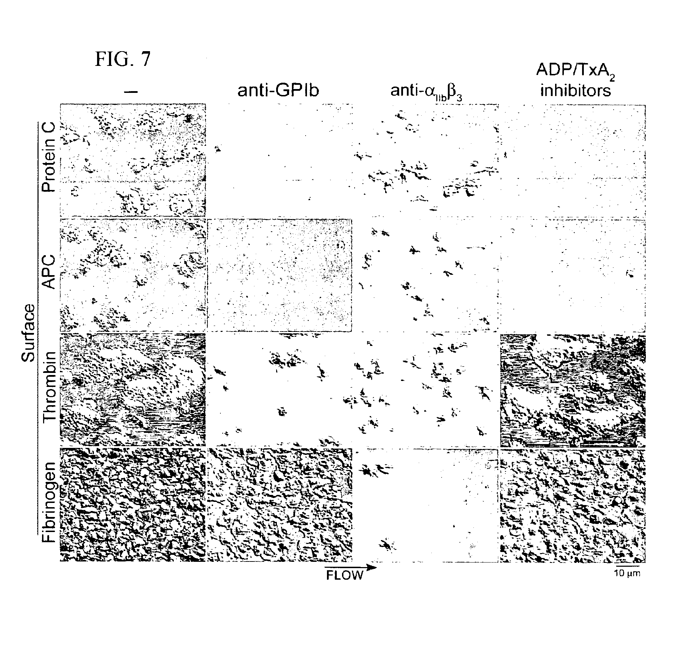

**FIG. 7** is a series of digital images showing platelet adhesion on immobilized PC, APC, thrombin or fibrinogen under shear. Reconstituted blood was perfused over the immobilized ligands at a shear rate of 300⁻¹ for 3 minutes. Platelets were either untreated or treated with an anti-GPIb mAb (10 µg/ml 6D1), an anti-α_{IIb}β₃ mAb (20 µg/ml eptifibatide), or ADP/TxA₂ inhibitors (2 U/ml apyrase; 10 µM indomethacin).

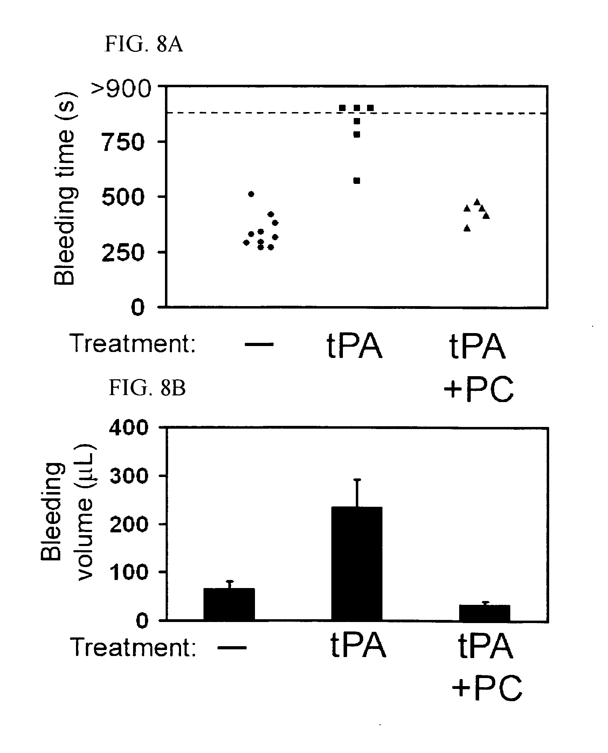

**FIG. 8A and FIG. 8B** are graphs demonstrating the effect of PC on bleeding time (A) and volume (B) in untreated mice (-) and tPA-treated mice.

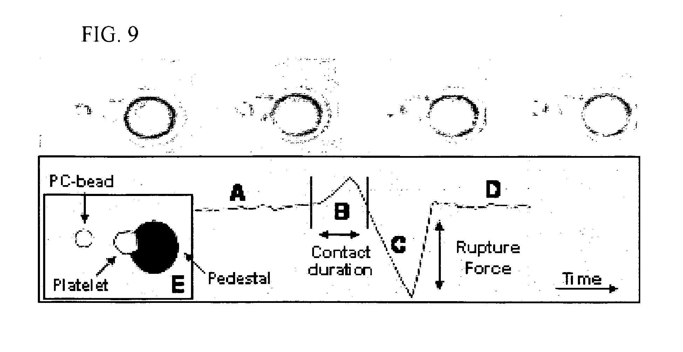

**FIG. 9** shows a representative data trace for a typical interaction between a PC-bead and an immobilized platelet, partitioned into four parts. The PC-bead, trapped near the center of the laser beam, is moved toward (Upper A) or away (Upper D) from the immobilized platelet, corresponding to zero force (Lower A,D). At the moment of contact (Upper B), the platelet stops the motion of the PC-bead while the laser beam continues in the same direction (right).

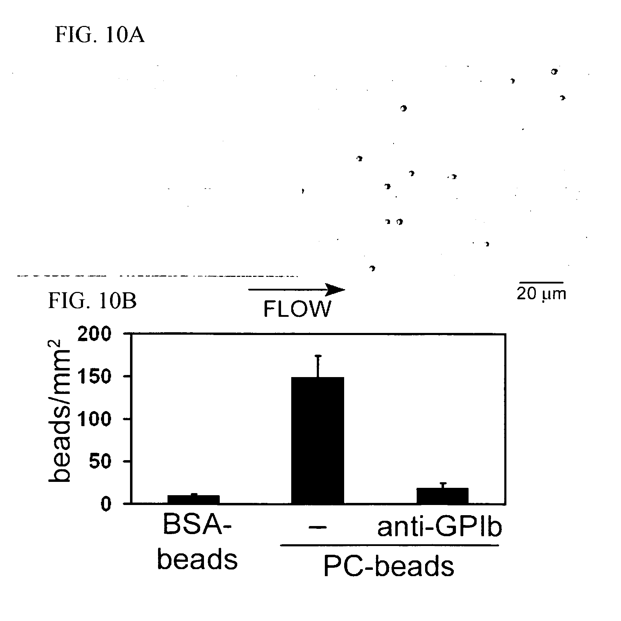

**FIG. 10A and FIG. 10B** are digital images and a graph, respectively, illustrating PC recruitment to immobilized platelets under flow. Platelets were immobilized onto a glass slide treated with 3-aminopropyltriethoxysilane (APES), followed by treatment with 1% BSA. PC or BSA was immobilized onto the surface of 10 µm-diameter polystyrene beads and perfused over the immobilized platelets at a shear rate of 150 s⁻¹ for 5 minutes, in the presence or absence of GPIb antibody.

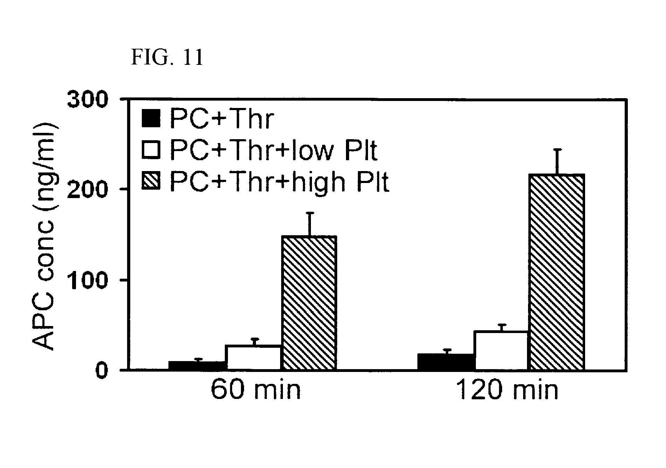

**FIG. 11** is a graph showing platelet-dependent APC generation. A platelet suspension was incubated with PC in the presence of thrombin (Thr) for 60 or 120 minutes. Levels of APC were determined using an APC-specific mAb in conjunction with a HAPC-1555 enzyme capture assay.

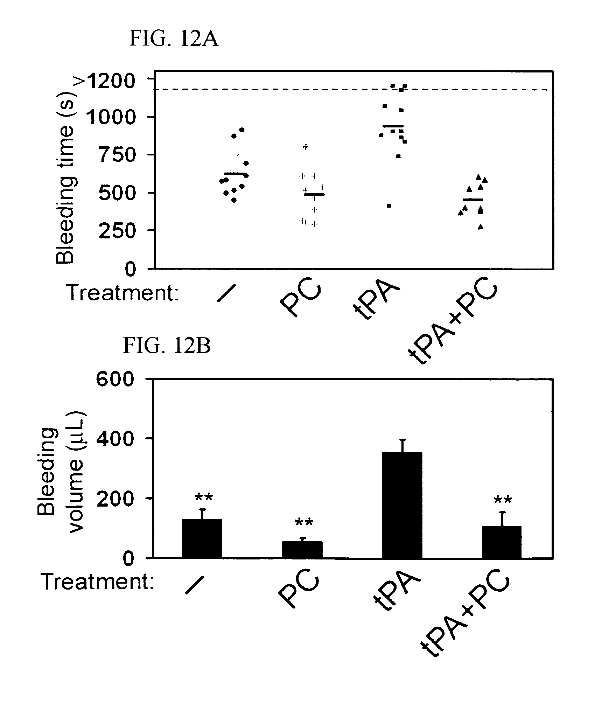

**FIG. 12A and FIG. 12B** are graphs showing the effect of treatment with PC, tPA. or both on hemostasis. Mice were given a bolus injection of 150 µl of either saline or tPA (2 mg/kg) in the presence of either vehicle or recombinant murine PC (3 mg/kg). Bleeding times (A) and bleeding volumes (B) are shown. No animals were allowed to bleed for more than 20 minutes. Bleeding times that exceeded 20 minutes were recorded as being off-scale (dashed line). Horizontal bars represent the mean bleeding time and volume values for each group of animals (n=10-12). ** *P* ≤ 0.01 with respect to bleeding volume in the presence of tPA exclusively.

### SEQUENCE LISTING

The nucleic and amino acid sequences listed in the accompanying sequence listing are shown using standard letter abbreviations for nucleotide bases, and three letter code for amino acids as defined in 37 C.F.R. 1.822. Only one strand of each nucleic acid sequence is shown, but the complementary strand is understood as included by any reference to the displayed strand. In the accompanying sequence listing:
**SEQ ID NOs: 1 and 2** are the nucleotide and an amino acid sequences of human protein C deposited under GenBank Accession No. NM_000312 on June 21, 2006.
**SEQ ID NOs: 3 and 4** are the nucleotide and amino acid sequences of human protein C deposited under GenBank Accession No. BC034377 on July 8, 2002.
**SEQ ID NOs: 5 and 6** are the nucleotide and amino acid sequences of human protein C deposited under GenBank Accession No. K02059 on April 27, 1993.
**SEQ ID NOs: 7 and 8** are the nucleotide and amino acid sequences of mouse protein C deposited under GenBank Accession No. D 10445 on April 29, 1993.
**SEQ ID NOs: 9 and 10** are the nucleotide and amino acid sequences of mouse protein C deposited under GenBank Accession No. NM_001042768 on August 17, 2006.
**SEQ ID NOs: 11 and 12** are the nucleotide and amino acid sequences of rat protein C deposited under GenBank Accession No. NM_012803 on February 16, 2000.
**SEQ ID NOs: 13 and 14** are the nucleotide and amino acid sequences of bovine protein C deposited under GenBank Accession No. XM_585990 on December 22, 2006.
**SEQ ID NOs: 15 and 16** are the nucleotide and amino acid sequences of porcine protein C deposited under GenBank Accession No. NM_213918 on May 20, 2004.
**SEQ ID NOs: 17 and 18** are the nucleotide and amino acid sequences of a mutant form of protein C.

### DETAILED DESCRIPTION

### 1. Abbreviations

| | |
|---|---|
| **ADP** | Adenosine diphosphate |
| **APC** | Activated protein C |
| **BSA** | Bovine serum albumin |
| **ECM** | Extracellular matrix |
| **FG** | Fibrinogen |
| **PC** | Protein C |
| **PRP** | Platelet-rich plasma |
| **rAPC** | Recombinant activated protein C |
| **RBC** | Red blood cell |
| **SDS-PAGE** | Sodium dodecyl sulfate-polyacrylamide gel electrophoresis |
| **SPR** | Surface plasmon resonance |
| **TF** | Tissue factor |
| **tPA** | Tissue-type plasminogen activator |
| **VWF** | von Willebrand factor |

### II. Terms

Unless otherwise noted, technical terms are used according to conventional usage. Definitions of common terms in molecular biology may be found in Benjamin Lewin, Genes V, published by Oxford University Press, 1994 (ISBN 0-19-8s4287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the disclosure, the following explanations of specific terms are provided:

**Anticoagulant:** A compound (such as a pharmaceutical agent or molecule) that prevents or inhibits the clotting of blood. Pharmaceutical anticoagulants can be used to treat thrombotic disorders, such as deep vein thrombosis, pulmonary embolism, myocardial infarction and stroke. The activated form of protein C (APC) is known to function as an anticoagulant.

**Bleeding disorder:** Refers to any congenital, acquired or induced defect that results in abnormal (or pathological) bleeding. Examples include, but are not limited to, disorders of insufficient clotting or hemostasis, such as hemophilia A (a deficiency in Factor VIII), hemophilia B (a deficiency in Factor IX), hemophilia C (a deficiency in Factor XI), other clotting factor deficiencies (such as Factor VII or Factor XIII), abnormal levels of clotting factor inhibitors, platelet disorders, thrombocytopenia, vitamin K deficiency and von Willebrand's disease.

**Bleeding episode:** Refers to an occurrence of uncontrolled, excessive and/or pathological bleeding. Bleeding episodes can result from, for example, drug-induced bleeding (such as bleeding induced by non-steroidal anti-inflammatory drugs or warfarin), anticoagulant overdose or poisoning, aneurysm. blood vessel rupture, surgery and traumatic injury (including, for example, abrasions, contusions, lacerations, incisions or gunshot wounds). Bleeding episodes can also result from diseases such as cancer. gastrointestinal ulceration or from infection.

**Fusion protein:** A protein generated by Expression of a nucleic acid sequence engineered from nucleic acid sequences encoding at least a portion of two different (heterologous) proteins. To create a fusion protein, the nucleic acid sequences must be in the same reading frame and contain no internal stop codons.

**Hemostasis:** Refers to the physiologic process whereby bleeding is halted. Hemostatic agents are those that prevent, treat or ameliorate abnormal bleeding, such as abnormal bleeding caused by a bleeding disorder or bleeding episode. Disorders of hemostasis include, for example, platelet disorders, such as idiopathic thrombocytopenic purpura, and disorders of coagulation, such as hemophilia. Hemostasis can also refer to the complex interaction between vessels, platelets, coagulation factors, coagulation inhibitors and fibrinolytic proteins to maintain the blood within the vascular compartment in a fluid state. The objective of the hemostatic system is to preserve intravascular integrity by achieving a balance between hemorrhage and thrombosis. As described herein, protein C polypeptides and polynucleotides promote hemostasis and are thus useful as hemostatic agents. As used herein, "promoting hemostasis" refers to the process of contributing to or improving hemostasis in a subject. For example, an agent that promotes hemostasis can be an agent that reduces abnormal bleeding, such as by halting bleeding more rapidly, or by reducing the amount of blood loss.

**Isolated:** An "isolated" biological component (such as a nucleic acid molecule or protein) has been substantially separated or purified away from other biological components of the cell or organism in which the component naturally occurs, such as other chromosomal and extra-chromosomal DNA and RNA, or proteins. Nucleic acids and proteins that have been "isolated" include nucleic acids and proteins purified by standard purification methods. The term also embraces nucleic acids and proteins prepared by recombinant expression in a host cell, as well as chemically synthesized nucleic acids or proteins, or fragments or variants thereof

**Operably linked:** A first nucleic acid sequence is operably linked to a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

**Parenteral:** Administered outside of the intestine, for example, not via the alimentary tract. Generally, parenteral formulations are those that will be administered through any possible mode except ingestion. This term especially refers to injections, whether administered intravenously, intrathecally, intramuscularly, intraperitoneally, or subcutaneously, and various surface applications including intranasal, intradermal, and topical application, for instance.

**Pharmaceutical agent:** A chemical compound or other composition capable of inducing a desired therapeutic or prophylactic effect when properly administered to a subject. Also referred to as a "drug."

**Pharmaceutically acceptable vehicles:** The pharmaceutically acceptable carriers (vehicles) useful in this disclosure are conventional. Remington's Pharmaceutical Sciences, by E. W. Martin, Mack Publishing Co.. Easton, PA, 15th Edition (1975), describes compositions and formulations suitable for pharmaceutical delivery of one or more therapeutic compounds or molecules, such as one or more protein C polypeptides or polynucleotides, and additional pharmaceutical agents.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Polypeptide:** A polymer in which the monomers are amino acid residues which are joined together through amide bonds. When the amino acids are alpha-amino acids, either the L-optical isomer or the D-optical isomer can be used. The terms "polypeptide" or "protein" as used herein are intended to encompass any amino acid sequence and include modified sequences such as glycoproteins. The term "polypeptide" is specifically intended to cover naturally occurring proteins, as well as those which are recombinantly or synthetically produced.

The term "residue" or "amino acid residue" includes reference to an amino acid that is incorporated into a protein, polypeptide, or peptide.

Conservative amino acid substitutions are those substitutions that, when made, least interfere with the properties of the original protein, that is, the structure and especially the function of the protein is conserved and not significantly changed by such substitutions. Examples of conservative substitutions are shown below.

| Original Residue | Conservative Substitutions |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu: Ile |
| Phe | Met; Leu; Tyr |
| Ser | Thr |
| Thr | Ser |
| Trp | Tyr |
| Tyr | Trp; Phe |
| Val | Ile; Leu |

Conservative substitutions generally maintain (a) the structure of the polypeptide backbone in the area of the substitution, for example, as a sheet or helical conformation, (b) the charge or hydrophobicity of the molecule at the target site, or (c) the bulk of the side chain.

The substitutions which in general are expected to produce the greatest changes in protein properties will be non-conservative, for instance changes in which (a) a hydrophilic residue, for example, seryl or threonyl, is substituted for (or by) a hydrophobic residue, for example, leucyl, isoleucyl, phenylalanyl, valyl or alanyl; (b) a cysteine or proline is substituted for (or by) any other residue; (c) a residue having an electropositive side chain, for example, lysyl, arginyl, or histadyl, is substituted for (or by) an electronegative residue, for example, glutamyl or aspartyl; or (d) a residue having a bulky side chain, for example. phenylalanine, is substituted for (or by) one not having a side chain, for example, glycine.

**Preventing, treating or ameliorating a disease:** "Preventing" a disease refers to inhibiting the full development of a disease. "Treating" refers to a therapeutic intervention that ameliorates one or more signs or symptoms of a disease or pathological condition after it has begun to develop. "Ameliorating" refers to the reduction in the number, duration or severity of signs and/or symptoms of a disease.

**Promoter:** A promoter is an array of nucleic acid control sequences which direct transcription of a nucleic acid. A promoter includes necessary nucleic acid sequences near the start site of transcription. A promoter also optionally includes distal enhancer or repressor elements. A "constitutive promoter" is a promoter that is continuously active and is not subject to regulation by external signals or molecules. In contrast, the activity of an "inducible promoter" is regulated by an external signal or molecule (for example, a transcription factor).

**Protein C (PC):** A plasma protein produced as a zymogen. The human protein C zymogen is produced in the liver as a 461 amino acid polypeptide. Proteolytic cleavage of protein C by thrombin produces activated protein C (APC), which possesses anti-coagulant and anti-thrombic activity. As disclosed herein, it has been surprisingly discovered that in contrast to APC, the zymogen form of PC has pro-hemostatic activity. The sequences of mammalian protein C polypeptides and polynucleotides are well known in the art, including those set forth herein as SEQ ID NOs 1-18. As used herein, "protein C polypeptide" includes homologs, variants and fragments of protein C that retain hemostatic activity. Such polypeptides are referred to herein as "hemostatic variants and fragments." Fragments and variants of protein C polypeptides are well known in the art (see, for example, U.S. Patent Nos. 5,151,268 and 7,226,999; U.S. Patent Application Publication Nos. 2004-0038288, 2005-0176083 and 2006-0204489; and PCT Publication Nos. WO2004/113385 and WO2006/044294,). In one example, the protein C polypeptide variant is a variant having one or more mutations in the catalytic site, such as the S360A mutant (see, for example, Gale et al. Protein Sci. 6:132-140, 199) .

Purified: The term "purified" does not require absolute purity; rather, it is intended as a relative term. Thus, for example, a purified polypeptide, protein or other active compound is one that is isolated in whole or in part from naturally associated proteins and other contaminants, in which the polypeptide, protein or other active compound is purified to a measurable degree relative to its naturally occurring state, for example, relative to its purity within a cell extract or chemical synthesis checker. Methods of purifying protein C polypeptides have been described (see, for example, Gale et al. Protein Sci. 6:132-140. 1997,).

The term "substantially purified" refers to a polypeptide, protein, or other active compound that has been isolated from a cell, cell culture medium, or other crude preparation and subjected to fractionation to remove various components of the initial preparation, such as proteins, cellular debris, and other components. Such purified preparations can include materials in covalent association with the polypeptide, such as glycoside residues or materials admixed or conjugated with the polypeptide, which may be desired to yield a modified derivative or analog of the polypeptide or produce a combinatorial therapeutic formulation, conjugate, fusion protein or the like. The term purified thus includes such desired products as peptide and protein analogs or mimetics or other biologically active compounds wherein additional compounds or moieties are bound to the polypeptide in order to allow for the attachment of other compounds and/or provide for formulations useful in therapeutic treatment or diagnostic procedures.

Generally, substantially purified polypeptides, proteins, or other active compounds include more than 80% of all macromolecular species present in a preparation prior to admixture or formulation of the respective compound with additional ingredients in a complete pharmaceutical formulation for therapeutic administration. Additional ingredients can include a pharmaceutical carrier, excipient, buffer, absorption enhancing agent, stabilizer, preservative, adjuvant or other like co-ingredients. More topically, the polypeptide, protein or other active compound is purified to represent greater than 90%, often greater than 95% of all macromolecular species present in a purified preparation prior to admixture with other formulation ingredients. In other cases, the purified preparation can be essentially homogeneous, wherein other macromolecular species are less than 1%.

**Recombinant:** A recombinant nucleic acid or polypeptide is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, for example, by genetic engineering techniques.

**Reporter gene:** A reporter gene is a gene operably linked to another gene or nucleic acid sequence of interest (such as a promoter sequence). Reporter genes are used to determine whether the gene or nucleic acid of interest is expressed in a cell or has been activated in a cell. Reporter genes typically have easily identifiable characteristics, such as fluorescence, or easily assayed products, such as an enzyme. Reporter genes can also confer antibiotic resistance to a host cell.

**Sequence identity:** The similarity between amino acid or nucleotide sequences is expressed in terms of the similarity between the sequences, otherwise referred to as sequence identity. Sequence identity is frequently measured in terms of percentage identity (or similarity or homology); the higher the percentage, the more similar the two sequences are.

Methods of alignment of sequences for comparison are well known in the art. Various programs and alignment algorithms are described in: Smith and Waterman, Adv. Appl. Math. 2:482, 1981; Needleman and Wunsch. J. Mol. Biol. 48:443, 1970; Pearson and Lipman, Proc. Natl. Acad. Sci. U.S.A. 85:2444, 1988; Higgins and Sharp, Gene 73:237-244, 1988; Higgins and Sharp, CABIOS 5:151-153, 1989: Corpet et al., Nucleic Acids Research 16:10881-10890, 1988; and Pearson and Lipman, Proc. Natl. Acad. Sci. U.SA. 85:2444, 1988. Altschul et al., Nature Genet. 6:119-129, 1994.

The NCBI Basic Local Alignment Search Tool (BLAST^{™}) (Altschul et al., J. Mol. Biol. 215:403-410, 1990.) is available from several sources, including the National Center for Biotechnology Information (NCBI, Bethesda, MD) and on the Internet, for use in connection with the sequence analysis programs blastp, blastn, blastx, tblastn and tblastx.

Variants and/or fragments of protein C generally comprise at least about 80%, at least about 85%, at least about 90%, at least about 95% or at least about 99% sequence identity with a protein C sequence, such as those described herein or described in the art. When less than the entire sequence is being compared for sequence identity, fragments will typically possess at least 80% sequence identity over the length of the fragment, and can possess sequence identities of at least 85%, 90%, 95% or 99%. One of skill in the art will appreciate that these sequence identity ranges are provided for guidance only; it is entirely possible that strongly significant homologs could be obtained that fall outside of the ranges provided.

**Subject:** Living multi-cellular vertebrate organisms, a category that includes both human and non-human mammals.

**Therapeutically effective amount:** A quantity of a specified pharmaceutical agent (such as a protein C polypeptide or polynucleotide) sufficient to achieve a desired effect in a subject being treated with the pharmaceutical agent. For example, this may be the amount of a protein C polypeptide useful for preventing, ameliorating, and/or treating a bleeding disorder. When used for prevention, the quantity of agent administered can also be referred to as a "prophylactically effective amount." Ideally, a therapeutically effective amount (or a prophylactically effective amount) of a pharmaceutical agent is an amount sufficient to promote hemostasis in a subject with a bleeding disorder or bleeding episode, or susceptible to a bleeding disorder or bleeding disorder, without undesired side effects. The effective amount of the pharmaceutical agent will be dependent on the subject being treated, the severity of the affliction, and the manner of administration of the therapeutic composition. For example, a therapeutically effective amount of an active ingredient can be measured as the concentration (moles per liter or molar-M) of the active ingredient in blood (*in vivo*) or a buffer (*in vitro*) that produces an effect. An effective amount of a compound can be administered in a single dose, or in several doses, for example daily. during a course of treatment.

**Thrombosis:** The formation or presence of a clot (also called a "thrombus") inside a blood vessel. obstructing the flow of blood through the circulatory system. Thrombosis is usually caused by abnormalities in the composition of the blood, quality of the vessel wall and/or nature of the blood flow. The formation of a clot is often caused by an injury to the vessel wall (such as from trauma or infection) and by the slowing or stagnation of blood flow past the point of injury. In some cases, abnormalities in coagulation cause thrombosis.

**Transformed:** A transformed cell is a cell into which has been introduced a nucleic acid molecule by molecular biology techniques. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Trauma:** As used herein, "trauma" or "traumatic injury" refers to a physical injury or wound to the body.

**Variants, fragments or fusions:** The disclosed protein C polypeptides (such as those set forth as SEQ ID NOs: 2, 4, 6, 8, 10, 12, 14, 16 or 18), include homologs, variants, fragments, and fusions thereof that retain protein C biological activity (such as its pro-hemostatic activity). In some embodiments, a variant or fragment of protein C comprises at least 80% sequence identity with a mammalian protein C polypeptide described herein and/or known in the art. Variants and fragments of protein C are well known in the art, including those described in U.S. Patent Nos. 5,151,268 and 7,226,999; U.S. Patent Application Publication Nos. 2004-0038288, 2005-0176083; 2006-0204489; and 2008-0658265: and PCT Publication Nos. WO2004/113385; WO2006/044294 and WO 2008/055145,. In one embodiment, the protein C polypeptide variant is the S360A mutant (see Gale et al. Protein Sci. 6:132-140, 1997 and WO 2008/055145,), which contains a serine to alanine change in the catalytic site of the activated enzyme. DNA sequences which encode for a polypeptide or fusion protein thereof, or a fragment or variant of thereof, can be engineered to allow the protein to be expressed in eukaryotic or prokaryotic cells, such as mammalian cells, bacterial cells, insect cells, and plant cells. To obtain expression, the DNA sequence can be altered and operably linked to other regulatory sequences. The final product, which contains the regulatory sequences and the protein of interest, is referred to as a vector. This vector can be introduced into the desired cell. Once inside the cell the vector allows the protein to be produced. One of ordinary skill in the art will appreciate that the DNA can be altered in numerous ways without affecting the biological activity of the encoded protein. For example, PCR can be used to produce variations in the DNA sequence that encodes a protein. Such variants can be variants optimized for codon preference in a host cell used to express the protein, or other sequence changes that facilitate Expression.

**Vector:** A vector is a nucleic acid molecule allowing insertion of foreign nucleic acid without disrupting the ability of the vector to replicate and/or integrate in a host cell. A vector can include nucleic acid sequences that permit it to replicate in a host cell, such as an origin of replication. An insertional vector is capable of inserting itself into a host nucleic acid. A vector can also include one or more selectable marker genes and other genetic elements. An expression vector is a vector that contains the necessary regulatory sequences to allow transcription and translation of inserted gene or genes.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. Hence "comprising A or B" means including A, or B, or A and B. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present disclosure, suitable methods and materials are described below.

### III. Overview of Several embodiments

Protein C is a plasma protein that exists as a zymogen until proteolytically cleaved by thrombin to produce activated protein C (APC). Through its serine protease activity, APC inactivates the coagulation factors Factor Va and Factor VIIIa, leading to an inhibition of clotting. The anticoagulant activity of APC is important for preventing excessive thrombus formation, which can lead to potentially fatal ischemic events, including stroke or myocardial infarction.

As described herein, it has been surprisingly discovered that protein C, in contrast to its activated form, exhibits hemostatic activity while retaining anti-thrombic activity. Thus, provided herein is a pharmaceutical composition comprising a protein C polypeptide, or a hemostatic fragment or variant thereof for use in maitaining hemostasis. Preferably, the subject has been diagnosed with a bleeding disorder or a bleeding episode, and the protein C polypeptide or a hemostatic fragment or variant thereof is administered in a dose that therapeutically improves the bleeding disorder or bleeding episode. Further provided is a composition for use in treating, preventing, preventing or ameliorating a bleeding disorder or bleeding episode in a subject wherein the composition comprises a therapeutically effective amount of a protein C polypeptide, or a hemostatic fragment or variant thereof.

The protein C polypeptides described herein are mammalian protein C polypeptides, including human, mouse, rat, bovine and porcine protein C polypeptides. In one embodiment of the methods, the protein C polypeptide or hemostatic fragment or variant thereof comprises at least 80%, at least 85%, at least 90%, at least 95% or at least 99% sequence identity with the amino acid sequence set forth as SEQ ID NO: 2, 4, 6, 8, 10, 12, 14, 16 or 18. Preferably the protein C polypeptide comprises the amino acid sequence set forth as SEQ ID NO: 2, 4, 6, 8, 10, 12, 14, 16 or 18. More preferably, the protein C polypeptide consists of the amino acid sequence set forth as SEQ ID NO: 2, 4, 6, 8, 10, 12, 14, 16 or 18. In some aspects of the present disclosure the protein C polypeptide is a fragment of protein C, or a variant of protein C, that retains its hemostatic activity (referred to herein as a "hemostatic fragment" or a "hemostatic variant" of protein C). In some aspects the protein C fragment comprises at least 50, at least 100, at least 200, at least 300 or at least 400 consecutive amino acids of a protein C polypeptide, such as a protein C polypeptide described herein or known in the art. Fragments and variants of protein C polypeptides are well known in the art. In some aspects the protein C polypeptide comprises one or more mutations in an active site of the enzyme. In one example, the protein C polypeptide variant is a protein C polypeptide comprising a mutation in the catalytic site, namely the S360A mutant. In another example, the protein C polypeptide variant comprises the amino acid sequence set forth as SEQ ID NO: 18.

The method of delivery of the polypeptide depends upon, in part, the bleeding disorder or bleeding episode being treated. In some aspects, the protein C polypeptide or hemostatic fragment or variant thereof is administered by a parenteral route. In one aspect the protein C polypeptide or hemostatic fragment or variant thereof is administered intravenously, such as by bolus injection or infusion. In another aspect the protein C polypeptide or hemostatic fragment or variant thereof is administered topically, for example, in a gel or ointment, or in a solid form similar to a styptic application in which the hemostatic agent is dissolved by the blood to act at a site of injury. In some cases, topical administration comprises administering the protein C polypeptide as part of a wound dressing. For example, the actual agent can be applied to a bandage or a bioadhesive, such as described in U.S. Patent Nos. 7.019.191; 7,022,125; 7,196,054; 7,211,651; and 7,230154,.

The dose of protein C polypeptide can vary depending upon a variety of factors, including the bleeding disorder or bleeding episode being treated and the subject being treated. A suitable dose can be determined by one of ordinary skill in the art. In some embodiments, the dose of protein C polypeptide, or hemostatic fragment or variant thereof, is about 0.1 mg/day to about 500 mg/day, such as about 0.5 mg/day, about 1.0 mg/day, about 2.5 mg/day, about 5.0 mg/day, about 10 mg/day, about 25 mg/day, about 50 mg/day, about 100 mg/day, about 200 mg/day, about 250 mg/day, about 300 mg/day, about 400 mg/day or about 500 mg/day. In one embodiment, the dose of the protein C polypeptide or hemostatic fragment or variant thereof is about 0.1 to 10 mg/day, such as about 0.1, about 0.2, about 0.5, about 1.0, about 2.5, about 5.0, about 7.5 or about 10 mg/day. In one embodiment, the dose is about 1 to about 100 ing/day. The dose of protein C can also be determined based on the weight of a subject to be treated. Thus, in some embodiments, the dose of a protein C polypeptide is about 0.1 mg/kg to about 10 mg/kg, such as about 0.1 mg/kg, about 0.2 mg/kg, about 0.5 mg/kg, about 1.0 mg/kg, about 2.5 mg/kg, about 5.0 mg/kg, about 7.5 mg/kg, or about 10 mg/kg. In one embodiment, the dose is about 1 to about 10 mg/kg.

The dosing schedule can also vary. In one embodiment, the protein C polypeptide or hemostatic fragment or variant thereof is administered in a single dose. In another embodiment, the protein C polypeptide or hemostatic fragment or variant thereof is administered in multiple doses. The timing of administration can vary depending the bleeding episode or disorder being treated. For example, a patient with a chronic bleeding disorder can be treated regularly, such as twice a day, once a day, twice a week, once a week, twice a month or once a month, or any other appropriate schedule necessary to control bleeding. In another example, a patient preparing to undergo surgery can be treated with protein C prior to surgery, as well as after surgery as needed.

Further provided herein is a vector comprising a protein C nucleic acid sequence for use in promoting hemostasis, wherein the protein C nucleic acid sequence encodes a protein C polypeptide or a hemostatic fragment or variant thereof. Also provided is a composition for use in a method of treating, preventing or ameliorating a bleeding disorder or bleeding episode in a subject wherein the composition comprises a vector comprising a protein C nucleic acid sequence, wherein the protein C nucleic acid sequence encodes a protein C polypeptide or a hemostatic fragment or variant thereof.

In some aspects, the protein C nucleic acid sequence comprises at least 80%, at least 85%, at least 90%, at least 95% or at least 99% sequence identity with the nucleotide sequence set forth as SEQ ID NO: 1, 3. 5, 7, 9, 11, 13, 15 or 17. In another aspect, the protein C nucleic acid sequence comprises SEQ ID NO: 1, 3, 5, 7, 9, 11. 13, 15 or 17. In another aspect, the protein C nucleic acid sequence consists of SEQ ID NO: 1, 3, 5, 7, 9. 11, 13, 15 or 17. In other aspects, the protein C nucleic acid sequence encodes a variant or fragment of protein C that retains its hemostatic activity. In one aspect, the protein C polynucleotide encoding a protein C variant comprises the nucleic acid sequence set forth as SEQ ID NO: 17. In another aspect the protein C nucleic sequence encodes a protein C variant comprising a mutation in the catalytic site, such as the S360A mutant.

In some aspects of the present disclosure the vector is a viral vector, such as, but not limited to an adenoviral vector, an adeno-associated viral vector, a herpesviral vector, a retroviral vector or a lentiviral vector. In another aspect the vector is a eukaryotic expression vector.

In some aspects, the vector is administered by a parenteral route, such as intravenously. In one aspect the vector is administered in a single dose. In another aspect, the vector is administered in multiple doses.

A protein C polypeptide or polynucleotide can be used as a hemostatic agent for any one of a number of different bleeding disorders or bleeding episodes. In some aspects, the bleeding disorder is associated with insufficient hemostasis, such as a clotting factor deficiency, a platelet disorder, thrombocytopenia, vitamin K deficiency or von Willebrand's disease. In one aspect, the clotting factor deficiency is hemophilia A. In another aspect, the clotting factor deficiency is hemophilia B. In another aspect, the clotting factor deficiency is hemophilia C. In some aspects, the bleeding episode is caused by a drug, an anticoagulant overdose, an aneurysm, blood vessel rupture, surgery, a traumatic injury, cancer, gastrointestinal bleeding (from ulceration or trauma) or an infection. In one aspect the drug is a non-steroidal anti-inflammatory drug. In another aspect, the drug is warfarin.

In some cases, a protein C polypeptide or polynucleotide is administered in combination with other pharmaceutical agents that treat bleeding disorders and bleeding episodes by promoting hemostasis. Such pharmaceutical agents include, but are not limited to fibrinogen, thrombin, Factor IX, Factor VII. Factor DC, Factor X, Factor XI, Factor XII, Factor XIII, or activated forms thereof. In addition, protein C polypeptides and polynucleotides can be administered in conjunction with other methods of controlling bleeding and promoting hemostasis, such as surgery, stitches, staples or cauterization.

### IV. Role of Platelets, Thrombin and Protein C in Thrombus Formation

The primary function of platelets is to arrest bleeding. This process requires an orchestrated series of receptor-mediated events facilitating platelet adhesion, rapid cellular activation, and the subsequent accumulation of fibrin and additional platelets into a growing hemostatic plug (Watson et al. J. Thromb. Haemost. 3:1752-1762, 2005). Initial platelet deposition is triggered by the denuding of the endothelium, resulting in the exposure of ECM proteins. ECM-bound von Willebrand factor (VWF) plays a critical role in the tethering of platelets at high shear levels due to the rapid on-rate of binding between GPIb and VWF (McCarty et al. J. Thromb. Haemost. 4:1367-1378, 2006). The rapid off-rate of GPIb-VWF interactions results in platelet translocation at the site of injury, allowing adhesive interactions of receptors with slower binding kinetics (such as integrins) to mediate the firm adhesion of platelets (Jurk and Kehrel, Semin. Thromb. Hemost. 31:381-392, 2005). Ultimately, these receptor-mediated interactions result in platelet activation, which in turn leads to a rapid remodeling of the actin cytoskeleton, the release of ADP and thromboxanes, and a negatively charged platelet surface (Watson et al. J. Thromb. Haemost. 3:1752-1762, 2005; J. M. Gibbins, J. Cell Sci. 117:3415-3425, 2004; Jackson et al. J. Thromb. Haemost. 1:1602-1612, 2003).

Following vascular injury, concomitant with platelet recruitment and activation, are the first steps of blood coagulation, namely the release of tissue factor (see FIG. 1) (Renne et al. Blood Cells Mol. Dis. 36:148-151, 2006; Renne et al. J. Exp. Med. 202:271-281, 2005; Steffel et al. Circulation 113:722-731, 2006). This process leads to the sequential conversion of other coagulation factors into their corresponding active forms as serine proteases. Protease activation culminates with the generation of thrombin. In the absence of thrombin, hemostatic plugs cannot form (S.R. Coughlin. J. Thromb. Haemost. 3:1800-1814, 2005; Mangin et a/. Blood 107:4346-4353, 2006; Sambrano et al. Nature 413:74-78. 2001). Thrombin not only attracts and activates platelets, and cleaves fibrinogen (which leads to fibrin production and clot formation), but thrombin also mediates the feedback activation of the coagulation cofactors, Factor V (FV), VIII (FVIII) and XI (FXI) (Adams and Huntington, Arterioscler. Thromb. Vasc. Biol. 26:1738-1745, 2006). This feedback mechanism leads to an autocatalyic cascade, resulting in rampant clot formation. The essential proenzymes in the hemostatic coagulation process (such as factors VII, IX, X, and prothrombin) require post-translational vitamin K-dependent gamma carboxylation of 9-12 glutamic acid residues on the amino tenninus (Gla domain) for proper function (J. Stenflo. Crit. Rev. Eukaryot. Gene Expr. 9:59-88, 1999). The two anticoagulant proteins, PC and protein S, also contain Gla domains (Dahlback and Villoutreix, J. Thromb. Haemost. 1:1525-1534. 2003; D.W. Stafford, J. Thromb. Haemost. 3:1873-1878, 2005).

The anticoagulant effects of PC are due to its activation by thrombin, which results in APC, through the cleavage and release of the PC activation peptide (Griffin et al. Blood Cells Mol. Dis. 36:211-216, 2006). This reaction is slowly catalyzed by thrombin in solution, but the binding of thrombin to its endothelial cell cofactor, thrombomodulin, results in a greater than thousand-fold enhancement of the rate of PC activation (Esmon and Owen, J. Thromb. Haemost. 2:209-213, 2004). The activation of PC is further enhanced (approximately 20-fold) by its binding to the endothelial cell PC receptor on the endothelium (C.T. Esmon, Crit. Care Med. 32:S298-301, 2004). Thus, the generation of APC is restricted to the endothelial cell surface in the current paradigm. As an anticoagulant enzyme. APC with the anticoagulant cofactor, protein S, degrades factors Va and VIIIa, which are required to sustain thrombin formation via the coagulation cascade (FIG. 1). Furthermore, APC cleavage of the endothelial PAR-1 receptor leads to the activation of intracellular G-proteins and the generation of cell protective (anti-apoptotic) responses (D.W. Stafford, J. Thromb. Haemost. 3:1873-1878, 2005). Thus, the coagulation cascade is believed to depend on a delicate balance between pro- and anticoagulant pathways. However, it is unknown whether catalysis of the anticoagulant process is restricted to the endothelium. The notion of restricted generation of the anticoagulant APC on the endothelial cell surface does not take into account the hemodynamics involved in the transport of APC, in whole blood, from the endothelium to the leading edge of the thrombus, a distance that may span from several micrometers to several centimeters (J.J. Hathcock, Arterioscler. Thromb. Vasc. Biol. 26:1729-1737, 2006). It is believed that binding of PC and APC to the platelet surface provides a mechanism for local deactivation of the coagulation cascade.

Stimuli originating from agonists released or generated at a site of vascular injury act via signaling networks to enhance (within seconds) the adhesive and pro-coagulant properties of platelets. As shown in FIG. 2, the platelet receptors α_{IIb}β₃ and GPVI, which bind fibrinogen and collagen, respectively, have been shown to induce platelet activation through a pathway that is dependent on the Src family and Syk tyosine kinases, and on the activation of the effector enzyme PLCy2, leading to a dramatic rise in cytosolic calcium flux (J. M. Gibbins, J. Cell Sci. 117:3415-3425, 2004; Jackson et al. J. Thromb. Haemost. 1:1602-1612, 2003). The G protein-coupled receptors PAR-1/4 and P2Y₁, which are activated by thrombin and ADP, respectively, lead to platelet activation in a PLCβ-dependent manner (Lundblad and White, Platelets 16:373-385, 2005; Oury et al. Curr. Pharm. Des. 12:859-875, 2006). A propagation phase follows, whereby platelets secrete mediators such as ADP and thromboxane A₂ (TxA₂), which activate other platelets to form aggregates. In the subsequent perpetuation phase of platelet plug formation, fibrin generation and post-aggregation events are believed to stabilize the thrombus.

The studies described in the Examples herein indicate that PC and APC binding to platelets induces ADP secretion and that PC- and APC-platelet interactions are pro-hemostatic and contribute to thrombus stability (FIG. 3A). This model is analogous to the scenario described for PC binding to endothelial cells which, upon the conversion of PC to APC, APC stimulates endothelial cell activation through the cleavage of PAR-1 (Feistritzer et al. J. Biol. Chem. 281:20077-20084, 2006). Furthermore, in parallel to the anticoagulant role that APC plays on the endothelial cell surface, the data described in the Examples herein indicates that APC generation on the platelet surface plays a role in downregulating thrombogenesis in the lumen of blood vessels (FIG. 3B).

### V. Protein C Polypeptides and Polynucleotides

This disclosure provides the surprising finding that administration of a protein C polypeptide promotes hemostasis by reducing bleeding time and blood volume lost. This finding is surprising given the function of APC as an anti-coagulant. The data described herein indicate that protein C is pro-hemostatic and administration of protein C can inhibit (including prevent), treat or ameliorate bleeding in a subject by promoting hemostasis in a subject with a bleeding disorder or bleeding episode. Thus, provided herein are mammalian protein C polypeptides, termed "protein C polypeptides" for use as hemostatic agents. Human protein C amino acid sequences are known in the art. including, but not limited to human protein C deposited under GenBank Accession No. MM_000312 on June 21, 2006 (SEQ ID NO: 2); GenBank Accession No. BC034377 on July 8, 2002 (SEQ ID NO: 4); and GenBank Accession No. K02059 on April 27, 1993 (SEQ ID NO: 6). Mouse protein C sequences, include but are not limited to the protein C sequences deposited under GenBank Accession No. D10445 on April 29, 1993 (SEQ ID NO: 8: and GenBank Accession No. NM_001042768 on August 17, 2006 (SEQ ID NO: 10). Rat, bovine and porcine protein C polypeptides respectively include the sequences deposited under GenBank Accession No. NM_012803 on February 16, 2000 (SEQ ID NO: 12); GenBank Accession No. XM_585990 on December 22, 2006 (SEQ ID NO: 14); and GenBank Accession No. NM_213918 on May 20, 2004 (SEQ ID NO: 16).

Specific, non-limiting examples of protein C polypeptides include variants and/or fragments of protein C polypeptides, including polypeptides having an amino acid sequence at least about 80%, at least about 85%, at least about 90%, at least about 95%, or at least about 99% identical to the amino acid sequence set forth as SEQ ID NO: 2, 4, 6, 8, 10, 12, 14, 16 or 18. In a Further aspect a protein C polypeptide is a conservative variant of SEQ ID NO: 2, 4, 6, 8, 10, 12, 14, 16 or 18, such that it includes no more than twenty-five conservative amino acid substitutions, such as no more than two, no more than five, no more than ten, no more than fifteen, no more than twenty, or no more than twenty-live conservative amino acid substitutions in SEQ ID NO: 2, 4, 6, 8, 10, 12, 14, 16 or 18. In another aspect a protein C polypeptide comprises an amino acid sequence as set forth as SEQ ID NO: 2, 4, 6, 8, 10. 12. 14, 16 or 18. In another aspect a protein C polypeptide consists of an amino acid sequence as set forth as SEQ ID NO: 2, 4, 6, 8, 10, 12, 14, 16 or 18.

Fragments and variants of a protein C polypeptide can readily be prepared by one of skill in the art using molecular techniques. In one embodiment, a fragment of a protein C polypeptide includes at least 50, 100, 200, 300 or 400 consecutive amino acids of the protein C polypeptide. In a further embodiment, a fragment or variant of protein C is a fragment or variant that retains hemostatic activity. Such fragments and variants are referred to as "hemostatic fragments and variants." Fragments and variants of protein C polypeptides are well known in the art (see, for example, U.S. Patent Nos. 5,151,268 and 7,226,999; U.S. Patent Application Publication Nos. 2004-0038288, 2005-0176083; 2006-0204489; and 2008-0658265; and PCT Publication Nos. WO 2004/113385: WO 2006/044294; and WO 2008/055145, ). In one aspect the protein C polypeptide variant comprises the amino acid sequence of SEQ ID NO: 18.

In another aspect, the protein C polypeptide variant is a protein C polypeptide having a mutation in a catalytic site, such as the S360A mutant (see WO 2008/055145 and Gale et al. Protein Sci. 6:132-140, 1997). The S360A mutant, when proteolyically processed to form APC, lacks amidolytic activity but retains significant (although not complete) anti-coagulant activity and is not inhibited by serine protease inhibitors. In addition, the S360A mutant has an increased half life *in vivo.* As shown herein, the S360A mutant of PC also exhibits pro-hemostatic activity, indicating that the hemostatic activity of PC does not require catalytic activity of APC.

One skilled in the art can purify a protein C polypeptide using standard techniques for protein purification. The substantially pure polypeptide will yield a single major band on a non-reducing polyacrylamide gel. The purity of the protein C polypeptide can also be determined by amino-terminal amino acid sequence analysis. Expression and purification of recombinant protein C has been described (Gale et al. Protein Sci. 6:132-140, 1997,)

Minor modifications of the protein C polypeptide primary amino acid sequences may result in peptides which have substantially equivalent activity as compared to the unmodified counterpart polypeptide described herein. Such modifications may be deliberate, as by site-directed mutagenesis, or may be spontaneous. All of the polypeptides produced by these modifications are included herein.

One of skill in the art can readily produce fusion proteins including a protein C polypeptide and a second polypeptide of interest. Optionally, a linker can be included between the protein C polypeptide and the second polypeptide of interest. Fusion proteins include, but are not limited to, a polypeptide including a protein C polypeptide and a marker protein. In one aspect, the marker protein can be used to identify or purify a protein C polypeptide. Exemplary fusion proteins include, but are not limited to, green fluorescent protein, six histidine residues, or myc, and a protein C polypeptide.

Polynucleotides encoding a mammalian protein C polypeptide are also provided, and are termed "protein C polynucleotides." These polynucleotides include DNA, cDNA and RNA sequences which encode a mammalian protein C. Exemplary polynucleotide sequences encoding protein C are known in the art, such as, but not limited to human protein C deposited under GenBank Accession No. NM_000312 on June 21, 2006 (SEQ ID NO: 1); GenBank Accession No. BC034377 on July 8, 2002 (SEQ ID NO: 3); and GenBank Accession No. K02059 on April 27, 1993 (SEQ ID NO: 5). Mouse protein C nucleic acid sequences, include but are not limited to the protein C sequences deposited under GenBank Accession No. D10445 on April 29, 1993 (SEQ ID NO: 7: and GenBank Accession No. NM_001042768 on August 17, 2006 (SEQ ID NO: 9). Rat. bovine and porcine protein C polynucleotides respectively include the sequences deposited under GenBank Accession No. NM_012803 on February 16, 2000 (SEQ ID NO: 11); GenBank Accession No. XM_585990 on December 22, 2006 (SEQ ID NO: 13); and GenBank Accession No. NM_213918 on May 20. 2004 (SEQ ID NO: 15).

Specific, non-limiting examples of protein C polynucleotides include polynucleotides that encode variants and/or fragments of protein C polypeptides. Such protein C polynucleotides include polynucleotides having a nucleic acid sequence at least about 80%, at least about 85%, at least about 90%, at least about 95%, or at least about 99% identical to the nucleic acid sequence set forth as SEQ ID NO: 1, 3, 5, 7, 9, 11, 13. 15 or 17. In a further aspect, a protein C polynucleotide encodes a conservative variant of the amino acid sequence set forth as SEQ ID NO: 2, 4, 6, 8, 10, 12, 14, 16 or 18, such that the encoded variant includes no more than twenty-five conservative amino acid substitutions, such as no more than two, no more than five, no more than ten, no more than fifteen, no more than twenty, or no more than twenty-five conservative amino acid substitutions in SEQ ID NO: 2, 4, 6, 8, 10, 12, 14. 16 or 18. In another aspect a protein C polynucleotide comprises a nucleic acid sequence as set forth as SEQ ID NO: 1, 3, 5, 7, 9, 11, 13, 15 or 17. In another aspect, a protein C polynucleotide consists of a nucleic acid sequence as set forth as SEQ ID NO: 1, 3, 5, 7, 9, 11, 13, 15 or 17.

In some aspects, the protein C polynucleotide encodes a protein C polypeptide variant. In one example, the nucleic acid encoding a protein C polypeptide variant comprises SEQ ID NO: 17. In another example, the protein C polynucleotide encodes a protein C polypeptide variant having a mutation in the catalytic site namely the S360A mutant.

A polynucleotide encoding a mammalian protein C polypeptide can be included in an expression vector to direct expression of the protein C nucleic acid sequence. Other expression control sequences, including appropriate promoters, enhancers, transcription terminators, a start codon, splicing signals for introns, and stop codons can be included in an expression vector. Generally expression control sequences include a promoter, a milimal sequence sufficient to direct transcription. Expression vectors comprising protein C polynucleotide sequences can be used to transform cells for amplification and purification of protein C polypeptide. Vectors encoding protein C polynucleotides also can be used to directly administer to a subject with a bleeding disorder or bleeding episode.

### VI. Vectors and Cells for Expression and Delivery of Protein C

Suitable vectors for expression of protein C will typically contain an origin of replication and a promoter. Vectors can optionally comprise a specific gene which allows for phenotypic selection of the transformed cells (for example, an antibiotic resistance cassette), a marker gene to enable purification of the expressed protein, and/or a reporter gene for detection of expression.

Suitable vectors include, but are not limited to, prokaryotic and eukaryotic expression vectors. Expression vectors are well known in the art (see for example, Eukaryolic Viral Vectors, Cold Spring Harbor Laboratory, Gluzman ed., 1982). Examples of eukaryotic expression vectors include pMSXND (Lee and Nathans, J. Biol. Chem. 263:3521, 1988), and pCIS2M (Gale et al. Protein Sci. 6:132-140, 1997 ).

Other suitable vectors include viral vectors. Viral vectors can be used to deliver protein C polynucleotides to cells for amplification and purification of protein C polypeptides, or viral vectors can be used to administer protein C polynucleotides to a subject. Specific, non-limiting examples of viral vectors include, but are not limited to, adenovirus vectors, adeno-associated virus vectors, retroviral vectors, lentiviral vectors, herpesviral vectors, and the like.

Adenovirus vectors can be first second, third and/or fourth generation adenoviral vectors or gutless adenoviral vectors. Adenovirus vectors can be generated to very high titers of infectious particles; infect a great variety of cells; efficiently transfer genes to cells that are not dividing; and are seldom integrated in the host genome, which avoids the risk of cellular transformation by insertional mutagenesis (Douglas and Curiel, Science and Medicine, March/April 1997, pages 44-53; Zern and Kresinam, Hepatology 25(2), 484-491, 1997). Representative adenoviral vectors which can be used for the methods provided herein are described by Stratford-Perricaudet et al. (J. Clin. Invest. 90: 626-630, 1992): Graham and Prevec (*In* Methods in Molecular Biology: Gene Transfer and Expression Protocols 7: 109-128, 1991); and Barr et al. (Gene Therapy, 2:151-155, 1995).

Adeno-associated virus (AAV) vectors also are suitable for expression and/or delivery of protein C. Methods of generating AAV vectors, administration of AAV vectors and their use are well known in the art (see, for example, U.S. Patent No. 6,951,753; U.S. Pre-Grant Publication Nos. 2007-036757, 2006-205079, 2005-163756, 2005-002908; and PCT Publication Nos. WO 2005/116224 and WO 2006/119458,

Retrovirus, including lentivirus, vectors can also be used with the methods described herein. Lentiviruses include, but are not limited to human immunodeficiency virus (such as HIV-1 and HIV-2), feline immunodeficiency virus, equine infectious anemia virus and simian immunodeficiency virus. Other retroviruses include, but are not limited to, human T-lymphotropic virus, simian T-lymphotropic virus, murine leukemia virus, bovine leukemia virus and feline leukemia virus. Methods of generating retrovirus and lentivirus vectors and their uses have been well described in the art (see, for example, U.S. Patent Nos. 7,211,247; 6,979,568; 7,198,784; 6,783,977; and 4,980,289 ).

Suitable herpesvirus vectors can be derived from any one of a number of different types of herpesviruses, including, but not limited to, herpes simplex virus-1 (HSV-1), HSV-2 and herpesvirus saimiri. Recombinant herpesvirus vectors, their construction and uses are well described in the art (see, for example, U.S. Patent Nos. 6,951,753; 6,379,6741 6,613,892; 6,692,955; 6,344,445; 6,319,703; and 6,261,552; and U.S. Pre-Grant Publication No. 2003-0083289.).

Expression vectors for use in expressing protein C polypeptides comprise a promoter capable of directing the transcription of a nucleic acid encoding protein C. Suitable promoters are well known in the art. Promoters for use in cultured mammalian cells include viral promoters and cellular promoters. Viral promoters include the SV40 promoter (Subramani et al. Mol. Cell. Biol. 1:854-864, 1981); the CMV promoter (Boshart et al. Cell 41:521-530, 1985); and the major late promoter from adenovirus 2 (Kaufman and Sharp, Mol. Cell. Biol. 2:1304-1319, 1982). Cellular promoters include the mouse kappa gene promoter (Bergman et al. Proc. Natl. Acad. Sci. U.S.A. 81:7041-7045, 1983); the mouse V_{H} promoter (Loh et al. Cell 33:85-93, 1983); and the mouse metallothionen-I promoter (Palmiter et al. Science 222:809-814, 1983). Other suitable promoters include, but are not limited to, the thymidine kinase promoter (TK) and the beta-actin promoter. The promoter can be inducible or constitutive. The promoter can also be tissue specific.

In one aspect the polynucleotide encoding a protein C polypeptide is located downstream of the desired promoter. Optionally, an enhancer element is also included, and can generally be located anywhere on the vector and still have an enhancing effect. However, the amount of increased activity will generally diminish with distance.

Expression vectors can also contain a set of RNA splice sites located downstream from the promoter and upstream from the insertion site for the protein C polynucleotide sequence. Preferred RNA splice sites can be obtained from adenovirus and/or an immunoglobulin gene. Expression vectors can optionally comprise a polyadenylation signal located downstream of the insertion site. Examples of polyadenylation signals include the early or late polyadenylation signal from SV40. the polyadenylation signal from the adenovirus 5 Elb region, or the human growth hormone gene terminator (DeNoto et al. Nucl. Acids Res. 9: 3719-3730, 1981).

Expression vectors comprising a polynucleotide encoding mammalian protein C can be used to transform host cells. Hosts can include isolated microbial, yeast, insect and mammalian cells, as well as cells located in an organism. Biologically functional viral and plasmid DNA vectors capable of expression and replication in a host are known in the art, and can be used to transfect any cell of interest. Where the cell is a mammalian cell, the genetic change is generally achieved by introduction of the DNA into the genome of the cell or as an episome. Thus, host cells can be used to produce protein C polypeptides. Alternatively, expression vectors can be used to transform host cells of interest.

A transfected or transformed cell is a cell into which a nucleic acid molecule (such as a nucleic acid molecule encoding a protein C polypeptide) has been introduced by means of recombinant DNA techniques. Transfection of a host cell with recombinant nucleic acid can be carried out by conventional techniques as are well known in the art. Where the host is prokaryotic, such as *E*. *coli,* competent cells which are capable of nucleic acid uptake can be prepared from cells harvested after exponential growth phase and subsequently treated by the CaCl, method using procedures well known in the art. Alternatively, MgCl₂ or RbCl can be used. Transformation can also be performed after forming a protoplast of the host cell if desired, or by electroporation. Nucleic acid sequences are introduced into cultured mammalian cells by, for example, calcium phosphate-mediated transfection (Wigler et al. Cell 14 :725-732, 1978; Corsaro and Pearson, Somatic Cell Genetics 7:603-616, 1981; Graham and Van der Eb, Virology 52d:456-467, 1973), electroporation (Neumann et al. EMBO J. 1:841-845, 1982), microinjection, liposome-mediated transfection, or by viral vector.

### VII. Administration ofprotein C compositions

The protein C polypeptides and polynucleotides described herein are for use in methods to treat, prevent or ameliorate a bleeding disorder or bleeding episode in a subject in need thereof. In one aspect the subject is administered a protein C polypeptide. In another aspect, the subject is administered a nucleic acid molecule encoding a protein C polyeptide. Such nucleic acid molecules encoding protein C polypeptide can be administered in the form of a vector, such as a viral vector.

Protein C polypeptides and polynucleotides are usually administered to a subject as compositions comprising one or more pharmaceutically acceptable carriers. Such carriers are determined in part by the particular composition being administered, as well as by the particular method used to administer the composition. Accordingly, there is a wide variety of suitable formulations of pharmaceutical compositions of the present disclosure.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution. Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives can also be present such as, for example, antimicrobials, anti-oxidants, chelating agents, and inert gases and the like. In one embodiment, the protein C polypeptides or polynucleotides are delivered intravenously in combination with a pharmaceutically acceptable carrier.

Formulations for topical administration can include ointments, lotions, creams, gels, drops, suppositories, sprays, liquids and powders. Conventional pharmaceutical carriers, aqueous, powder or oily bases, thickeners and the like may be necessary or desirable. In one embodiment, the protein C polypeptide is administered topically as part of a wound dressing.

Administration can be accomplished by single or multiple doses. The dose required will vary from subject to subject depending on the species, age, weight, general condition of the subject, the particular bleeding disorder or episode being treated, the particular protein C polypeptide or polynucleotide being used and its mode of administration. An appropriate dose can be determined by one of ordinary skill in the art using only routine experimentation. In some embodiments, the dose of protein C polypeptide is about 0.1 mg/day to about 500 mg/day, such as about 0.5 mg/day, about 1.0 mg/day, about 2.5 mg/day, about 5.0 mg/day, about 10 mg/day, about 25 mg/day, about 50 mg/day, about 100 mg/day, about 200 mg/day, about 250 mg/day, about 300 mg/day, about 400 mg/day or about 500 mg/day. In one embodiment, the dose is about 1 to about 100 mg/day. The dose of protein C can also be determined based on the weight of a subject to be treated. Thus, in some embodiments, the dose of a protein C polypeptide is about 0.1 mg/kg to about 10 mg/kg, such as about 0.1 mg/kg, about 0.2 mg/kg, about 0.5 mg/kg, about 1.0 mg/kg, about 2.5 mg/kg, about 5.0 mg/kg, about 7.5 mg/kg, or about 10 mg/kg. In one embodiment, the dose is about 1 to about 10 mg/kg.

For treating a chronic bleeding disorder, a subject can be administered an appropriate dose of protein C polypeptide or polynucleotide on a regular schedule, such as, for example, weekly, daily or twice daily. To prophylactically treat a patient at risk for developing a bleeding episode (such as a subject scheduled for surgery), the protein C polypeptides or polynucleotides can be administered prior to the bleeding episode (i.e., the surgery), such as 12, 24 or 48 hours prior. In the case of a scheduled surgery, it may be appropriate to administer protein C in a single dose or in multiple doses. In the case of a wound treated with protein C polypeptide topically, the dosing schedule can depend, in part, on the frequency with which the wound dressing is replaced.

Protein C polypeptides and polynucleotides can also be administered in combination with other pharmaceutical agents that treat bleeding disorders and bleeding episodes. Such pharmaceutical agents include, but are not limited to fibrinogen, thrombin, Factor IX, Factor VII, Factor DC, Factor X, Factor XI, Factor XII, Factor XIII, or activated forms thereof. In addition, protein C polypeptides and polynucleotides can be administered in conjunction with other methods of controlling bleeding, such as surgery, stitches, staples or cauterization.

The following examples are provided to illustrate certain particular features and/or embodiments. These examples should not be construed to limit the disclosure to the particular features or embodiments described.

### EXAMPLES

### Example 1: Methods

### Protein C (PC) and activated protein C (APC) polypeptides

Human plasma derived PC and APC can be purchased from Hematologic Technologies (Essex Junction, VT). These PC preparations are free of detectable APC (<0.1%) and thrombin (<0.04 pM in 100 nM PC) as determined by amidolytic assays (Feistritzer et al. J Biol Chem. 281:20077-20084, 2006).

PC was purified from plasma factor IX concentrate using immunoaffinity chromatography (Gruber et al. Circulation 82:578-585,1990; Gruber et al. Circulation 84:2454-2462, 1991). Anti-human PC light-chain mAbs designated C3'5 were coupled to CNBr-activated Sepharose 4B (Pharmacia; 3 mg protein/mL gel) in a coupling buffer (0.5 mol/L NaCl. 0.05 mol/L borate, pH 8.5) overnight at 4°C. The Factor IX concentrate was passed over C3-Sepharose in a buffer containing 0.1 mol/L NaCl, 2 mmol/L EDTA. 2 mmol/L benzamidine. 0.02% Na-azide, 0.02% Tween-20, and 0.02 mol/L Tris-HCl. pH 7.4: and subsequently eluted with 3 mol/L NaSCN in 1.0 mol/L NaCl, 4 mmol/L benzamidine, 2 mmol/L EDTA, 0.02% Na-azide, 0.05% Tween-20, and 0.05 mol/L Tris, pH 7.0. This purification process yields PC that is >98% pure when analyzed using sodium dodecyl sulfate-polyacrylamide gel electrophoresis (SDS-PAGE).

To generate APC, PC in tris-buffered saline (TBS) (0.15 mol/L NaCl. 0.01 mol/L Tris, pH 7.4) was activated using thrombin-Sepharose beads. This process generates APC that does not contain any detectable thrombin, as measured by a clotting assay, and is >95% activated according to SDS-PAGE (Gale et al. Protein Sci. 6:132-140. 1997: Gale et al. J. Biol. Chem. 277:28836-28840, 2002).

Mutant PC expression vectors can be constructed, and recombinant PC mutants can be purified from conditioned media (Gale et al. Protein Sci. 6:132-140, 1997; Gale et al. J. Biol. Chem. 277:28836-28840, 2002). Purified PC can be activated by thrombin to form APC. PC in HBS (50 mM HEPES. 150 mM NaCl) with 2 mM EDTA and 0.5% BSA. pH 7.4. at a concentration of 600 µg/mL is incubated for 2.5 hrs with 12 µg/mL thrombin at 37°C followed by the addition of 1.1 U of hirudin per U of thrombin to inactivate the thrombin. Subsequently, thrombin is removed by anion-exchange chromatography with NaCl gradient elution. Residual thrombin, as determined by fibrin clotting, accounts for <0.00025% (moles of thrombin per moles of APC) of the protein (Mosnier et al. Blood 104:1740-1744, 2004). Concentrations of recombinant wild-type APC and APC mutants are determined by active-site titration using APC at 8 µM in HBS and *p*-nitrophenol-guanidino benzoate at 0.1 mM and using an extinction coefficient for p-nitrophenol of 11 400 M⁻¹cm⁻¹ (at pH 7.4) (Gale et al. Protein Sci. 6:132-140, 1997; Mosnier et al. Blood 104:1740-1744. 2004). The concentration of S360A-APC can be determined by Asserachrom PC ELISA from American Bioproducts (Parsippany, NJ).

### Glycocalicin Purification

Glycocalicin was isolated from outdated human platelets (Simon et al. J. Exp. Med. 192:193-204, 2000; Yun et al. J. Biol. Chem. 278:48112-48119, 2003). Erythrocyte-free platelets were isolated from 10 liters of outdated platelet-rich plasma by centrifugation and washing with buffer A (13 mM Na₃ citrate, 120 mM NaCl, and 30 mM glucose, pH 7.0). After a second centrifugation, the platelet pellet was suspended in 500 ml of buffer B (10 mM Tris/HCl, 150 mM NaCl, and 2 mM CaCl₂, pH 7.4) and then sonicated. The resultant suspension was incubated at 37°C for 30 minutes to allow the calpain released from the platelets during sonication to cleave membrane-bound GPIbα releasing glycocalicin. After ultracentrifugation to remove cell components, the glycocalicin-containing supernatant was applied to a wheat germ Sepharose 4B column. Bound crude glycocalicin was eluted with 2.5% N-acetyl-D-glucosamine and 20 mM Tris/HCl, pH 7.4. Further purification by ion exchange chromatography (on Q-Sepharose Fast-flow column; Pharmacia) was used to remove residual contaminants. Glycocalicin was eluted with a linear salt gradient of 0-0.7 M NaCl in 20 mM Tris/HCl, pH 7.4.

### Platelet Purification

Human venous blood from healthy volunteers was drawn by venipuncture into sodium citrate and acid/citrate/dextrose (ACD) (McCarty et al. J. Biol. Chem. 280:39474-39484, 2005: McCarty et al. J. Thromb. Haemost. 4:1367-1378, 2006). Platelet-rich plasma (PRP) was prepared by centrifugation of whole blood at 200g for 20 minutes. Platelets were isolated from PRP by centrifugation at 1000g for 10 minutes in the presence of prostacyclin (0.1 µg/ml). Platelet pellets were resuspended in Tyrodes containing 0.1 µg/ml prostacyclin, then washed and resuspended in modified Tyrodes buffer (129 NaCl mM, 0.34 mM Na₂HPO₄, 2.9 mM KCl. 12 mM NaHCO₃, 20 mM HEPES, 5 mM glucose, 1 mM MgCl₂; pH 7.3).

For flow adhesion studies using reconstituted blood, autologous RBCs were isolated following the initial centrifugation of whole blood (200g for 20 minutes). After the removal of PRP, the RBCs were pelleted by further centrifugation (2000g for 10 min), followed by washing (3x) with a HEPES buffer (10 mM HEPES, 140 mM NaCl. 5 mM glucose). Washed platelets (3 × 10⁸/ml final) were reconstituted with 50% (v/v) autologous-packed RBCs.

### Hemostasis test in mice

C57BL6 mice weighing 21-23 grams were purchased from Charles River Laboratories (Madison, WI) and used for hemostasis assessment with the tail transection test. Each animal was euthanized following the bleeding test. Mice were anesthetized with isoflurane and infused for 2 minutes through the right femoral vein with tPA (2 mg/kg, 150 µL total vehicle volume) to impair hemostasis. The effect of protein C on hemostasis was tested by co-administration of wild-type or active site mutated (S360A) mouse protein C (3 mg/kg) in combination with tPA. Control animals received physiological saline with or without tPA. Anesthesia was terminated immediately after the infusion, and mice were immobilized in a restraining device that allowed free access to the tail. The tail transection bleeding test was performed 20 minutes after the end of tPA/PC or vehicle administration. A disposable surgical blade was used to cut the tail at the point where the tail diameter reached approximately 1.5 mm (2-4 mm from the tip). After transection, the tail was immediately placed in a 1.7 ml tube filled with 500 µl of room temperature water. Bleeding time (by visual observation) and total volume of blood accumulated in the tube until the end of bleeding were recorded for up to 20 minutes.

### Examples 2: Characterization of platelet adhesion and activation on PC

To characterize the ability of platelets to bind PC and APC, purified human platelets were gently pipetted over surface-immobilized PC and APC. The purity of PC and APC was verified by SDS-PAGE. Platelet spreading, which is contingent on platelet activation, was monitored using Normarski DIC microscopy. As shown in FIG. 4A, human platelets underwent complete spreading on PC and APC, and the spreading was characterized by the generation of limited small filopodia followed by wave-like lamellipodia appearing before filopodia formation was complete. Actin stress fiber formation was observed on both PC and APC (FIG. 4B). A similar pattern of platelet spreading and stress fiber formation was observed on thrombin, while a sequential formation of discrete filopodia and lamellipodia was observed on fibrinogen.

To determine whether platelet adhesion to PC or APC resulted from either a receptor-mediated or an agonist-induced mechanism, the effect of PC, APC or thrombin in suspension on platelet binding was evaluated. Purified human platelets (2 × 10⁷/ml) were placed on BSA, PC, APC, recombinant APC (rAPC), or thrombin-coated coverslips for 45 minutes at 37°C. In designated experiments, washed platelets were resuspended in buffer containing exogenously added PC, APC, rAPC or thrombin (50 µg/ml), or the ADP-scavenger apyrase (apy) and TxA₂ inhibitor indomethacin (indo) (2 U/ml apy; 10 µM indomethacin), prior to exposure to the immobilized protein surface. Adherent platelets are reported as mean ± SEM >300 cells from 3-6 experiments. The results demonstrated that the addition of PC and APC in suspension abrogated the ability of platelets to bind to immobilized PC and APC surfaces, respectively (Table 1).

**Table 1**

| **Effect of ligands in suspension on platelet binding to immobilized ligands** | | | |
|---|---|---|---|
| **Surface** | **Suspension treatment** | **Platelet adhesion (cells/mm² × 10⁻²)** | **Platelet surface area (µm²)** |
| PC | - | 35.4 ± 4.4 | 30.2 ± 1.1 |
| PC | PC | 3.8 ± 1.8* | 9.9 ± 0.2 |
| PC | apy/indo | 11.2 ± 2.0* | 16.2 ± 0.9* |
| APC | - | 50.5 ± 8.5 | 31.8 ± 1.0 |
| APC | APC | 4.2 ± 2.5* | 10.2 ± 0.4* |
| APC | apy/indo | 18.8 + 6.3* | 15.1 ± 0.8* |
| rAPC | - | 41.8 ± 2.9 | 31.2 ± 1.3 |
| rAPC | rAPC | 2.6 ± 1.2* | 10.0 ± 0.5* |
| thrombin | - | 140.0 ± 9.6 | 39.1 ± 0.6 |
| thrombin | thrombin | 110.8 ± 6.5 | 40.0 ± 0.5 |
| thrombin | apy/indo | 111.4 ± 17.0 | 38.4 ± 0.7 |

| | | | |
|---|---|---|---|
| * *P* < 0.01 with respect to untreated samples for each surface | | | |

Platelets were found to bind to and spread on recombinant APC (rAPC). This adhesion to rAPC was eliminated when rAPC was present in suspension, indicating that adhesion to APC was unlikely due to contaminating factors, such as fibrinogen or VWF, in the plasma-derived APC. The presence of thrombin in the platelet suspension did not alter the number of platelets binding to immobilized thrombin. Together, these results suggest that platelet binding to PC and to APC is a receptor-mediated, rather than an enzymatic, process as evidenced by the ability of PC, APC, and rAPC in solution to competitively inhibit binding to immobilized ligands.

To evaluate the ability of PC and APC to induce outside-in signaling, intracellular Ca²⁺ flux of adherent platelets was examined on each surface. Platelets loaded with the Ca²⁺-sensitive dye, Oregon Green BAPTA 1-AM, were pipetted onto each surface and imaged in real-time with fluorescence microscopy. Following a delay of up to 120 seconds, adhesion to PC or APC generated a rapid and sustained Ca²⁺ spike, which subsequently declined over a period of 3-10 minutes (FIG. 5). Minimal oscillations were observed during the sustained elevation of Ca²⁺. A distinct pattern of intracellular Ca²⁺ was observed in platelets on thrombin. An initial, rapid elevation in intracellular Ca²⁺ was followed by a declining phase of Ca²⁺ levels that was superimposed by a series of small Ca²⁺ oscillations (FIG. 5). In contrast, a series of rhythmic spikes of Ca²⁺, each lasting 4-8 seconds, was observed in platelets on fibrinogen (FG), while the presence of the intracellular Ca²⁺ chelator, BAPTA-AM, abrogated intracellular Ca²⁺ elevations in platelets on all surfaces (FIG. 5).

### Examples 3: Molecular mechanisms regulating platelet adhesion and activation

Purified human platelets were exposed to immobilized ligands for 45 minutes at 37°C in the presence of vehicle, function-blocking antibodies or pharmacological pathway inhibitors. Adherent platelets were fixed and imaged via DIC microscopy. The results showed that addition of the ADP-scavenger apyrase and TxA₂ inhibitor indomethacin resulted in a >60% reduction in platelet adhesion on PC and on APC, as well as a substantial reduction in the degree of platelet lamellipodia formation (FIG. 6 and Table 1). In contrast, apyrase and indomethacin had no effect on platelet adhesion and spreading on thrombin. Moreover, platelet lamellipodia fonnation on PC, APC or thrombin was abrogated in the presence of the α_{IIb}β₃ mAb eptifibatide (FIG. 6). The presence of either a blocking α_{IIb}β₃ or GPIb mAb, whether alone or in combination, did not affect the degree of platelet adhesion to PC, APC, and thrombin under static conditions. Platelets treated with the Src kinase inhibitor, PP2, failed to form full lamellipodia on PC, APC, and fibrinogen, while the platelets retained full lamellipodia formation on thrombin. The inhibitory action of PP2 was overcome by the exogenous addition of thrombin as evidenced by the ability of platelets to form lamellipodia on PC, APC and fibrinogen in the presence of thrombin in solution subsequent to PP2 treatment. Platelets treated with the intracellular Ca²⁺ chelator, BAPTA-AM, were able to form filopodia, but lacked the ability to form lamellipodia on all surfaces. Platelet adhesion to thrombin, but not to PC, APC, or fibrinogen was abrogated in the presence of PPACK (a thrombin inhibitor), which irreversibly inactivates the active site of enzymes. Taken together, the data suggests that PC is capable of supporting pro-hemostatic platelet adhesion and activation in an ADP-dependent manner.

In the majority of previous studies (Rand et al. Transfus. Apher. Sci. 28:307-317, 2003), platelet activation and spreading have been evaluated under static conditions or in the low-shear conditions present in an aggregometer, whereas thrombus formation *in vivo* occurs under progressively increasing shear due to blood flow through the narrowing lumen. Therefore, to characterize the molecular mechanisms of APC- and PC-platelet interactions under physiologically relevant conditions, a parallel-plate flow chamber was utilized to mimic the shear conditions prevalent in the vasculature. As shown in FIG. 7, immobilized PC or APC supported platelet adhesion and limited the formation of platelet aggregates following a perfusion of reconstituted blood at 300 s⁻¹ for 3 minutes. Moreover, immobilized thrombin supported substantial platelet adhesion and robust aggregation under flow, while immobilized fibrinogen supported a confluent layer of adherent platelet, with only limited platelet aggregation. Platelet recruitment to immobilized PC, APC, and thrombin, but not to fibrinogen, was substantially inhibited in the presence of the function-blocking GPIb mAb 6D1 (10 µg/ml). The formation of platelet aggregates, but not platelet-APC or platelet-PC binding, was eliminated in the presence of an anti-α_{IIb}β₃ mAb. Platelet adhesion on fibrinogen was abrogated in the presence of the anti-α_{IIb}β₃ mAb eptifibatide. The presence of inhibitors to ADP/TxA₂ abrogated platelet deposition on APC and PC. but not on thrombin or fibrinogen. Platelets in sodium-citrate anti-coagulated whole blood failed to adhere to immobilized PC or APC under flow, in contrast to the substantial degree of platelets observed to adhere to thrombin and fibrinogen in whole blood. Platelet adhesion and aggregation was absent on PC, APC, and thrombin, but not fibrinogen, surfaces using PPACK-anticoagulated whole blood (Table 2).

**Table 2**

| **Platelet adhesion and aggregation under flow*** **(expressed as percent surface coverage)** | | | |
|---|---|---|---|
| **Surface** | **Reconstituted blood** | **NaCit WB** | **PPACK WB** |
| PC | 10.7 ± 1.2 | 0.2 ± 0.1 | 0.3 ± 0.1 |
| APC | 9.53 ± 1.3 | 0.1 ± 0.1 | 0.2 ± 0.1 |
| thrombin | 54.5 ± 4.1 | 5.7 ± 2.3 | 0.1 ± 0.1 |
| fibrinogen | 88.2 ± 6.1 | 89.1 ± 3.8 | 91.3 ± 2.9 |

| | | | |
|---|---|---|---|
| *Reconstituted blood or whole blood (WB) anticoagulated with either sodium citrate (NaCit) (0.38%) or PPACK (40 µM) was perfused over surfaces of immobilized PC, APC, thrombin and fibrinogen. Platelet adhesion is expressed as the percentage of surface coverage by platelets. Values are reported as mean ± SEM; n=3. | | | |

Real-time video microscopy studies revealed that PC, APC, and thrombin supported a substantial degree of platelet tethering and rolling under shear conditions using PPACK-anticoagulated whole blood. Human whole blood anticoagulated with PPACK (40 µM) was flowed over surfaces of immobilized PC, APC and thrombin. The mean number of platelets tethering to each surface during 50 sec ± SEM is shown in Table 3. Anti-GPIb mAb 6D1 (20 µg/mL) was added to the blood for select experiments. The number of interacting platelets (cells that bound to the surface for > 100 msec before rolling along the surface or resuming the velocity of free-flowing non-interacting cells) was significantly reduced in the presence of the anti-GPIb mAb 6D 1. These results indicate that GPIb plays a crucial role in mediating platelet binding to APC/PC under shear flow conditions. The results also demonstrated a critical role for the secondary mediators ADP and TxA₂ in mediating platelet adhesion and aggregation to APC/PC under shear conditions.

**Table 3**

| **Interactions between APC/PC and platelets under flow** | | |
|---|---|---|
| **Surface** | **Suspension treatment** | **Platelet interaction** **(cells/mm²/sec)** |
| BSA | - | 32.3 ± 14.6 |
| PC | - | 109.3 ± 21.5 |
| PC | 6D1 | 28.5 ± 7.2* |
| APC | - | 155.5 ± 45.5 |
| APC | 6D1 | 33.7 ± 64* |
| thrombin | - | 226.2 ± 145.4 |
| thrombin | 6D1 | 58.5 ± 19.2* |

| | | |
|---|---|---|
| **p* < (1.01 with respect to untreated samples. | | |

### Examples 4: Role of protein C in Hemostasis

To define the role of PC in hemostasis, tail bleeding assays were performed on mice. Following anesthetization, a 3-mm segment of the tail tip was cut off with a scalpel, and the tail was placed in a microcentrifuge tube of room temperature water. Both the time to cessation of bleeding and the total blood loss were recorded. The experiment was stopped after 900 seconds when the bleeding did not cease naturally. To enhance bleeding, in selected experiment prior to tail clipping, anesthetized mice were infused with tissue-type plasminogen activator (tPA; 2 mg/kg), which activates plasminogen, leading to fibrin degradation and increased bleeding. The results indicated that intravenous injection of murine PC (3 mg/kg) into tPA-treated wild-type mice significantly reduced the bleeding time (FIG. 8A). This reduction was further corroborated by a significant decrease in the amount of blood loss in PC-treated mice (235 ± 56.7 µl compared with 34.4 ± 5.55 µl for tPA-treated mice in the presence of vehicle or PC, respectively) (FIG. 8B). These results suggest that PC plays a pro-hemostatic role in the mouse model of tail bleeding.

The results described herein demonstrate that PC-platelet interactions play a pro-hemostatic role by contributing to platelet activation in a GPIb dependent manner.

### Example 5: Biophysical characterization of receptor-ligand interactions

The interaction of PC and APC with GPIb was evaluated using surface plasmon resonance (SPR). PC, APC, thrombin and the AI domain of VWF were each perfused over a glycocalicin (the proteolytic fragment of GPIb) coated sensor tip. The sensorgrams at different concentrations were obtained and normalized by subtracting background signals from the glycocalicin surface. Specific and saturable binding of all four ligands to glycocalicin was observed at a range of concentrations from 78 nM to 5.0 µM. After fitting SPR data to a Langmuir one-to-one binding model, the *K*_{d} for the interaction of PC and APC with glycocalicin was calculated at 330 and 308 nM, respectively, which is nearly 10-fold less than that between GPIb and VWF A1 (Table 4). Taken together, these findings demonstrate that GPIb serves as a receptor for PC and APC.

**Table 4**

| **Dissociation constants for glycocalicin** | |
|---|---|
| **Ligand** | ***K*_{d}** |
| PC | 330 ± 26 nM |
| APC | 308 ± 21 nM |
| thrombin | 361 ± 29 nM |
| VWF A1 | 36 ± 4.0 nM |

To characterize APC- and PC-platelet interactions, the biophysical parameters associated with APC/PC binding to the platelet surface was determined by immobilizing PC onto a 0.99-µm carboylate-modified latex bead (PC-bead). An individual platelet was then trapped from a suspension of washed platelets (5×10⁶ plt/ml) and PC-beads (2×10⁵/ml), and was manually attached to a 5-µm diameter fibrinogen-coated silica bead that had been immobilized onto a glass surface. Subsequently, a PC- bead, trapped by the laser light, was brought within a distance of 2-3 µm from the immobilized platelet. Oscillation of the PC-bead was initiated at 50 Hz with a 0.8 µm peak-to-peak amplitude, and then the bead was brought into contact with the platelet by micromanipulation of the stage. The lateral forces that the trap exerted on the bead were measured with a quadrant detector conjugated to the back focal plane of the condenser and were calibrated from the low-frequency component of the Brownian motion.

FIG. 9 shows a representative data trace for a typical interaction between a PC-bead and an immobilized platelet, partitioned into four parts. The PC-bead, trapped near the center of the laser beam, is moved toward (Upper A) or away (Upper D) from the immobilized platelet, corresponding to zero force (Lower A,D). At the moment of contact (Upper B), the platelet stops the motion of the PC-bead while the laser beam continues in the same direction (right). The laser trap exerts a positive, compressive force on the platelet and bead (Lower B). The trap motion is then reversed, and the compressive force declines to zero. Peak B (Lower) represents the time that the platelet and the PC-bead are under a compressive force (contact duration time). When the PC-bead and platelet unite (Upper C), the bead position remains nearly constant as the laser continues to move to the left. The force on the bead increases in the negative direction (Lower C), almost linearly until the PC-bead-platelet bond is ruptured and the force rapidly returns to nearly zero. These results suggest that PC is capable of binding platelets under non-equilibrium conditions.

### Example 6: Characterization of PC binding to the platelet surface

To characterize the agility of platelets to recruit PC to the platelet surface and to catalyze the APC generation, platelets were immobilized onto a glass slide treated with 3-aminopropyltriethoxysilane (APES). The platelet-coated slides were treated with 1% BSA for 10 min prior to their use in adhesion assays to prevent non-specific interactions. PC was immobilized onto the surface of 10 µm-diameter polystyrene beads (PC-beads). Using a parallel-plate flow chamber, PC-beads were perfused over immobilized platelets at a shear rate of 150/second for 5 minutes.

As shown in FIG. 10, immobilized platelets supported the recruitment and firm adhesion of PC-coated beads. The presence of a blocking GPIb mAb 6D1 prevented PC-bead attachment to platelets under shear flow conditions. Beads coated with BSA failed to bind to immobilized platelets. These results indicate that platelets support a GPIb-dependent recruitment of PC to the platelet surface under shear.

It is believed that, in addition to promoting pro-hemostatic platelet activation at sites of injury, platelets facilitate the anti-thrombotic local generation of APC. To characterize the ability of platelet receptors to potentiate the conversion of PC to APC, a suspension of 50 µl of platelets (at both 7×10⁸/ml (low platelet count) and 4.5 ×10⁹/ml (high platelet count)) was incubated with 100 nM PC in the presence of thrombin. Reactions were stopped after 60-120 minutes though the addition of 100 nM hirudin, and levels of APC were determined using an APC-specific mAb in conjunction with a HAPC-1555 enzyme capture assay (Liaw et al. J. Thromb. Haemost. 1:662-670, 2003). These data demonstrate that the presence of activated platelets potentiated a 3-fold and 15-fold increase in the concentration of (unbound) APC in the fluid phase at low and high platelet concentrations, respectively (FIG. 11). This assay indicates that the thrombin-catalyzed activation of PC is augmented by the platelet surface.

Taken altogether, these data indicate that APC- and PC-platelet interactions play a dual role during hemostasis and thrombosis, and provide evidence that PC-platelet binding is pro-hemostatic, while platelet facilitated APC generation is anti-thrombotic.

### Example 7: Effect of wild-type PC and an active site PC mutant on hemostasis

This example further describes the hemostatic activity of PC and demonstrates that a variant of PC retains hemostatic activity. Murine tail bleeding assays were used to assess the effect of elevated levels of plasma protein C on hemostasis. The effects of tPA (2 mg/kg) and/or recombinant murine protein C (3 mg/kg) on tail bleeding assays are summarized in FIG. 12. The baseline mean bleeding time (623 ± 49.3 seconds; n=10) was significantly prolonged following administration of the fibrinolytic agent tPA (932 ± 65.3 seconds; n=12, p<0.05). Bleeding volume following tPA administration (357 ± 41.2 µL; n=12) was also increased compared to vehicle control (130 ± 32.1 µL: n=10; p<0.01). Infusion of murine protein C with vehicle reduced bleeding time to 483 ± 51.7 seconds and blood loss to 56.8 ± 11.4 µL (n=10; p<0.01). Co-administration of protein C with tPA reduced the bleeding time (454 ± 35.6 seconds: n=10; p<0.01) and bleeding volume (109 ± 45.6 µL; n=10; p<0.01) to near vehicle control levels.

A similar reduction in bleeding time and volume was observed following co-infusion of tPA with the recombinant murine form of the enzymatically inactive (active site mutant S360A) protein C (578 ± 35.6 seconds and 81.8 ± 36.4 µL for bleeding time and volume, respectively; n=8; p<0.01). These data suggest that recombinant protein C acts as a hemostatic agent during tPA-induced hemostasis impairment in mice, and that the enzymatic active site of protein C is not required for this effect.

### Example 8: Treatment of a bleeding disorder with a protein C polypeptide

This example describes the treatment of a patient diagnosed with hemophilia with a human recombinant protein C polypeptide. A patient diagnosed with hemophilia is treated prophylactically with human protein C polypeptide by administration of purified protein C polypeptide having an amino acid sequence of SEQ ID NO: 2 in a pharmaceutically acceptable carrier. Protein C is administered to the patient intravenously by bolus injection at a dose of 3 mg/kg once a week. The dose and dosing schedule of protein C administration can vary and is determined in part by the severity of the disease, and the age, weight and general health of the patient. Patients having moderate to severe hemophilia prone to episodes of spontaneous bleeding, can receive repeated doses at regular intervals, such as once a day, twice a week, once a week, twice a month, or once a month. An appropriate dose and timing of administration can be determined by a skilled practitioner.

A patient with mild hemophilia that exhibits uncontrolled bleeding in response to trauma or surgery can be treated with protein C as needed. For example, a patient diagnosed with mild hemophilia that requires surgery can be treated with protein C prior to surgery. The patient with mild hemophilia is administered purified recombinant human protein C having an amino acid sequence of SEQ ID NO: 2 in a pharmaceutically acceptable carrier. Protein C is administered by intravenous bolus injection at a dose of 3 mg/kg approximately one hour prior to surgery, and every twelve hours following surgery as needed. The patient can be monitored for uncontrolled bleeding and treated with additional protein C as needed.

### Examples 9: Treatment of a bleeding episode with a protein C polypeptide

The example describes the treatment of a patient having a traumatic wound with a human recombinant protein C polypeptide. A patient presenting with a stab wound is first treated surgically to close the wound. The patient is then treated with human protein C polypeptide by administration of purified protein C polypeptide having an amino acid sequence of SEQ ID NO: 2 in a pharmaceutically acceptable carrier. Protein C is administered to the patient intravenously by bolus injection at a dose of 3 mg/kg every 12 hours as needed. The dose and dosing schedule of protein C administration can vary and is determined in part by the severity of the wound, and the age, weight and general health of the patient. An appropriate dose and administration schedule can be determined by a skilled practitioner. For example, the patient can be administered protein C every 12 hours until bleeding is controlled.

## Claims

1. A protein C polypeptide, or a hemostatic fragment or hemostatic variant thereof, or a vector comprising a protein C nucleic acid sequence which encodes a protein C polypeptide or a hemostatic fragment or hemostatic variant thereof, for use in a method of promoting hemostasis in a subject, the method comprising administering to the subject the protein C polypeptide, or a hemostatic fragment or hemostatic variant thereof, or the vector, in a therapeutically effective dose sufficient to achieve hemostasis, wherein the protein C polypeptide or hemostatic fragment or hemostatic variant thereof comprises at least 90% sequence identity with the amino acid sequence set forth as SEQ ID NO: 2, 4, 6, 8, 10, 12, 14, 16 or 18.

2. Use of a protein C polypeptide, or a hemostatic fragment or hemostatic variant thereof, or a vector comprising a protein C nucleic acid sequence which encodes a protein C polypeptide or a hemostatic fragment or hemostatic variant thereof , in the manufacture of a medicament for promoting hemostasis in a subject, wherein the promoting hemostasis is by a method of treatment which comprises administering to the subject the protein C polypeptide, or hemostatic fragment or hemostatic variant thereof, or the vector, in a therapeutically effective dose sufficient to achieve hemostasis, wherein the protein C polypeptide or hemostatic fragment or hemostatic variant thereof comprises at least 90% sequence identity with the amino acid sequence set forth as SEQ ID NO: 2, 4, 6, 8, 10, 12, 14, 16 or 18.

3. The protein C polypeptide, hemostatic fragment or hemostatic variant thereof, or vector, for use according to claim 1 or the use of a protein C polypeptide, or a hemostatic fragment or hemostatic variant thereof, or vector, according to claim 2, wherein the subject has been diagnosed with a bleeding disorder or a bleeding episode.

4. The protein C polypeptide, hemostatic fragment or hemostatic variant thereof, or the vector, for use, or the use of a protein C polypeptide, or a hemostatic fragment or hemostatic variant thereof , or the vector, according to any one of claims 1-3, wherein the protein C polypeptide or hemostatic fragment or hemostatic variant thereof comprises at least 95% sequence identity with the amino acid sequence set forth as SEQ ID NO: 2, 4, 6, 8, 10, 12, 14, 16 or 18.

5. The protein C polypeptide, hemostatic fragment or hemostatic variant thereof, or the vector, for use, or the use of a protein C polypeptide, or a hemostatic fragment or hemostatic variant thereof, or the vector, according to claim 4, wherein the protein C polypeptide or hemostatic fragment or hemostatic variant thereof comprises at least 99% sequence identity with the amino acid sequence set forth as SEQ ID NO: 2, 4, 6, 8, 10, 12, 14, 16 or 18.

6. The protein C polypeptide or vector comprising a protein C nucleic acid sequence which encodes the protein C polypeptide for use, or the use of a protein C polypeptide or vector comprising a protein C nucleic acid sequence which encodes the protein C polypeptide, according to claim 5, wherein the protein C polypeptide comprises the amino acid sequence set forth as SEQ ID NO: 2, 4, 6, 8, 10, 12, 14, 16 or 18.

7. The protein C polypeptide or vector comprising a protein C nucleic acid sequence which encodes the protein C polypeptide for use, or the use of a protein C polypeptide or vector comprising a protein C nucleic acid sequence which encodes the protein C polypeptide, according to any one of claims 1-3, wherein the protein C polypeptide consists of the amino acid sequence set forth as SEQ ID NO: 2, 4, 6, 8, 10, 12, 14, 16 or 18.

8. The protein C polypeptide hemostatic variant or vector comprising a protein C nucleic acid sequence which encodes the protein C polypeptide hemostatic variant, for use, or the use of a protein C polypeptide hemostatic variant or vector comprising a protein C nucleic acid sequence which encodes the protein C polypeptide hemostatic variant, according to any one of claims 1-3, wherein the protein C polypeptide hemostatic variant is the S360A mutant.

9. The protein C polypeptide, hemostatic fragment or hemostatic variant thereof, or vector, for use, or the use of a protein C polypeptide, or a hemostatic fragment or hemostatic variant thereof, or vector, according to any one of claims 1-8 wherein the protein C polypeptide, hemostatic fragment or hemostatic variant thereof, is in a fusion protein with a different protein.

10. The protein C polypeptide, hemostatic fragment or hemostatic variant thereof, or vector, for use, or the use of a protein C polypeptide, or a hemostatic fragment or hemostatic variant thereof, or vector, according to any one of claims 1-9, wherein the protein C polypeptide or hemostatic fragment or variant thereof is administered in said method by a parenteral route, optionally intravenously, or is administered in said method topically.

11. The protein C polypeptide, hemostatic fragment or hemostatic variant thereof, for use, or the use of a protein C polypeptide, or a hemostatic fragment or hemostatic variant thereof, according to claim 10, wherein the protein C polypeptide or hemostatic fragment or hemostatic variant thereof is administered in said method as part of a wound dressing.

12. The protein C polypeptide, hemostatic fragment or hemostatic variant thereof, for use, or the use of a protein C polypeptide, or a hemostatic fragment or hemostatic variant thereof, according to any one of claims 1-10, wherein the protein C polypeptide or hemostatic fragment or hemostatic variant thereof is administered in said method at a dose of 1 to 100 mg/day, optionally at a dose of 1 to 10 mg/kg.

13. The protein C polypeptide, hemostatic fragment or hemostatic variant thereof, or vector, for use, or the use of a protein C polypeptide, or a hemostatic fragment or hemostatic variant thereof, or vector, according to any one of claims 1-12, wherein the protein C polypeptide or hemostatic fragment or hemostatic variant thereof or the vector is administered in said method in a single dose or in multiple doses.

14. The vector for use, or the use of a vector, according to any one of claims 1-3, wherein said method comprises administering said vector, wherein the protein C nucleic acid sequence comprises at least 90% sequence identity with the nucleotide sequence set forth as SEQ ID NO: 1, 3, 5, 7, 9, 11, 13, 15 or 17.

15. The vector for use, or the use of a vector, according to claim 14, wherein the protein C nucleic acid sequence comprises at least 95% sequence identity with the nucleotide sequence set forth as SEQ ID NO: 1, 3, 5, 7, 9, 11, 13, 15 or 17.

16. The vector for use, or the use of a vector, according to claim 15, wherein the protein C nucleic acid sequence comprises at least 99% sequence identity with the nucleotide sequence set forth as SEQ ID NO: 1, 3, 5, 7, 9, 11, 13, 15 or 17.

17. The vector for use, or the use of a vector, according to claim 16, wherein the nucleic acid sequence comprises SEQ ID NO: 1, 3, 5, 7, 9, 11, 13, 15 or 17.

18. The vector for use, or the use of a vector, according to any one of claims 1-3, wherein the nucleic acid sequence consists of SEQ ID NO: 1, 3, 5, 7, 9, 11, 13, 15 or 17.

19. The vector for use, or the use of a vector, according to any preceding claim, wherein the vector is a viral vector or a eukaryotic expression vector.

20. The protein C polypeptide, hemostatic fragment or hemostatic variant thereof, or vector, for use, or the use of a protein C polypeptide, or a hemostatic fragment or hemostatic variant thereof, or vector, according to any one of claims 1-19, wherein the bleeding disorder is a clotting factor deficiency, a platelet disorder, thrombocytopenia, vitamin K deficiency or von Willebrand's disease, wherein the clotting factor deficiency is optionally hemophilia A, hemophilia B or hemophilia C; or wherein the bleeding episode is caused by a drug, an anticoagulant overdose, an aneurysm, blood vessel rupture, surgery, a traumatic injury, cancer, gastrointestinal ulceration or an infection.

## Patentansprüche

1. Protein-C-Polypeptid oder ein hämostatisches Fragment oder eine hämostatische Variante davon oder Vektor, der eine Protein-C-Nucleinsäuresequenz umfasst, die für ein Protein-C-Polypeptid oder ein hämostatisches Fragment oder eine hämostatische Variante davon kodiert, zur Verwendung in einem Verfahren zur Förderung von Hämostase bei einem Individuum, wobei das Verfahren das Verabreichen des Protein-C-Polypeptids oder eines hämostatischen Fragments oder einer hämostatischen Variante davon oder des Vektors an den Patienten in einer therapeutisch wirksamen Dosis, die ausreicht, um Hämostase zu erreichen, umfasst, worin das Protein-C-Polypeptid, das hämostatische Fragment oder die hämostatische Variante davon zumindest 90 % Sequenzidentität mit der als Seq.-ID Nr. 2, 4, 6, 8, 10, 12, 14, 16 oder 18 dargelegten Aminosäuresequenz umfasst.

2. Verwendung eines Protein-C-Polypeptids oder eines hämostatischen Fragments oder einer hämostatischen Variante davon oder eines Vektors, der eine Protein-C-Nucleinsäuresequenz umfasst, die für ein Protein-C-Polypeptid oder ein hämostatisches Fragment oder eine hämostatische Variante davon kodiert, bei der Herstellung eines Medikaments zur Förderung von Hämostase bei einem Individuum, wobei die Förderung von Hämostase durch ein Behandlungsverfahren erfolgt, das das Verabreichen des Protein-C-Polypeptids oder eines hämostatischen Fragments oder einer hämostatischen Variante davon oder des Vektors an den Patienten in einer therapeutisch wirksamen Dosis, die ausreicht, um Hämostase zu erreichen, umfasst, worin das Protein-C-Polypeptid oder das hämostatische Fragment oder die hämostatische Variante davon zumindest 90 % Sequenzidentität mit der als Seq.-ID Nr. 2, 4, 6, 8, 10, 12, 14, 16 oder 18 dargelegten Aminosäuresequenz umfasst.

3. Protein-C-Polypeptid oder ein hämostatisches Fragment oder oder eine hämostatische Variante davon oder Vektor zur Verwendung nach Anspruch 1 oder Verwendung eines Protein-C-Polypeptids oder eines hämostatischen Fragments oder einer hämostatischen Variante davon oder eines Vektors nach Anspruch 2, worin bei dem Individuum eine Blutungsstörung oder eine Blutungsepisode diagnostiziert wurde.

4. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach einem der Ansprüche 1 bis 3, worin das Protein-C-Polypeptid oder das hämostatische Fragment oder die hämostatische Variante davon zumindest 95 % Sequenzidentität mit der als Seq.-ID Nr. 2, 4, 6, 8, 10, 12, 14, 16 oder 18 dargelegten Aminosäuresequenz umfasst.

5. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach Anspruch 4, worin das Protein-C-Polypeptid oder das hämostatische Fragment oder die hämostatische Variante davon zumindest 99 % Sequenzidentität mit der als Seq.-ID Nr. 2, 4, 6, 8, 10, 12, 14, 16 oder 18 dargelegten Aminosäuresequenz umfasst.

6. Protein-C-Polypeptid oder Vektor zur Verwendung oder Verwendung eines Protein-C-Polypeptids oder eines hämostatischen Fragments oder einer hämostatischen Variante davon oder eines Vektors nach Anspruch 5, worin das Protein-C-Polypeptid die als Seq.-ID Nr. 2, 4, 6, 8, 10, 12, 14, 16 oder 18 dargelegte Aminosäuresequenz umfasst.

7. Protein-C-Polypeptid oder Vektor zur Verwendung oder Verwendung eines Protein-C-Polypeptids oder eines hämostatischen Fragments oder einer hämostatischen Variante davon oder eines Vektors nach einem der Ansprüche 1 bis 3, worin das Protein-C-Polypeptid aus der als Seq.-ID Nr. 2, 4, 6, 8, 10, 12, 14, 16 oder 18 dargelegten Aminosäuresequenz besteht.

8. Hämostatische Variante eines Protein-C-Polypeptids oder Vektor, der eine Protein-C-Nucleinsäuresequenz umfasst, die für eine hämostatische Variante eines Protein-C-Polypeptids kodiert, zur Verwendung oder Verwendung einer hämostatischen Variante eines Protein-C-Polypeptids oder eines Vektors, der eine Protein-C-Nucleinsäuresequenz umfasst, die für eine hämostatische Variante eines Protein-C-Polypeptids kodiert, nach einem der Ansprüche 1 bis 3, worin die hämostatische Variante eines Protein-C-Polypeptids die S360A-Mutante ist.

9. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach einem der Ansprüche 1 bis 8, worin das Protein-C-Polypeptid oder das hämostatische Fragment oder die hämostatische Variante davon zusammen mit einem anderen Protein in einem Fusionsprotein vorliegt.

10. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach einem der Ansprüche 1 bis 8, worin das Protein-C-Polypeptid oder das hämostatische Fragment oder die hämostatische Variante davon in den Verfahren auf parenteralem Wege, gegebenenfalls intravenös, oder in dem Verfahren topisch verabreicht wird.

11. Protein-C-Polypeptid oder ein hämostatisches Fragment oder eine hämostatische Variante davon zur Verwendung oder Verwendung eines Protein-C-Polypeptids oder eines hämostatischen Fragments oder einer hämostatischen Variante davon nach Anspruch 10, worin das Protein-C-Polypeptid oder das hämostatische Fragment oder die hämostatische Variante davon in den Verfahren als Teil eines Wundverbands verabreicht wird.

12. Protein-C-Polypeptid oder ein hämostatisches Fragment oder eine hämostatische Variante davon zur Verwendung oder Verwendung eines Protein-C-Polypeptids oder eines hämostatischen Fragments oder einer hämostatischen Variante davon nach einem der Ansprüche 1 bis 10, worin das Protein-C-Polypeptid oder das hämostatische Fragment oder die hämostatische Variante davon in den Verfahren in einer Dosis von 1 bis 100 mg/Tag, gegebenenfalls in einer Dosis von 1 bis 10 mg/kg, verabreicht wird.

13. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach einem der Ansprüche 1 bis 12, worin das Protein-C-Polypeptid oder das hämostatische Fragment oder die hämostatische Variante davon oder der Vektor in dem Verfahren in einer einzigen Dosis oder in mehreren Dosen verabreicht wird.

14. Vektor zur Verwendung oder Verwendung eines Vektors nach einem der Ansprüche 1 bis 3, worin das Verfahren das Verabreichen des Vektors umfasst, worin die Protein-C-Nucleinsäuresequenz zumindest 90 % Sequenzidentität mit der als Seq.-ID Nr. 1, 3, 5, 7, 9, 11, 13, 15 oder 17 dargelegten Nucleinsäuresequenz umfasst.

15. Vektor zur Verwendung oder Verwendung eines Vektors nach Anspruch 14, worin die Protein-C-Nucleinsäuresequenz zumindest 95 % Sequenzidentität mit der als Seq.-ID Nr. 1, 3, 5, 7, 9, 11, 13, 15 oder 17 dargelegten Nucleinsäuresequenz umfasst.

16. Vektor zur Verwendung oder Verwendung eines Vektors nach Anspruch 15, worin die Protein-C-Nucleinsäuresequenz zumindest 99 % Sequenzidentität mit der als Seq.-ID Nr. 1, 3, 5, 7, 9, 11, 13, 15 oder 17 dargelegten Nucleinsäuresequenz umfasst.

17. Vektor zur Verwendung oder Verwendung eines Vektors nach Anspruch 16, worin die Nucleinsäuresequenz Seq.-ID Nr. 1, 3, 5, 7, 9, 11, 13, 15 oder 17 umfasst.

18. Vektor zur Verwendung oder Verwendung eines Vektors nach einem der Ansprüche 1 bis 3, worin die Nucleinsäuresequenz aus Seq.-ID Nr. 1, 3, 5, 7, 9, 11, 13, 15 oder 17 besteht.

19. Vektor zur Verwendung oder Verwendung eines Vektors nach einem der vorangegangenen Ansprüche, worin der Vektor ein viraler Vektor oder ein eukaryotischer Expressionsvektor ist.

20. Protein-C-Polypeptid oder ein hämostatisches Fragment oder eine hämostatische Variante davon oder Vektor zur Verwendung oder Verwendung eines Protein-C-Polypeptids oder eines hämostatischen Fragments oder einer hämostatischen Variante davon oder eines Vektors nach einem der Ansprüche 1 bis 19, worin die Blutungsstörung eine Gerinnungsfaktordefizienz, eine Plättchenstörung, Thrombozytopenie, Vitamin-K-Mangel oder Willebrand-Jürgens-Syndrom ist, worin die Gerinnungsfaktordefizienz gegebenenfalls Hämophilie A, Hämophilie B oder Hämophilie C ist, oder worin die Blutungsepisode durch ein Arzneimittel, eine Überdosis eines gerinnungshemmenden Mittels, ein Aneurysma, einen Riss eines Blutgefäßes, eine Operation, eine traumatische Verletzung, Krebs, die Bildung eines Magen-Darm-Geschwürs oder eine Infektion verursacht wird.

## Revendications

1. Polypeptide de protéine C, ou fragment hémostatique ou variant hémostatique de celui-ci, ou vecteur comprenant une séquence d'acide nucléique de protéine C qui code pour un polypeptide de protéine C ou un fragment hémostatique ou un variant hémostatique de celui-ci, pour une utilisation dans un procédé destiné à promouvoir l'hémostase chez un sujet, le procédé consistant à administrer au sujet le polypeptide de protéine C, ou un fragment hémostatique ou un variant hémostatique de celui-ci, ou le vecteur, en une dose thérapeutiquement efficace qui est suffisante pour obtenir une hémostase, où le polypeptide de protéine C ou fragment hémostatique ou variant hémostatique de celui-ci comprend au moins 90 % d'identité de séquence avec la séquence d'acides aminés décrite en tant que SEQ ID NO: 2, 4, 6, 8, 10, 12, 14, 16 ou 18.

2. Utilisation d'un polypeptide de protéine C, ou d'un fragment hémostatique ou variant hémostatique de celui-ci, ou d'un vecteur comprenant une séquence d'acide nucléique de protéine C qui code pour un polypeptide de protéine C ou un fragment hémostatique ou un variant hémostatique de celui-ci, dans la fabrication d'un médicament destiné à promouvoir l'hémostase chez un sujet, où la promotion de l'hémostase est réalisé par un procédé de traitement qui consiste à administrer au sujet le polypeptide de protéine C, ou un fragment hémostatique ou variant hémostatique de celui-ci, ou le vecteur, en une dose thérapeutiquement efficace qui est suffisante pour obtenir une hémostase, où le polypeptide de protéine C ou fragment hémostatique ou variant hémostatique de celui-ci comprend au moins 90 % d'identité de séquence avec la séquence d' acides aminés décrite en tant que SEQ ID NO: 2, 4, 6, 8, 10, 12, 14, 16 ou 18.

3. Polypeptide de protéine C, fragment hémostatique ou variant hémostatique de celui-ci, ou vecteur, pour une utilisation selon la revendication 1, ou utilisation d'un polypeptide de protéine C, ou d'un fragment hémostatique ou variant hémostatique de celui-ci, ou d'un vecteur, selon la revendication 2, où le sujet a reçu un diagnostic de trouble hémorragique ou d'épisode hémorragique.

4. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou du vecteur, selon l'une quelconque des revendications 1 à 3, où le polypeptide de protéine C ou fragment hémostatique ou variant hémostatique de celui-ci comprend au moins 95 % d'identité de séquence avec la séquence d'acides aminés décrite en tant que SEQ ID NO: 2, 4, 6, 8, 10, 12, 14, 16 ou 18.

5. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou du vecteur, selon la revendication 4, où le polypeptide de protéine C ou fragment hémostatique ou variant hémostatique de celui-ci comprend au moins 99 % d'identité de séquence avec la séquence d'acides aminés décrite en tant que SEQ ID NO: 2, 4, 6, 8, 10, 12, 14, 16 ou 18.

6. Polypeptide de protéine C ou vecteur comprenant une séquence d'acide nucléique de protéine C qui code pour le polypeptide de protéine C pour une utilisation, ou utilisation d'un polypeptide de protéine C ou d'un vecteur comprenant une séquence d'acide nucléique de protéine C qui code pour le polypeptide de protéine C, selon la revendication 5, où le polypeptide de protéine C comprend la séquence d'acides aminés décrite en tant que SEQ ID NO: 2, 4, 6, 8, 10, 12, 14, 16 ou 18.

7. Polypeptide de protéine C ou vecteur comprenant une séquence d'acide nucléique de protéine C qui code pour le polypeptide de protéine C pour une utilisation, ou utilisation d'un polypeptide de protéine C ou d'un vecteur comprenant une séquence d'acide nucléique de protéine C qui code pour le polypeptide de protéine C, selon l'une quelconque des revendications 1 à 3, où le polypeptide de protéine C consiste en la séquence d'acides aminés décrite en tant que SEQ ID NO: 2, 4, 6, 8, 10, 12, 14, 16 ou 18.

8. Variant hémostatique de polypeptide de protéine C ou vecteur comprenant une séquence d'acide nucléique de protéine C qui code pour le variant hémostatique de polypeptide de protéine C, pour une utilisation, ou utilisation d'un variant hémostatique de polypeptide de protéine C ou d'un vecteur comprenant une séquence d'acide nucléique de protéine C qui code pour le variant hémostatique de polypeptide de protéine C, selon l'une quelconque des revendications 1 à 3, où le variant hémostatique de polypeptide de protéine C est le mutant S360A.

9. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou d'un vecteur, selon l'une quelconque des revendications 1 à 8, où le polypeptide de protéine C, fragment hémostatique ou variant hémostatique de celui-ci, est dans une protéine de fusion avec une protéine différente.

10. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou d'un vecteur, selon l'une quelconque des revendications 1 à 9, où le polypeptide de protéine C ou fragment ou variant hémostatique de celui-ci est administré dans ledit procédé par une voie parentérale, facultativement intraveineuse, ou est administré dans ledit procédé par voie topique.

11. Polypeptide de protéine C, fragment hémostatique ou variant hémostatique de celui-ci, pour une utilisation, ou utilisation d'un polypeptide de protéine C, ou d'un fragment hémostatique ou variant hémostatique de celui-ci, selon la revendication 10, où le polypeptide de protéine C ou fragment hémostatique ou variant hémostatique de celui-ci est administré dans ledit procédé en tant qu'élément d'un pansement pour une plaie.

12. Polypeptide de protéine C, fragment hémostatique ou variant hémostatique de celui-ci, pour une utilisation, ou utilisation d'un polypeptide de protéine C, ou d'un fragment hémostatique ou variant hémostatique de celui-ci, selon l'une quelconque des revendications 1 à 10, où le polypeptide de protéine C ou fragment hémostatique ou variant hémostatique de celui-ci est administré dans ledit procédé à une dose de 1 à 100 mg/jour, facultativement à une dose de 1 à 10 mg/kg.

13. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou d'un vecteur, selon l'une quelconque des revendications 1 à 12, où le polypeptide de protéine C ou fragment hémostatique ou variant hémostatique de celui-ci ou le vecteur est administré dans ledit procédé en une dose unique ou en doses multiples.

14. Vecteur pour une utilisation, ou utilisation d'un vecteur, selon l'une quelconque des revendications 1 à 3, où ledit procédé consiste à administrer ledit vecteur, où la séquence d'acide nucléique de protéine C comprend au moins 90 % d'identité de séquence avec la séquence de nucléotides décrite en tant que SEQ ID NO: 1, 3, 5, 7, 9, 11, 13, 15 ou 17.

15. Vecteur pour une utilisation, ou utilisation d'un vecteur, selon la revendication 14, où la séquence d'acide nucléique de protéine C comprend au moins 95 % d'identité de séquence avec la séquence de nucléotides décrite en tant que SEQ ID NO: 1, 3, 5, 7, 9, 11, 13, 15 ou 17.

16. Vecteur pour une utilisation, ou utilisation d'un vecteur, selon la revendication 15, où la séquence d'acide nucléique de protéine C comprend au moins 99 % d'identité de séquence avec la séquence de nucléotides décrite en tant que SEQ ID NO: 1, 3, 5, 7, 9, 11, 13, 15 ou 17.

17. Vecteur pour une utilisation, ou utilisation d'un vecteur, selon la revendication 16, où la séquence d'acide nucléique comprend SEQ ID NO: 1, 3, 5, 7, 9, 11, 13, 15 ou 17.

18. Vecteur pour une utilisation, ou utilisation d'un vecteur, selon l'une quelconque des revendications 1 à 3, où la séquence d'acide nucléique consiste en SEQ ID NO: 1, 3, 5, 7, 9, 11, 13, 15 ou 17.

19. Vecteur pour une utilisation, ou utilisation d'un vecteur, selon l'une quelconque des revendications précédentes, où le vecteur est un vecteur viral ou un vecteur d'expression eucaryote.

20. Polypeptide de protéine C, fragment hémostatique ou variant hémostatique de celui-ci, ou vecteur, pour une utilisation, ou utilisation d'un polypeptide de protéine C, ou d'un fragment hémostatique ou variant hémostatique de celui-ci, ou d'un vecteur, selon l'une quelconque des revendications 1 à 19, dans lequel le trouble hémorragique est un déficit en facteur de la coagulation, un trouble plaquettaire, une thrombocytopénie, un déficit en vitamine K ou la maladie de von Willebrand, dans lequel le déficit en facteur de la coagulation est facultativement l'hémophilie A, l'hémophilie B ou l'hémophilie C; ou dans lequel l'épisode hémorragique est provoqué par un médicament, une dose excessive d'anticoagulant, un anévrysme, la rupture d'un vaisseau sanguin, une intervention chirurgicale, une lésion traumatique, un cancer, un ulcère gastro-intestinal ou une infection.
